# EUROPÄISCHE PATENTANMELDUNG

(11) **EP 2 194 052 A1**
(43) Veröffentlichungstag der Anmeldung: **09.06.2010**
(21) Anmeldenummer: 08021241.8
(22) Anmeldetag: 06.12.2008
(51) Int. Cl.: C07D 417/04, C07D 417/14, A01N 43/74, A01N 43/78

(54) **Substituierte 1-(Thiazolyl)- und 1-(Isothiazolyl)pyrazol-4-yl-essigsäuren, Verfahren zu deren Herstellung und deren Verwendung als Herbizide und Pflanzenwachstumsregulatoren**

(71) Anmelder: Bayer CropScience AG, 40789 Monheim (DE)
(72) Erfinder: Jakobi, Harald, Dr., 60598 Frankfurt (DE); Martelletti, Arianna, Dr., 65843 Sulzbach (DE); Dittgen, Jan, Dr., 60316 Frankfurt (DE); Haeuser-Hahn, Isolde, Dr., 51375 Leverkusen (DE); Feucht, Dieter, Dr., 65760 Eschborn (DE); Rosinger, Christopher, Dr., 65719 Hofheim (DE)

(57) **Zusammenfassung**

Gegenstand der Erfindung sind 1-(Thiazolyl)- und 1-(Isothiazolyl)pyrazol-4-yl-essigsäuren Derivate der allgemeinen Formel (I) und deren Salze, 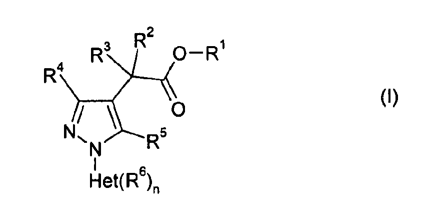 worin Het, R¹, R², R³, R⁴, R⁵, R⁶ und n wie in Anspruch 1 definiert sind.
Die Verbindungen (I) oder deren Salze sind als Herbizide und
Pflanzenwachstumsregulatoren, insbesondere als Herbizide zur selektiven Bekämpfung von Schadpflanzen in Nutzpflanzenkulturen geeignet und können nach Verfahren hergestellt werden, wie sie in Anspruch 9 beschrieben sind.

## Beschreibung

Die Erfindung betrifft das technische Gebiet der Herbizide und Pflanzenwachstumsregulatoren, beispielsweise der Herbizide zur Bekämpfung von Unkräutern und Ungräsern in Nutzpflanzenkulturen oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es (a) dass sie keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen oder ein toxikologisch ungünstiges Profil besitzen. Andere Wirkstoffe, die als Pflanzenwachstumsregulatoren bei einigen Nutzpflanzen eingesetzt werden können, führen bei anderen Nutzpflanzen zu unerwünscht verminderten Ernteerträgen oder sind mit der Kulturpflanze nicht oder nur in einem engen Aufwandmengenbereich verträglich. Andere bekannte Wirkstoffe lassen sich wegen schwer zugänglicher Vorprodukte und Reagenzien im industriellen Maßstab nicht wirtschaftlich herstellen oder besitzen nur unzureichende chemische Stabilitäten.
Bei anderen Wirkstoffen hängt die Wirkung zu stark von Umweltbedingungen, wie Wetter- und Bodenverhältnissen ab.

Aus EP-A-0822187 und dort zitierter Literatur sind herbizide 3-(Hetero)Aryl-4-[(hetero)aryl-carbonyl]-pyrazolverbindungen bekannt.

Aus US 4146721 sind Pyrazolylessigsäuren als Analgetika, Antipiretika und Entzündungshemmer bekannt, jedoch ist keine Anwendung als Pestizide, besonders Herbizide beschrieben.

In US 4,095,025 sind 1,3-Diaryl-pyrazol-4-acrylsäure(derivate) für pharmazeutische (z.B. entzündungshemmende) Zwecke beschrieben.

WO 2004/089931 beschreibt substituierte Pyrazole mit gegebenenfalls substituierten Phenyl- oder Pyrid-3-yl-Resten am N-Atom in Position 1 des Pyrazols für die Behandlung und Prophylaxe von Krankheiten, die durch die Bindung der Verbindungen an 5 HT Rezeptoren beeinflusst werden.

WO2008080504 beschreibt substituierte 1-(3-Pyridinyl)pyrazol-4-yl essigsäuren als Herbizide und Pflanzenwachstumsregulatoren.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.

Gegenstand der vorliegenden Erfindung sind Verbindungen der Formel (I) oder deren Salze, 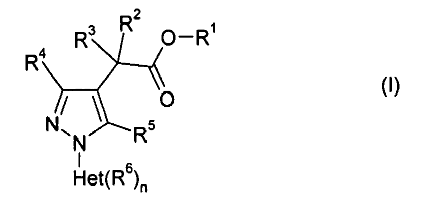 in welcher
- Het: einen fünfgliedrigen heteroaromatischen Rest mit zwei Heteroatomen als Ringatome, wobei eines der Heteroatome im Ring ein Stickstoffatom und das andere ein Schwefelatom ist und das Stickstoffatom im Ring in 1,3-Stellung zum Ring-C-atom steht, das am Pyrazolrest gebunden ist,
- R¹: Wasserstoff oder einen hydrolysierbaren Rest, vorzugsweise Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C- Atome, vorzugsweise 1 bis 24 C-Atome, insbesondere 1 bis 20 C-Atome aufweist, oder
einen Rest der Formel SiR^{a}R^{b}R^{c}, -NR^{a}R^{b} oder -N=CR^{c}R^{d},
wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C- Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten, wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
- R²: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
- R³: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
- R² und R³: zusammen mit dem C-Atom, an das sie gebunden sind, einen carbocyclischen gesättigten oder teilweise ungesättigten Ring mit 3 bis 6 C- Atomen, der unsubstituiert oder durch einen oder mehrere Reste aus der Gruppe Halogen und (C₁-C₄)Alkyl substituiert ist, und
- R⁴: Wasserstoff, Halogen, Cyano, (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, Cyano, Hydroxy, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist oder vorzugsweise unsubstituiert oder durch einen oder mehrere Reste aus der Gruppe bestehend aus Halogen, Cyano, (C₁-C₄)Alkoxy , (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
- R⁵: einen Arylrest, der unsubstituiert oder vorzugsweise substituiert ist und inklusive Substituenten 6 bis 30 C-Atome, vorzugsweise 6 bis 24 C- Atome, insbesondere 6 bis 20 C-Atome aufweist, oder
einen heteroaromatischen Rest mit 1 bis 4 Heteroringatomen aus der Gruppe N, O und S, der unsubstituiert oder substituiert ist und inklusive Substituenten 1 bis 30 C-Atome, vorzugsweise 1 bis 24 C-Atome, insbesondere 1 bis 20 C-Atome aufweist, und
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio- (C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
- n: 0, 1, oder 2 bedeuten.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze.

In der Formel (I) und allen nachfolgenden Formeln werden Bezeichnungen für chemische Reste verwendet, die insbesondere die nachstehend erläuterten Bedeutungen haben.

Ein hydrolysierbarer Rest (siehe Definition von R¹) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit der Carbonsäureestergruppe -CO-OR¹ (R¹ ungleich Wasserstoff) zur Verbindung der Formel (I) mit der Carbonsäuregruppe -CO-OH (d. h. Verbindung (I) mit R¹ = H) hydrolysiert wird. In der Definition der hydrolysierbaren Reste sind auch ausdrücklich die Reste mit R¹ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf.

Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen. Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl.

Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.
(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfaßt, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.
Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.
Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.
Im Falle von gegebenenfalls substituiertem Heterocyclyl werden auch mehrcyclische Systeme umfaßt, wie beispielsweise 8-Aza-bicyclo[3.2.1]octanyl oder 1-Azabicyclo[2.2.1]heptyl.
Im Falle von gegebenenfalls substituiertem Heterocyclyl werden auch spirocyclische Systeme umfaßt, wie beispielsweise 1-Oxa-5-aza-spiro[2.3]hexyl.

Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl;
weiterhin bevorzugt ist er ein Rest eines entsprechenden heteroaromatischen Rings mit 2, 3 oder 4 Heteroatomen, z. B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.

Bevorzugt ist er dabei ein Rest eines heteroaromatischen Fünf- oder Sechsrings mit 1 bis 4 Heteroatomen, wie z. B. 1,2,3-Triazolyl, 1 ,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.
Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl;
weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl;
weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl,
weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl;
weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl,
weiter bevorzugt sind auch heteroaromatische Reste von Sechsring-Heterocyclen, wie 1,2,4,5-Tetrazin-3-yl;
weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl;
weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazin-5-yl.

Weiterhin bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl, Isochinolyl, Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Mit "Basisrest" wird der jeweilige Grundkörper eines Restes bezeichnet, an dem Substituenten einer Substituentenebene gebunden sind.

### Bevorzugt Substituenten für die Substituentenebenen sind beispielsweise

Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.
Zwei Substituenten auch gemeinsam eine gesättigte oder ungesättigte Kohlenwasserstoff-Brücke oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.
Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.
Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.
Weiter bevorzugt bedeutet Acyl einen Alkanoylrest mit 1 bis 6 C-Atomen, insbesondere 1 bis 4 C-Atomen. (C₁-C₄)Alkanoyl bedeutet dabei den Rest einer Alkansäure mit 1 bis 4 C-Atomen nach Abtrennen der OH-Gruppe der Säuregruppe, d.h. Formyl, Acetyl, n-Propionyl, i-Propionyl oder n-, i-, sec. oder tert.-Butanoyl.

Die "yl-Position" eines Restes bezeichnet das C-Atom mit der freien Bindung. Erfindungsgemäße bzw. erfindungsgemäß verwendete Verbindungen der Formel (I) (und ggf deren Salze) werden kurz auch als "Verbindungen (I)" bezeichnet.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe R¹, R², R³, R⁴, R⁵ und (R⁶)ₙ und der den allgemeinen Resten entsprechenden Unterbedeutungen, und vorzugsweise in Kombination mit bevorzugten Bedeutungen von einem oder mehreren dieser Reste, sind erfindungsgemäße Verbindungen bzw. erfindungsgemäße Verwendungen von Verbindungen mit nachfolgend aufgeführten bevorzugten Bedeutungen der betreffenden Reste von besonderem Interesse.

Bevorzugt sind die erfindungsgemäßen Verbindungen der Formel (I) oder deren Salze, in welcher
- R¹: Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome, vorzugsweise bis 24 C- Atome, insbesondere bis 20 C-Atome aufweist, oder einen Heterocyclylrest mit 3 bis 9 Ringatomen, der 1 bis 4 Heteroatome aus der Gruppe N, O und S enthält und der unsubstituiert oder oder substituiert ist und der inklusive Substituenten 1 bis 30 C-Atome, vorzugsweise 1 bis 24 C- Atome, insbesondere 1 bis 20 C-Atome aufweist, bedeutet.

Weiter bevorzugt sind dabei auch Verbindungen (I) oder deren Salze, worin
- R¹: Wasserstoff bedeutet.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder substituiert ist und inklusive Substituenten bis zu 30 C-Atome, vorzugsweise bis 24 C-Atome, insbesondere bis 20 C-Atome aufweist.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl,
wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR^{*}R^{**}, -CO-NR^{*}R^{**} und -O-CO-NR^{*}R^{**},
wobei jeder der Reste R^{*} und R^{**} in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]- carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]- carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]- carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R"' H oder (C₁-C₄)Alkyl bedeuten,
substituiert ist,
bedeutet.

Weiter bevorzugt sind dabei auch Verbindungen (I) oder deren Salze, worin
- R¹: H, (C₁-C₁₂)Alkyl, (C₂-C₁₂)Alkenyl, (C₂-C₁₂)Alkinyl, (C₃-C₆)Cycloalkyl, (C₅-C₆)Cycloalkenyl, (C₅-C₆)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, die letztgenannten 7 nur im Falle cyclischer Basisreste, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, Reste der Formel -NR^{*}R^{**}, -CO-NR^{*}R^{**} und -O-CO-NR^{*}R^{**},
wobei jeder der Reste R^{*} und R^{**} in den letztgenannten 3 Formeln unabhängig voneinander H, (C₁-C₄)Alkyl, (C₂-C₄)Alkenyl, (C₂-C₄)Alkinyl, Benzyl, Phenyl, das unsubstituiert oder durch einen oder mehrere Reste aus der Gruppe Halogen, (C₁-C₄)Alkyl, (C₁-C₄)Haloalkyl und (C₁-C₄)Alkoxy substituiert ist, oder zusammen mit dem N-Atom einen Piperidin-, Piperazin-, Pyrrolidin-, Pyrazolidin-, Piperazolidin- oder Morpholinrest, welcher unsubstituiert oder durch einen oder mehrere Reste aus der Gruppe (C₁-C₄)Alkyl und (C₁-C₄)Haloalkyl substituiert ist, bedeutet,
und [(C₁-C₄)Alkoxy]-carbonyl, [(C₁-C₄)Alkyl]-carbonylamino, [(C₁-C₄)Alkoxy]- carbonylamino, [(C₁-C₄Alkylamino]-carbonylamino, [(C₁-C₄)Alkyl]-carbonyloxy, [(C₁-C₄)Alkoxy]-carbonyloxy und (C₁-C₄)Alkylsulfonyl,
wobei jeder der letztgenannten 7 Reste unsubstituiert oder durch einen oder mehrere Reste aus der Gruppe Halogen, NO₂, (C₁-C₄)Alkoxy und Phenyl, das unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Phenyl, Phenyl-(C₁-C₄)alkoxy, Phenyl-[(C₁-C₄)alkoxy]-carbonyl, Phenoxy, Phenoxy-(C₁-C₄)alkoxy, Phenoxy-[(C₁-C₄)alkoxy]-carbonyl, Phenoxycarbonyl, Phenylcarbonyloxy, Phenylcarbonylamino, Phenyl-[(C₁-C₄)alkyl]- carbonylamino, Phenyl-[(C₁-C₄)alkyl]-carbonyloxy, (C₃-C₆)Cycloalkyl und (C₃-C₆)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -CO-O-NR'₂, -O-N=CR'₂, -N=CR'₂, -O-NR'₂, -CH(OR')₂
und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₄)Alkylengruppe bedeuten und R"' H oder (C₁-C₂)Alkyl bedeuten,
substituiert ist,
bedeutet.

Weiter bevorzugt sind dabei auch Verbindungen (I) oder deren Salze, worin
- R¹: H, (C₁-C₆)Alkyl, (C₂-C₆)Alkenyl, (C₂-C₆)Alkinyl oder (C₃-C₆)Cycloalkyl, wobei jeder der letztgenannten 4 Reste unsubstituiert oder durch einen oder mehrere Reste aus der Gruppe Halogen, (C₁-C₆)Alkyl, letzterer nur Substituent im Fall cyclischer Basisreste, (C₁-C₄)Alkoxy, (C₁-C₄)Alkylthio, (C₃-C₆)Cycloalkyl, das unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Besonders bevorzugt sind dabei auch Verbindungen (I) oder deren Salze, worin
- R¹: H, (C₁-C₄)Alkyl, (C₂-C₄)Alkenyl oder (C₂-C₄)Alkinyl, wobei jeder der letztgenannten 3 Reste unsubstituiert oder durch einen oder mehrere Reste aus der Gruppe Halogen, (C₁-C₄)Alkoxy, (C₁-C₄)Alkylthio, Cyclopropyl, Cyclobutyl, wobei jeder der letztgenannten beiden Reste unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist,
substituiert ist,
bedeutet, ganz besonders
- R¹: H, Methyl, Ethyl, n-Propyl, i-Propyl, Phenyl, Benzyl, CH₂(4-Cl-Ph), CH₂(4-F- Ph), CH₂(4-OMe-Ph), 2-Methoxyethyl, Tetrahydrofuran-2-yl-methyl, 2- (Dimethylamino)ethyl, Oxetan-3-yl, (3-Methyloxetan-3-yl)methyl, 2,2,2- Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2- Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3- Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1- yl, 4-Chlor-but-2-in-1-yl, 3-Methyl-but-2-en-1-yl, 3-Methyl-but-1-en-1-yl, 1- (2E)-1-methylbut-2-en-1-yl, (E)-Pent-3-en-2-yl oder (Z)-Pent-3-en-2-yl, Cyclobutylmethyl, Cyclopentylmethyl, Cyclohexylmethyl oder 1-Ethyl-5- methyl-1H-pyrazol-4-methyl
bedeutet.

Bevorzugt sind auch Verbindungen (I) oder deren Salze, worin
- R¹: einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält und der unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy- (C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Bevorzugt sind auch Verbindungen (I) oder deren Salze, worin
- R¹: einen Rest der Formel SiR^{a}R^{b}R^{c}, -NR^{a}R^{b} oder -N=CR^{c}R^{d}, vorzugsweise der Formel -NR^{a}R^{b} oder -N=CR^{c}R^{d},
wobei in den letztgenannten 5 Formeln jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet oder R^{a} und R^{b} zusammen mit dem N-Atom einen 3- bis 8- gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten,
bedeutet.

Bevorzugt sind auch Verbindungen der Formel (I) oder deren Salze, worin
- R²: Wasserstoff, Halogen oder (C₁-C₄)Alkyl, das unsubstituiert oder durch einen oder mehrere Reste aus der Gruppe Halogen, wie Fluor oder Chlor substituiert ist, vorzugweise Wasserstoff oder (C₁-C₄)Alkyl, insbesondere Wasserstoff, Methyl oder Ethyl, ganz besonders Wasserstoff oder Methyl
bedeutet.

Bevorzugt sind auch die Verbindungen der Formel (I) oder deren Salze, worin
- R³: Wasserstoff, Halogen oder (C₁-C₄)Alkyl, das unsubstituiert oder durch einen oder mehrere Reste aus der Gruppe Halogen, wie Fluor oder Chlor, substituiert ist, vorzugweise Wasserstoff oder (C₁-C₄)Alkyl, insbesondere Wasserstoff oder Methyl, ganz besonders Wasserstoff
bedeutet.

Bevorzugt sind auch die Verbindungen der Formel (I) oder deren Salze, worin R² und R³ zusammen mit dem C-Atom, an das sie gebunden sind, (C₃-C₆)Cycloalkyl oder (C₅-C₆)Cycloalkenyl, vorzugsweise (C₃-C₆)Cycloalkyl, wobei jeder der letztgenannten 3 Reste unsubstituiert oder durch einen oder mehrere Reste aus der Gruppe Halogen und (C₁-C₄)Alkyl substituiert ist, bedeuten.
Bevorzugt bedeuten dabei R² und R³ zusammen mit dem C-Atom, an das sie gebunden sind, Cyclopropyl, Cyclobutyl, Cyclopentyl oder Cyclohexyl, insbesondere Cyclopropyl, wobei jeder der letztgenannten 4 Reste unsubstituiert oder durch einen oder mehrere Reste aus der Gruppe Halogen und Methyl, vorzugsweise Fluor, Chlor und Methyl substituiert ist.

Bevorzugt sind auch die Verbindungen der Formel (I) oder deren Salze, worin
- R⁴: Wasserstoff, Halogen, Cyano, (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen und Hydroxy substituiert ist, vorzugsweise unsubstituiert oder durch einen oder mehrere Reste aus der Gruppe Halogen, wie Fluor und Chlor substituiert ist, oder (C₃-C₆)Cycloalkyl, das unsubstituiert oder durch einen oder mehrere Reste aus der Gruppe bestehend aus Halogen und (C₁-C₄)Alkyl substituiert ist, oder Phenyl, das unsubstituiert oder durch einen oder mehrere Reste aus der Gruppe bestehend aus Halogen, Nitro, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]- carbonyl substituiert ist, oder (C₁-C₄)Alkanoyl, das unsubstituiert oder durch einen oder mehrere Reste aus der Gruppe bestehend aus Halogen, wie Fluor und Chlor, Cyano, (C₁-C₄)Alkoxy, (C₁-C₄)Haloalkoxy und (C₁-C₂)Alkoxy-(C₁-C₂)alkoxy substituiert ist, vorzugsweise Formyl, oder [(C₁-C₄)Alkoxy]-carbonyl, das unsubstituiert oder durch einen oder mehrere Reste aus der Gruppe bestehend aus Halogen, wie Fluor und Chlor, substituiert ist, oder [(C₃-C₆)Cycloalkoxy]-carbonyl, das unsubstituiert oder durch einen oder mehrere Reste aus der Gruppe bestehend aus Halogen und (C₁-C₄)Alkyl substituiert ist,
bedeutet.

Weiter bevorzugt sind auch die Verbindungen der Formel (I) oder deren Salze, worin
- R⁴: Wasserstoff, Halogen, wie Fluor oder Chlor, Cyano, (C₁-C₄)Alkyl, das gegebenenfalls durch Hydroxy substituiert ist [= (C₁-C₄)Hydroxyalkyl], (C₁- C₄)Haloalkyl, Cyclopropyl oder Cyclobutyl, wobei jeder der letztgenannten beiden Reste unsubstituiert oder durch einen oder mehrere Reste aus der Gruppe bestehend aus Halogen, wie Fluor und Chlor, und (C₁-C₄)Alkyl substituiert ist, oder
Phenyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy und (C₁-C₄)Alkylthio substituiert ist, oder
(C₁-C₄)Alkanoyl, das unsubstituiert oder durch einen oder mehrere Reste aus der Gruppe bestehend aus Halogen, wie Fluor und Chlor, substituiert ist, vorzugsweise Formyl, oder
[(C₁-C₄)Alkoxy]-carbonyl oder [(C₁-C₄)Haloalkoxy]-carbonyl
bedeutet, vorzugsweise
- R⁴: Wasserstoff, Halogen, wie Fluor oder Chlor, Cyano, Methyl, Ethyl, n-Propyl, i- Propyl, CH₂Cl, CHCl₂, CCl₃, CH₂F, CHF₂,CF₃ oder Formyl
bedeutet.

Weiter bevorzugt sind dabei die genannten bevorzugten bzw. besonders bevorzugten Verbindungen (I) oder deren Salze, worin R⁴ Cyano bedeutet oder worin R⁴ für eine der anderen genannten Bedeutungen als Cyano oder Formyl steht.

Weiter bevorzugt ist R⁴ eine der für R⁴ genannten und von Wasserstoff verschiedenen Reste.

Weiter bevorzugt ist R⁴ Wasserstoff.

Bevorzugt sind auch die Verbindungen der Formel (I) oder deren Salze, worin
- R⁵: Phenyl, das unsubstituiert oder vorzugsweise substituiert ist und inklusive Substituenten 6 bis 24 C-Atome, insbesondere 6 bis 20 C-Atome aufweist, oder
einen 5- oder 6-gliedrigen heteroaromatischen Rest mit 1 bis 3 Heteroringatomen aus der Gruppe N, O und S, der unsubstituiert oder substituiert ist und inklusive Substituenten 1 bis 24 C-Atome, insbesondere 1 bis 20 C-Atome aufweist,
bedeutet.

Weiter bevorzugt sind auch die Anwendungen von Verbindungen der Formel (I) oder deren Salze, worin
- R⁵: Phenyl, das unsubstituiert oder vorzugsweise durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di- [(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, das gegebenenfalls auch halogeniert sein kann [= (C₁-C₆)Haloalkoxy)], (C₁-C₄)Alkoxy- (C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di- [(C₁-C₆)acyl]-amino, Mono- und Di-[(C₁-C₄)alkyl]-amino, N- [(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist, oder
- R⁵: einen 5- oder 6-gliedrigen heteroaromatischen Rest mit 1 bis 3 Heteroringatomen aus der Gruppe N, O und S, der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy- (C₁-C₄)alkyl, (C₁-C₄)Alkylthio-(C₁-C₄)alkyl, Mono- und Di-[(C₁-C₄)alkyl]- aminoalkyl, Hydroxy-(C₁-C₄)alkyl, Carboxy-(C₁-C₄)alkyl, Cyano- (C₁-C₄)alkyl, (C₁-C₆)Alkoxy, das gegebenenfalls auch halogeniert sein kann [= (C₁-C₆)Haloalkoxy], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]- carbonyl, (C₁-C₆)Alkanoyl, (C₁-C₆)Haloalkanoyl, Mono- und Di- [(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
bedeutet.

Weiter bevorzugt sind dabei auch die Verbindungen der Formel (I) oder deren Salze, worin
- R⁵: Phenyl, das unsubstituiert oder vorzugsweise durch einen oder mehrere Reste aus der Gruppe bestehend aus Halogen, Hydroxy, Nitro, Carboxy, Cyano, (C₁-C₄)Alkyl, (C₁-C₄)Haloalkyl, (C₁-C₄)Alkoxy- (C₁-C₄)alkyl, (C₁-C₄)Alkylthio-(C₁-C₄)alkyl, Hydroxy-(C₁-C₄)alkyl, (C₁- C₄)Alkoxy, das gegebenenfalls auch halogeniert sein kann [= (C₁- C₄)Haloalkoxy], (C₁-C₄)Alkoxy-(C₁-C₄)alkoxy, (C₁-C₄)Alkylthio, [(C₁- C₄)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Carboxy, Cyano, (C₁-C₄)Alkyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, das gegebenenfalls auch halogeniert sein kann [= (C₁-C₄)Haloalkoxy], (C₁-C₄)Alkoxy-(C₁-C₄)alkoxy, (C₁-C₄)Alkylthio, [(C₁-C₆)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl und (C₃-C₆)Cycloalkyl, das unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist,
bedeutet.

Noch weiter bevorzugt sind dabei auch die Verbindungen der Formel (I) oder deren Salze, worin
- R⁵: Phenyl, das unsubstituiert oder vorzugsweise durch einen oder mehrere Reste aus der Gruppe bestehend aus Halogen, Cyano, (C₁-C₄)Alkyl, (C₁-C₄)Haloalkyl, (C₁-C₄)Alkoxy und (C₁-C₄)Alkylthio substituiert und gegebenenfalls benzokondensiert ist, oder
- R⁵: einen 5- oder 6-gliedrigen heteroaromatischen Rest mit 1 bis 3 Heteroringatomen aus der Gruppe N, O und S, der unsubstituiert oder durch einen oder mehrere Reste aus der Gruppe bestehend aus Halogen, Hydroxy, (C₁-C₄)Alkyl, (C₁-C₄)Haloalkyl, (C₁-C₄)Alkoxy und (C₁-C₄)Alkylthio substituiert und gegebenenfalls benzokondensiert oder benzoannelliert ist,
bedeutet.

Besonders bevorzugt sind dabei Verbindungen der Formel (I) oder deren Salze, worin
- R⁵: Phenyl, das unsubstituiert oder vorzugsweise durch einen oder mehrere Reste aus der Gruppe bestehend aus Halogen, wie Fluor, Chlor, Brom und Iod, Cyano, Methyl, Ethyl, n-Propyl, i-Propyl, n-Butyl, sec-Butyl, i- Butyl, t-Butyl, Trifluormethyl, Trichlormethyl, Methoxy und Ethoxy substituiert und gegebenenfalls benzokondensiert ist,
bedeutet, vorzugsweise
- R⁵: Phenyl, 2-Fluorphenyl, 2-Chlorphenyl, 2-Bromphenyl, 2-lodphenyl, 2-Methylphenyl, 4-(tert.-Butyl)-phenyl, 2-Trifluormethyl-phenyl, 2- Methoxyphenyl, 3-Fluorphenyl, 3-Chlorphenyl, 3-Bromphenyl, 3-lodphenyl, 3- Methylphenyl, 3-Trifluormethyl-phenyl, 3-Methoxyphenyl, 4-Carboxy-phenyl, 4-Fluorphenyl, 4-Chlorphenyl, 4-Bromphenyl, 4-lodphenyl, 4-Methylphenyl, 4-Trifluormethyl-phenyl, 4-Methoxyphenyl, 2,3-Dichlorphenyl, 2,3-Dimethylphenyl, 2,4-Dichlorphenyl, 2,4-Dimethylphenyl, 2,5-Dichlorphenyl, 2,5-Dimethylphenyl, 2,6-Dichlorphenyl, 2,6-Dimethylphenyl, 3,4-Dichlorphenyl, 3,4-Dimethylphenyl, 3,5-Dichlorphenyl, 3,5-Dimethylphenyl, 2-Chlor-3-methylphenyl, 2-Chlor-4- methylphenyl, 2-Chlor-5-methylphenyl, 2-Chlor-6-methylphenyl, 3-Chlor-4- methylphenyl, 3-Chlor-5-methylphenyl, 3-Chlor-2-methylphenyl, 4-Chlor-2- methylphenyl, 4-Chlor-3-methylphenyl oder 5-Chlor-2-methylphenyl, 4- Phenyl-phenyl, 3-Trifluormethyl-4-chlor-phenyl, 4-Phenoxy-phenyl, 4- Carboxymethyl-phenyl, 4-Acetyl-phenyl oder 1,3-Benzodioxol-5-yl
bedeutet.

Bevorzugt sind dabei auch Verbindungen der Formel (I) oder deren Salze, worin
- R⁵: 2-Pyridyl, 3-Pyridyl, 4-Pyridyl, 2-Thienyl, 3-Thienyl, 2-Furyl, 3-Furyl, 2-Pyrrolyl, 3-Pyrrolyl, 2-Pyrimidinyl, 4-Pyrimidinyl, 5-Pyrimidinyl, 3-Pyridazinyl, 4-Pyridazinyl, 2-Pyrazinyl, 3-Pyrazinyl, 2-Imidazolinyl, 4-Imidazolinyl, 1- Pyrazolyl, 3-Pyrazolyl, 4-Pyrazolyl, 5-Pyrazolyl, 1,3,5-Triazin-2-yl, 1,2,4- Triazin-3-yl, 1,2,4-Triazin-5-yl, 2-Thiazolyl, 2-(1,3-Benzothiazolyl), Isochinolin- 3-yl, Chinolin-2-yl, 4-Thiazolyl, 5-Thiazolyl, 3-Isothiazolyl, 4-Isothiazolyl, 5-Isothiazolyl, 2-Oxazolyl, 4-Oxazolyl, 5-Oxazolyl, 3-Isoxazolyl, 4-Isoxazolyl, 5-Isoxazolyl, Thiadiazolyl oder Triazolyl, vorzugsweise 2-Pyridyl, 3-Pyridyl, 4- Pyridyl, 2-Pyrimidinyl, 2-Pyrazinyl, 2-Thienyl, 3-Thienyl, 2-Furyl oder 2- Thiazolyl, wobei jeder der vorstehend genannten heteroaromatischen Reste unsubstituiert oder substituiert ist, vorzugsweise durch die oben bereits bevorzugt genannten Reste substituiert ist, insbesondere durch einen oder mehrere Reste aus der Gruppe bestehend aus Halogen, Hydroxy, (C₁-C₄)Alkyl, (C₁-C₄)Haloalkyl, (C₁-C₄)Alkoxy, (C₁-C₄)Alkylthio substituiert ist,
bedeutet.

Besonders bevorzugt sind dabei auch Verbindungen der Formel (I) oder deren Salze, worin
- R⁵: 2-Pyridyl, 3-Fluor-pyrid-2-yl, 3-Chlor-pyrid-2-yl, 3-Brom-pyrid-2-yl, 3-Methyl- pyrid-2-yl, 3-Methoxy-pyrid-2-yl, 3-Trifluormethyl-pyrid-2-yl, 4-Fluor-pyrid-2-yl, 4-Chlor-pyrid-2-yl, 4-Brom-pyrid-2-yl, 4-Methyl-pyrid-2-yl, 4-Methoxy-pyrid-2- yl, 4-Trifluormethyl-pyrid-2-yl, 5-Fluor-pyrid-2-yl, 5-Chlor-pyrid-2-yl, 5-Brom- pyrid-2-yl, 5-Methyl-pyrid-2-yl, 5-Methoxy-pyrid-2-yl, 5-Trifluormethyl-pyrid-2- yl, 6-Fluor-pyrid-2-yl, 6-Chlor-pyrid-2-yl, 6-Brom-pyrid-2-yl, 6-Methyl-pyrid-2-yl, 6-Methoxy-pyrid-2-yl, 6-Trifluormethyl-pyrid-2-yl, 4,6-Dimethyl-pyridin-2-yl, 3-Pyridyl, 2-Fluor-pyrid-3-yl, 2-Chlor-pyrid-3-yl, 2-Brom-pyrid-3-yl, 2-Methyl- pyrid-3-yl, 2-Methoxy-pyrid-3-yl, 2-Trifluormethyl-pyrid-3-yl, 4-Fluor-pyrid-3-yl, 4-Chlor-pyrid-3-yl, 4-Brom-pyrid-3-yl, 4-Methyl-pyrid-3-yl, 4-Methoxy-pyrid-3- yl, 4-Trifluormethyl-pyrid-3-yl, 5-Fluor-pyrid-3-yl, 5-Chlor-pyrid-3-yl, 5-Brom- pyrid-3-yl, 5-Methyl-pyrid-3-yl, 5-Methoxy-pyrid-3-yl, 5-Trifluormethyl-pyrid-3- yl, 6-Fluor-pyrid-3-yl, 6-Chlor-pyrid-3-yl, 6-Brom-pyrid-3-yl, 6-Methyl-pyrid-3-yl, 6-Methoxy-pyrid-3-yl, 6-Trifluormethyl-pyrid-3-yl, 6-Hydroxy-pyridin-3-yl, 4-Pyridyl, 2-Fluor-pyrid-4-yl, 2-Chlor-pyrid-4-yl, 2-Brom-pyrid-4-yl, 2-Methyl- pyrid-4-yl, 2-Methoxy-pyrid-4-yl, 2-Trifluormethyl-pyrid-4-yl, 3-Fluor-pyrid-4-yl, 3-Chlor-pyrid-4-yl, 3-Brom-pyrid-4-yl, 3-Methyl-pyrid-3-yl, 3-Methoxy-pyrid-4- yl, 3-Trifluormethyl-pyrid-4-yl,
2-Thienyl, 3-Fluor-thien-2-yl, 3-Chlor-thien-2-yl, 3-Brom-thien-2-yl, 3-Methyl- thien-2-yl, 3-Methoxy-thien-2-yl, 3-Trifluormethyl-thien-2-yl, 4-Fluor-thien-2-yl, 4-Chlor-thien-2-yl, 4-Brom-thien-2-yl, 4-Methyl-thien-2-yl, 4-Methoxy-thien-2- yl, 4-Trifluormethyl-thien-2-yl, 5-Fluor-thien-2-yl, 5-Chlor-thien-2-yl, 5-Brom- thien-2-yl, 5-lod-2-thienyl, 5-Methyl-thien-2-yl, 5-Methoxy-thien-2-yl, 5- Trifluormethyl-thien-2-yl,
3-Thienyl, 2-Fluor-thien-3-yl, 2-Chlor-thien-3-yl, 2-Brom-thien-3-yl, 2-Methyl- thien-3-yl, 2-Methoxy-thien-3-yl, 2-Trifluormethyl-thien-3-yl, 4-Fluor-thien-3-yl, 4-Chlor-thien-3-yl, 4-Brom-thien-3-yl, 4-Methyl-thien-3-yl, 4-Methoxy-thien-3- yl, 4-Trifluormethyl-thien-3-yl, 5-Fluor-thien-3-yl, 5-Chlor-thien-3-yl, 5-Brom- thien-3-yl, 5-Methyl-thien-3-yl, 5-Methoxy-thien-3-yl, 5-Trifluormethyl-thien-3- yl,
2-Furyl, 3-Fluor-fur-2-yl, 3-Chlor-fur-2-yl, 3-Brom-fur-2-yl, 3-Methyl-fur-2-yl, 3- Methoxy-fur-2-yl, 3-Trifluormethyl-fur-2-yl, 4-Fluor-fur-2-yl, 4-Chlor-fur-2-yl, 4- Brom-fur-2-yl, 4-Methyl-fur-2-yl, 4-Methoxy-fur-2-yl, 4-Trifluormethyl-fur-2-yl, 5-Fluor-fur-2-yl, 5-Chlor-fur-2-yl, 5-Brom-fur-2-yl, 5-Methyl-fur-2-yl, 5-Methoxy- fur-2-yl oder 5-Trifluormethyl-fur-2-yl,
2-Thiazolyl, 4-Me-thiazol-2-yl, 5-Methyl-thiazol-2-yl, 5-Brom-thiazol-2-yl, 5- Chlor-thiazol-2-yl, 4,5-Dimethyl-thiazol-2-yl, 4,5-Dichlor-thiazol-2-yl, 2-(1,3-Benzothiazolyl), 7-Chlor-(1,3-benzothiazol-2-yl), Isochinolin-3-yl, Chinolin-2-yl,
2-Pyrazinyl, 5-Methyl-pyrazin-2-yl,
1,5-Dimethyl-pyrazol-3-yl,
2-Pyrimidinyl, 5-F-pyrimidin-2-yl, 5-Chlor-pyrimidin-2-yl, 5-Brom-pyrimidin-2-yl,
5-Methyl-pyrimidin-2-yl, 4,6-Dimethyl-pyrimidin-2-yl,
3-Pyridazinyl, 6-Methyl-pyridazin-3-yl, 3-(1,2,4)-triazinyl, 6-Methyl-(1,2,4)-triazin-3-yl
bedeutet, vorzugsweise
- R⁵: 2-Pyridyl, 5-Fluor-pyrid-2-yl, 5-Chlor-pyrid-2-yl, 5-Brom-pyrid-2-yl, 5- Methyl-pyrid-2-yl, 5-Methoxy-pyrid-2-yl, 5-Trifluormethyl-pyrid-2-yl, 3-Pyridyl, 6-Fluor-pyrid-3-yl, 6-Chlor-pyrid-3-yl, 6-Brom-pyrid-3-yl, 6-Methyl-pyrid-3-yl, 6- Methoxy-pyrid-3-yl oder 6-Trifluormethyl-pyrid-3-yl, 4,6-Me₂-pyridin-2-yl,2- Thienyl, 3-Chlor-thien-2-yl, 3-Methyl-thien-2-yl, 4-Chlor-thien-2-yl, 4-Methyl- thien-2-yl, 5-Chlor-thien-2-yl, 5-Brom-thien-2-yl, 5-lod-2-thienyl, 5-Methyl- thien-2-yl 2-Thiazolyl, 5-Brom-thiazol-2-yl, 5-Chlor-thiazol-2-yl, 4,5-Dimethyl- thiazol-2-yl, 4,5-Dichlor-thiazol-2-yl, 2-(1,3-Benzothiazolyl), Isochinolin-3-yl, Chinolin-2-yl, 2-Pyrazinyl, 5-Methyl-pyrazin-2-yl, 1,5-Dimethyl-pyrazol-3-yl, 2- Pyrimidinyl, 5-Brom-pyrimidin-2-yl, 5-Methyl-pyrimidin-2-yl, 4,6-Dimethyl- pyrimidin-2-yl, 3-Pyridazinyl, oder 6-Methyl-pyridazin-3-yl oder auch 4-Fluor-pyrid-2-yl, 4-Chlor-pyrid-2-yl, 4-Brom-pyrid-2-yl, 4-Methyl- pyrid-2-yl oder 4-Trifluor-pyrid-2-yl
bedeutet.

Bevorzugt sind auch die Verbindungen der Formel (I) oder deren Salze, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, Cyano, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl oder (C₁-C₄)Haloalkylsulfonyl, (C₁-C₄)Alkoxycarbonyl bedeutet, und
- n: 0, 1, oder 2, vorzugweise 0 oder 1
bedeuten.

Bevorzugt sind dabei auch die Verbindungen der Formel (I) oder deren Salze, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, wie Fluor, Chlor, Brom oder Iod, Methyl, Ethyl, Trifluormethyl, Methoxy, Ethoxy, Methylthio, Ethylthio, Methylsulfinyl, Ethylsulfinyl, Methylsulfonyl, Ethylsulfonyl, Methoxycarbonyl oder Ethoxycarbonyl bedeutet, und
- n: 0, 1, oder 2, vorzugweise 0 oder 1
bedeuten.

Bevorzugt sind Verbindungen der Formel (I) oder deren Salzen, worin Het einen Rest der Formel (Het-a), (Het-b) oder (Het-c) 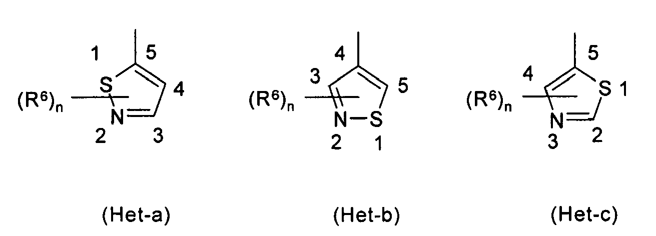 bedeutet und darin (R⁶)ₙ die genannte bzw. bevorzugt genannte Bedeutung haben.

Weiter bevorzugt sind Verbindungen der Formel (I) oder deren Salze, worin
- Het: den genannten Rest (Het-a) bedeutet, worin
- n: 0 (= die Zahl Null, d. h. keine Substituenten R⁶ vorhanden, d. h. alle freien Bindungen am Ring sind mit Wasserstoff besetzt) bedeutet oder (R⁶)ₙ 3-Brom, 3-Chlor, 3-Fluor, 3-Cyano, 3-Methyl, 3-Ethyl, 3-CF₃, 3-Methoxy, 3- Ethoxy, 3-Methylthio, 3-Methylsulfinyl, 3-Methylsulfonyl, 4-Fluor, 4-Chlor, 4- Brom, 4-Cyano, 4-Methyl, 4-Ethyl, 4-CF₃, 4-Methoxy, 4-Ethoxy, 4-Methylthio, 4-Methylsulfinyl, 4-Methylsulfonyl, 3,4-Dimethyl, 3,4-Difluor, 3,4-Dichlor bedeutet,
wobei die Bezifferung der Reste sich auf die Position des Restes am Isothiazol-5-yl-Rest bezieht, in dem die S und N-Ringatome jeweils in 1- und 2-Position im Ring liegen.

Besonders bevorzugt sind dabei die Verbindungen der Formel (I) oder deren Salze, worin
- Het: den genannten Rest (Het-a) bedeutet, worin n = 0 bedeutet oder
- (R⁶)ₙ: 4-Fluor, 4-Chlor, 4-Methyl, 4-Trifluormethyl, 4-Methoxy, 4-Methylsulfonyl, 4- Methylsulfinyl oder 4-Methylthio, vorzugsweise 4-Methyl bedeutet.

Weiter bevorzugt sind Verbindungen der Formel (I) oder deren Salze, worin
- Het: den genannten Rest (Het-b) bedeutet, worin
- n: 0 (= die Zahl Null, d. h. keine Substituenten R⁶ vorhanden, d. h. alle freien Bindungen am Ring sind mit Wasserstoff besetzt) oder
- (R⁶)ₙ: 5-Fluor, 5-Chlor, 5-Brom, 5-Cyano, 5-Methyl, 5-Ethyl, 5-Trifluormethyl, 5- Methoxy, 5-Ethoxy, 5-Methylthio, 5-Methylsulfinyl, 5-Methylsulfonyl, 3-Fluor, 3-Chlor, 3-Brom, 3-Cyano, 3-Methyl, 3-Ethyl, 3-Trifluormethyl, 3- Methoxycarbonyl, 3-Methoxy, 3-Ethoxy, 3-Methylthio, 3-Methylsulfinyl, 3- Methylsulfonyl, 3,5-Dimethoxycarbonyl, 3,5-Dimethyl, 3,5-Difluor oder 3,5- Dichlor
bedeutet,
wobei die Bezifferung der Reste sich auf die Position des Restes am Isothiazol-4-yl-Rest bezieht, in dem die S und N-Ringatome jeweils in 1- und 2-Position im Ring liegen.

Besonders bevorzugt sind dabei die Verbindungen der Formel (I) oder deren Salze, worin
- Het: den genannten Rest (Het-b) bedeutet, worin n = 0 bedeutet oder
- (R⁶)ₙ: 5-Fluor, 5-Chlor, 5-Methyl, 5-Trifluromethyl, 5-Methoxy, 5-Methylsulfonyl, 5- Methylsulfinyl oder 5-Methylthio, vorzugsweise 5-Methyl bedeutet.

Weiter bevorzugt sind Verbindungen der Formel (I) oder deren Salze, worin
- Het: den genannten Rest (Het-c) bedeutet, worin
- n: 0 (= die Zahl Null, d. h. keine Substituenten R⁶ vorhanden, d. h. alle freien Bindungen am Ring sind mit Wasserstoff besetzt) oder
- (R⁶)ₙ: 2-Brom, 2-Chlor, 2-Fluor, 2-Cyano, 2-Methyl, 2-Ethyl, 2-CF₃, 2-Methoxy, 2- Ethoxy, 2-Methylthio, 2-Methylsulfinyl, 2-Methylsulfonyl, 4-Fluor, 4-Chlor, 4- Brom, 4-Cyano, 4-Methyl, 4-Ethyl, 4-CF₃, 4-Methoxy, 4-Ethoxy, 4-Methylthio, 4-Methylsulfinyl, 4-Methylsulfonyl, 4-Ethoxycarbonyl, 2,4-Dimethyl, 2,4-Difluor, 2,4-Dichlor bedeutet,
wobei die Bezifferung der Reste sich auf die Position des Restes am Thiazol-5-yl bezieht, in dem die S und N-Ringatome jeweils in 1- und 3-Position im Ring liegen.

Besonders bevorzugt sind dabei die Verbindungen der Formel (I) oder deren Salze, worin
- Het: den genannten Rest (Het-c) bedeutet, worin n = 0 bedeutet oder worin
- (R⁶)ₙ: 4-Fluor, 4-Chlor, 4-Methyl, 4-Trifluormethyl, 4-Methoxy, 4-Methylsulfonyl, 4- Methylsulfinyl oder 4-Methylthio, vorzugsweise 4-Methyl, bedeutet.

Bevorzugt sind auch die Verbindungen der Formel (I) oder deren Salze, worin Het den genannten Rest (Het-a), (Het-b), oder (Het-c), worin jeweils n = 0 ist, bedeutet.

Bevorzugt sind auch die Verbindungen der Formel (I) oder deren Salze, worin die Reste Het, R¹, R², R³, R⁴, R⁵, R⁶ und n gemäß zwei oder mehreren der genannten bevorzugten Bedeutungen ausgewählt worden sind.

Bevorzugt sind als Verbindungen der Formel (I) oder deren Salze die Verbindungen der Formel (la), (Ib), oder (Ic) oder deren Salze, 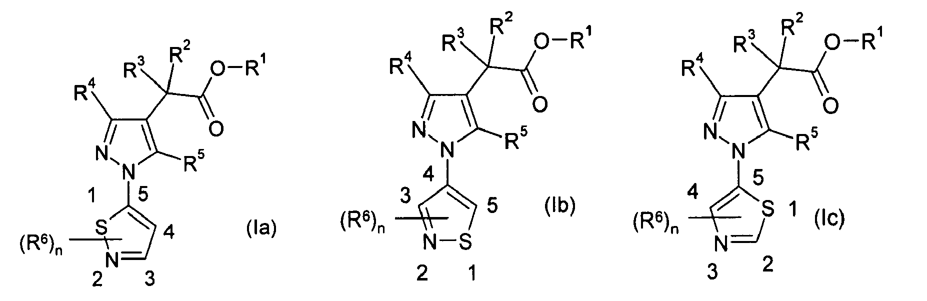 worin R¹, R², R³, R⁴, R⁵, R⁶ und n wie in Formel (I) bzw. gemäß den genannten bevorzugten Bedeutungen definiert sind.

Besonders bevorzugt sind die Verbindungen der allgemeinen Formel (la) oder deren Salze, worin
- R¹: Wasserstoff bedeutet und R², R³, R⁴, R⁵, R⁶ und n wie in Formel (I) definiert sind [= Verbindungen der Formel (la")] oder
- R¹: Methyl bedeutet und R², R³, R⁴, R⁵, R⁶ und n wie in Formel (I) definiert sind, [= Verbindungen der Formel (Ia"')] oder

R² und R³ jeweils Wasserstoff bedeuten und R¹, R⁴, R⁵, R⁶ und n wie in Formel (I) definiert sind [= Verbindungen der Formel (Ia"")].

Besonders bevorzugt sind auch die Verbindungen der allgemeinen Formel (Ib) oder deren Salze, worin
- R¹: Wasserstoff bedeutet und R², R³, R⁴, R⁵, R⁶ und n wie in Formel (I) definiert sind [= Verbindungen der Formel (Ib")] oder
- R¹: Methyl bedeutet und R², R³, R⁴, R⁵, R⁶ und n wie in Formel (I) definiert sind, [= Verbindungen der Formel (Ib"')] oder

R² und R³ jeweils Wasserstoff bedeuten und R¹, R⁴, R⁵, R⁶ und n wie in Formel (I) definiert sind [= Verbindungen der Formel (Ib"")].

Besonders bevorzugt sind die Verbindungen der allgemeinen Formel (Ic) oder deren Salze, worin
- R¹: Wasserstoff bedeutet und R², R³, R⁴, R⁵, R⁶ und n wie in Formel (I) definiert sind [= Verbindungen der Formel (Ic")] oder
- R¹: Methyl bedeutet und R², R³, R⁴, R⁵, R⁶ und n wie in Formel (I) definiert sind, [= Verbindungen der Formel (Ic"')] oder

R² und R³ jeweils Wasserstoff bedeuten und R¹, R⁴, R⁵, R⁶ und n wie in Formel (I) definiert sind [= Verbindungen der Formel (Ic"")].

Dabei sind besonders die Verbindungen der Formel (I), (la), (Ib), oder (Ic) oder deren Salze bevorzugt, in welcher ein oder mehrere der Reste R¹ bis R⁶ die in den Beispieltabellen verwendeten Restebedeutungen aufweisen.

Dabei sind besonders die Verbindungen der Formel (I) und deren Salze bevorzugt, in welcher ein oder mehrere der Reste R¹ bis R⁶ die in den Beispieltabellen verwendeten Restebedeutungen aufweisen.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Gegenstand der vorliegenden Erfindung sind auch Verfahren zur Herstellung der Verbindungen der allgemeinen Formel (I) und/oder deren Salze.

Die erfindungsgemäßen Verbindungen der Formel (I) können alternativ durch verschiedene Verfahren dargestellt werden.

In den nachfolgenden Verfahren werden partiell Lösemittel verwendet. In diesem Zusammenhang bezeichnen "inerte Lösemitteln" jeweils Lösemittel, die unter den jeweiligen Reaktionsbedingungen inert sind, jedoch nicht unter beliebigen Reaktionsbedingungen inert sein müssen.

In den nachfolgenden Verfahren a), b), c), d), e), f), g), h), i) können die beschriebenen Reaktionen alternativ auch in einem Mikrowellenofen durchgeführt werden.
(a) Zur Herstellung von Verbindungen der allgemeinen Formel (I) oder deren Salzen, worin Het, R¹, R², R³, R⁴, R⁵, R⁶ die gemäß Formel (I) zuvor angegebenen Bedeutungen haben, wird nach Herstellungsverfahren (a) eine Verbindung der Formel (II),

   H₂N-NH-Het(R₆)ₙ (II)

   worin Het und (R⁶)ₙ wie in Formel (I) definiert ist,
   mit einer Verbindung der Formel (III), 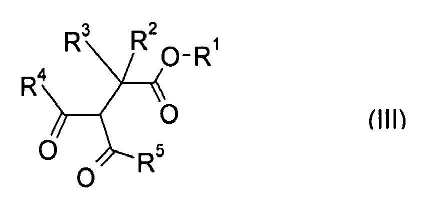 worin R¹, R², R³, R⁴ und R⁵ wie in Formel (I) definiert sind,
   zur Verbindung der Formel (I) oder deren Salz umgesetzt.
   Die beim erfindungsgemäßen Verfahren (a) zur Herstellung von Verbindungen der Formel (I) als Ausgangsstoffe verwendeten substituierten 1,3-Dicarbonylverbindungen der Formel (III) sind vorzugsweise solche, bei denen die Reste R¹, R², R³, R⁴ und R⁵ die bevorzugten Bedeutungen aufweisen, die bereits oben im Zusammenhang mit der Beschreibung der erfindungsgemäßen Verbindungen der Formel (I) als bevorzugt angegeben worden sind. Die beim erfindungsgemäßen Verfahren (a) zur Herstellung von Verbindungen der Formel (I) als Ausgangsstoffe verwendeten substituierten Heteroarylhydrazine der Formel (II) hat daher auch vorzugsweise diejenigen Bedeutungen für (R⁶)ₙ, die bereits oben im Zusammenhang mit der Beschreibung der erfindungsgemäßen Verbindungen der Formel (I) vorzugsweise für (R⁶)ₙ,und insbesondere bevorzugt in Abhängigkeit der Reste Het angegeben worden sind.
   Hydrazine der Formel (II) oder deren Salze als Ausgangsstoffe sind bekannt und/oder können nach bekannten Verfahren hergestellt werden (vgl. z.B. Methoden der organischen Chemie (Houben-Weyl, D. Klamann, Ed.) Band E16a, Teil 1, S. 421 ff., Georg Thieme Verlag, Stuttgart 1990 und dort zitierte Literatur; J. Am. Chem. Soc., 1954, 76, 596; Monatshefte für Chemie 1988, 119, 333; J. Heterocyclic Chem. 1988, 25, 1055; J. Heterocyclic Chem. 1989, 26, 475; Heterocycles 1994, 37, 379).
   Die Umsetzung der Verbindungen der Formel (II) und (III) kann ohne Katalysator oder in Gegenwart von Katalysatoren, bespielsweise von einer Säure als Katalysator, erfolgen, vorzugsweise in einem organischen Lösungsmittel, wie z.B. Tetrahydrofuran (THF), Dioxan, Acetonitril, Dimethylformamid (DMF), Methanol und Ethanol, bei Temperaturen zwischen 20 °C bis zum Siedepunkt des Lösungsmittels, vorzugsweise bei 50 °C bis 150 °C. Falls Säureadditionssalze der Formel (II) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalihydrogencarbonate, Alkalialkoholate, Erdalkalihydroxide, Erdalkalihydride, Erdalkalicarbonate oder organische Basen wie Triethylamin, Diisopropyl-ethylamin oder 1,8-Diazabicyclo[5.4.0]undec-7-en (DBU).
   Analoge Verfahren sind in der Literatur z. B. beschrieben in WO 2004/037793.
(b) Im Falle, dass R¹ in Formel (I) von Wasserstoff verschieden ist, ist das Herstellungsverfahren (b) **dadurch gekennzeichnet, dass** man eine Verbindung der Formel (I'), 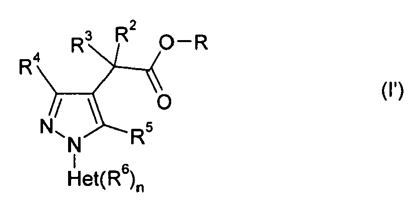 in welcher Het, R², R³, R⁴, R⁵ und R⁶ wie in Formel (I) definiert sind und
   - R: einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet, bedeutet,
   mit einer Verbindung der Formel (IV),

   R¹ - OH (IV)

   worin R¹ wie in Formel (I) definiert ist,
   zur Verbindung der Formel (I) umsetzt oder
(c) im Falle, dass R¹ in Formel (I) von Wasserstoff verschieden ist, ist das Herstellungsverfahren (c) **dadurch gekennzeichnet, dass** man eine Verbindung der Formel (I") 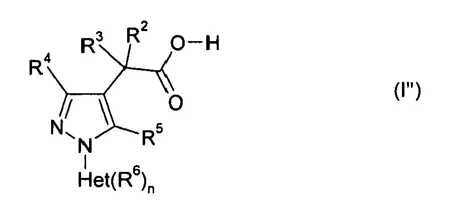 in welcher Het, R², R³, R⁴, R⁵ und R⁶ wie in Formel (I) definiert sind, gegebenenfalls nach Aktivierung der Säuregruppe, mit einer Verbindung der Formel (IV),

   R¹ - OH (IV)

   worin R¹ wie in Formel (I) definiert ist,
   zur Verbindung der Formel (I) umsetzt (verestert) oder
(d) im Falle, dass die Verbindung der Formel (I), worin R = H, oder deren Salz hergestellt wird, ist das Herstellungsverfahren (d) **dadurch gekennzeichnet, dass** man eine Verbindung der Formel (I') [siehe Definition in Variante (b)] zur Verbindung der Formel (I) oder deren Salz hydrolysiert.
   Die Ausgangsverbindungen der Formeln (II), (III) und (IV) sind in der Regel bekannt oder können analog bekannten Verfahren hergestellt werden.
   Die Umsetzung der Verbindungen der Formel (I') und (IV) kann nach Standardmethoden der Umesterung oder der Veresterung über aktivierte Carbonsäuren erfolgen.
   Die Umsetzung der Verbindungen der Formel (I") und (IV) kann nach Standardmethoden der Versterungen oder gegebenenfalls über aktivierte Carbonsäuren erfolgen.
   Die Herstellung von Verbindungen der Formel (I") aus Verbindungen (I') kann nach Standardmethoden der Verseifung erfolgen.
e) Die Verbindungen der Formel (III) lassen sich beispielsweise herstellen durch Umsetzung einer Dicarbonylverbindung der Formel (V)

   R⁴ - CO -CH₂ - CO - R⁵ (V)

   mit einer Verbindung der Formel (VI),

   R²R³C(Hal)-CO-OR¹ (VI)

   wobei R¹, R², R³, R⁴ und R⁵ wie in Formel (III) definiert sind, vorzugsweise R¹ Methyl oder Ethyl bedeutet, und Hal eine Abgangsgruppe, vorzugsweise ein reaktives Halogen wie ein Chlor-atom oder insbesondere ein Brom-atom, oder auch p-Toluolsulfonyl (Tosyl) oder Methylsulfonyl (Mesyl) bedeutet.
   Die erfindungsgemäßen Verbindungen der Formel (I) können gemäß den genannten Verfahren a) bis e)analog bekannten Methoden dargestellt werden, wie sie z. B. in Methoden der organischen Chemie (Houben-Weyl, E. Schaumann, Ed.) Band E8b, Hetarene III, Teil 2, S. 399-710, Georg Thieme Verlag, Stuttgart 1994 und dort zitierter Literatur beschrieben sind, wobei die Synthesen nach Methoden der organischen Chemie (Houben-Weyl, E. Schaumann, Ed.) Band E8b, Hetarene III, Teil 2, S. 420 ff., Georg Thieme Verlag, Stuttgart 1994 und dort zitierte Literatur; Synthesis, 1986, 409; J. Chinese Chem. Soc., 2001, 48, 45 und besonders US 4146721, DE2141124 , DOS 1946370 und Justus Liebigs Ann. Chem. 1973, 1919 von besonderem Interesse sind.
f) Die Verbindungen der Formel (V) lassen sich beispielsweise auch herstellen durch Umsetzung einer Verbindung der Formel (VII)

   R⁵ - CO- OR⁷ (VII)

   mit einer Verbindung der Formel (VIII),

   CH₃ - CO - R⁴ (VIII)

   wobei R⁴ und R⁵ wie in Formel (I) definiert sind, R⁷ (C₁-C₆)Alkyl bedeutet, vorzugsweise Methyl oder Ethyl bedeutet, in Gegenwart einer geeigneten organischen Base wie z. B. Natriummethanolat oder Natriumethanolat, in einem geigneten Lösemittel, z. B. Methanol, Ethanol, oder bevorzugt Tetrahydrofuran, in einem Temperaturbereich zwischen -10 und 50°C, bevorzugt 0°C und gegebenenfalls unter einer Inertgas-Atmosphäre, z.B. Stickstoff.
   Analoge Reaktionen für die Umsetzung sind in der Literatur beschrieben, z.B. Supramolecular Chemistry (2003), 15(7-8), 529-547; J. Am. Chem. Soc. (1951), 73 5614-16; J. of Med. Chem. (1990), 33(7), 1859-65; WO 00/08002.
   Alternativ können Verbindungen der Formel (V) auch durch Umsetzung einer Verbindung der Formel (IX)

   R⁴ - CO- OR⁷ (IX)

   mit einer Verbindung der Formel (X),

   CH₃ - CO - R⁵ (X)

   unter analogen Bedingungen wie oben unter f) beschrieben, erhalten werden, wobei R⁴ und R⁵ wie in Formel (I) definiert sind, R⁷ (C₁-C₆)Alkyl bedeutet, vorzugsweise Methyl oder Ethyl bedeutet.
   Analoge Reaktionen für die Umsetzung sind in der Literatur beschrieben, z. B. in J. Am. Chem. Soc. (1950), 72 1352-6.
g) Zur Herstellung einer Verbindung der allgemeinen Formel (I), 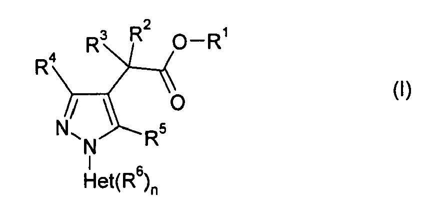 worin Het, R¹, R², R³, R⁴, R⁵ und R⁶ wie in Formel (I) definiert sind, kann beispielsweise auch eine Verbindung der allgemeinen Formel (XI), mit einem Bor-Derivat der Formel (XII), in Gegenwart eines geeigneten Cu(I) oder Cu(II) Salzes und einer organischen Base gegebenenfalls in einem Lösemittel zur Reaktion gebracht werden, wie in folgenden Schema dargestellt: 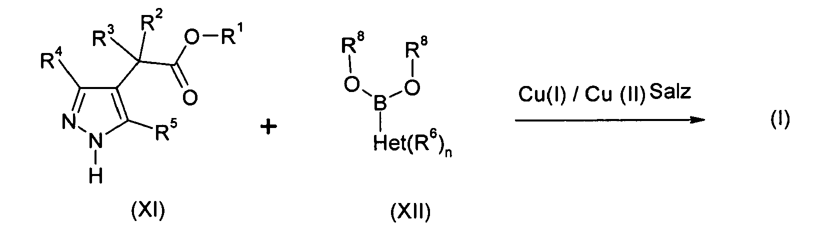 worin Het, R¹, R², R³, R⁴, R⁵, R⁶, die gemäß Formel (I) zuvor angegebenen Bedeutungen haben, und R⁸ gleich H oder (C₁-C₆)-Alkyl, bevorzugt Methyl, oder beide Alkylreste R⁸ zyklisch miteinander verknüpft sind.
   Die Umsetzung wird in Gegenwart eines geeigneten anorganischen oder organischen Kupfer(I)- oder Kupfer(II)-Salzes, bevorzugt Cul, Cu₂O, besonders bevorzugt Cu(OAc)₂, eingesetzt als 0.1 bis 1.5 Äquivalenten, mit mehr als einem Äquivalent Bor-Derivat (XII), bevorzugt zwischen 1.5 und 2.5 Äquivalenten, durchgeführt. Im Fall wo der Kupfer-Katalysator nicht stöchiometrisch eingesetzt wird, kann den Zusatz eines milden Oxidationsmittels helfen, geeignet sind z.B 2,2,6,6-Tetramethyl-1-piperiniloxy (TEMPO), Pyridin-Oxid, Sauerstoff oder trockene Luft.
   Dazu gibt man eine geeignete organischen Base wie z.B. Pyridin, Triethylamin oder Kalium-tert-butoxid und, um die Transmetallierung effizienter zu gestalten, eine Fluorid-Anionen Quelle, bevorzugt Cäsiumfluorid zu.
   Man arbeitet in einem geeigneten Lösemittel, bevorzugt einem halogenierten Lösemittel z.B. Trichlormethan oder bevorzugt Dichlormethan, in einem Temperaturbereich zwischen 0 und 40°C, bevorzugt zwischen 20 und 30°C und gegebenenfalls unter einer Inertgas-Atmosphäre, z.B. Stickstoff, bis zur erfolgten Umsetzung, wobei teilweise lange Reaktionszeiten erforderlich sein können..
   Analoge Methoden für Kupfer-induzierte C-N Kupplungen sind in der Literatur beschrieben, z.B. in Tet. Lett. 1998, 39, 2941; Tet. Lett. 1998, 39, 2933; Tet. Lett. 44 (2003) 3863-3865; J. Comb. Chem. 2004, 6, 385-390; Tet. Lett. 41 (2000) 9053 bis 9057.
   Analoge Methoden für Kupfer-induzierte C-N Kupplungen in Gegenwart von Fluorid-Anionen sind in der Literatur beschrieben, z.B. in Eur. J. Org. Chem. 2007, 1318-1323 und Org. Lett. 2007, 9 (5), 761.
   Analoge Methoden für katalytische Kupfer-induzierte C-N Kupplungen sind in der Literatur beschrieben, z.B. in Tet. Lett. 2001, 3415; Org. Lett. 2003, 5 (23), 4397 und Org. Lett. 2001, 3 (13), 2077.
   Analoge Methoden für Kupfer-induzierte C-N Kupplungen in einem Microwellenreaktor sind in der Literatur beschrieben, z.B. J. Comb. Chem. 358 2008, 10, 358-360.
   Verbindungen der allgemeinen Formel (XI) sind nach dem Fachmann bekannten Verfahren darstellbar, beispielsweise durch Umsetzung von einer Verbindung der allgemeine Formel (III), worin R¹, R², R³, R⁴ und R⁵ wie in Formel (I) definiert sind, mit Hydrazin(hydrat) wie beschrieben in Can. J. Chem. 2001, 79 (2), 183-194.
   Verbindungen der allgemeinen Formel (XII) sind dem Fachmann bekannt und teilweise kommerziell erhältlich oder nach dem Fachmann bekannten Verfahren darstellbar, beispielsweise wie beschrieben in a) Science of Synthesis, Houben-Weyl (Methods of Molecular Transformations), Category 2, Volume 6, Ed. E. Schaumann; b) Houben-Weyl (Methoden der organische Chemie), Band 13, Organoborverbindungen I-Teil 3a, Ed. E. Schaumann.
h) Zur Herstellung einer Verbindung der allgemeinen Formel (I), 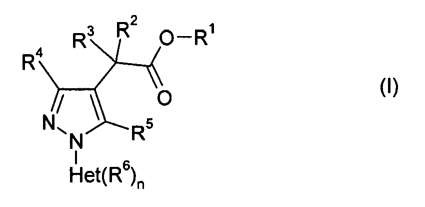 worin Het, R¹, R², R³, R⁴, R⁵ und R⁶ wie in Formel (I) definiert sind, kann beispielsweise auch eine Verbindung der allgemeinen Formel (XI), mit einer Verbindung der Formel (XIII), worin R⁶ die gemäß Formel (I) zuvor angegebene Bedeutung hat, in Gegenwart eines geeigneten Katalysator/Liganden-Systems, mit einer geeigneten Base und in einem geeigneten Lösemittel zur Reaktion gebracht werden, wie in folgendem Schema dargestellt: 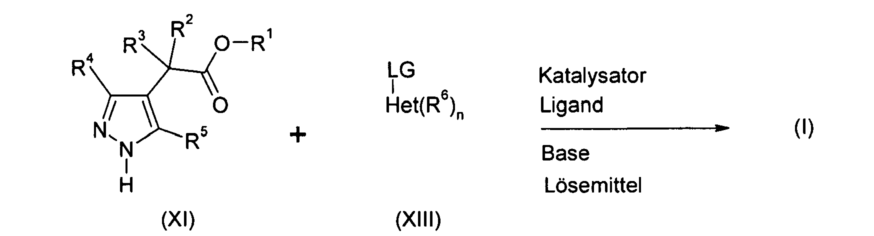 worin Het, R¹, R², R³, R⁴, R⁵, R⁶, die gemäß Formel (I) zuvor angegebenen Bedeutungen haben. LG bedeutet eine Abgangsgruppe, wobei als Abgangsgruppen beispielsweise Chlor, Brom, Iod, Phenylsulfonat, Tosylat oder Triflat fungieren können.
   Verbindungen der Formel (XIII) können mit Verbindungen der Formel (XI) in Gegenwart eines geeigneten Katalysator/Liganden-System zu Verbindungen der Formel (I) umgesetzt werden. Die Reaktion wird bevorzugt unter Anwendung eines geeigneten anorganischen oder organischen Kupfer(I)- oder Kupfer(II)-Salzes, bevorzugt, z. B. Cu(OAc)₂ (Kupferdiacetat), Cu(Acac)₂ (Kupferdiacetylacetat), Cul (Kupferiodid), CuBr (Kupferbromid), Cu₂O (Kupfer-(I)-oxid), [Cu(OH)TMEDA]₂Cl₂ (ein Kupferkomplexsalz, TMEDA bedeutet "Tetramethylethylendiamin"), oder Cu(0) (Kupfer-(II)-oxid), durchgeführt, mit einem geeigneten Liganden wie z.B. L-Proline, N,N'-Dimethylethan-1,2-diamin, trans- N,N'-Dimethylcyclohexan-1,2-diamin, Dimethylglycin, Salicylaldoxim, 1,1'-Binaphthalen-2,2'-diol (BINOL) und einer organischen oder anorganische Base wie z.B. Triethylamin, Pyridin, 2,6-Lutidine, Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid, Acetonitril.
   Man kann die Reaktion auch in Gegenwart eines geeignetes Palladium-Katalysators, z. B. Palladium(II)acetat, oder Dipalladium-tri-[(1E,4E)-1,5-diphenylpenta-1,4-dien-3-on] durchführen, mit einem Phosphin Liganden wie z. B. 2,2'-Bis(diphenylphospino)-1,1'-binaphthyl (BINAP), 1,1'-Diphenylphosphinoferrocene (DPPF), 2-Di-tert-butylphosphinobiphenyl (JohnPhos), 2-Dicyclohexylphosphino-2'-dimethylaminobiphenyl (DavePhos), 2-Dicyclohexylphosphino-2',4',6'-triisopropylbiphenyl (XPhos), 2-Dicyclohexylphosphino-2'-methylbiphenyl (MePhos), 4,5-Bis(diphenylphosphino)xanthene (XANTPHOS), Tri-tert-butylphosphoniumtetrafluoroborat oder Di-tert-butylphosphinsäure mit einer anorganische Base wie z.B. Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid oder Acetonitril oder deren Gemischen.
   Man kann die Reaktion auch in Gegenwart eines geeigneten Fe(III) Komplexes, durchführen, wie z.B. Eisen(III)oxid, Eisentrichlorid oder Eisentriacetylacetonat mit einem geeignetem Liganden wie z.B. L-Prolin, N,N'-Dimethylethan-1,2-diamin, trans- N,N'-Dimethylcyclohexan-1,2-diamin, oder Dimethylglycin und einer anorganische Base wie z.B. Cäsiumcarbonat, Kaliumcarbonat, Kaliumphosphat gegebenenfalls in einem Lösemittel, wie z.B. Toluol, 1,4-Dioxan, Dichlormethan, Dimethylformamid, Dimethylacetamid oder Acetonitril oder deren Gemischen.
   Man kann die Reaktion auch in Gegenwart eines Gemisches der Palladium- und Kupfer- oder Eisen- und Kupfer -basierten Katalysatoren durchführen.
   Die Umsetzung wird in der Regel in Gegenwart von mehr als einem Äquivalent einer Verbindung der Formel (XIII), bevorzugt zwischen 1 und 1.5 Äquivalenten, durchgeführt.
   Man arbeitet beispielsweise in einem Temperaturbereich zwischen 0 und 150°C, bevorzugt zwischen 60 und 120°C und gegebenenfalls unter einer Inertgas-Atmosphäre, z.B. Stickstoff, bis zur erfolgten Umsetzung, wobei teilweise lange Reaktionszeiten erforderlich sein können.
   Analoge Methoden für Kupfer-induzierte C-N Kupplungen sind in der Literatur beschrieben, z. B. Tet. Lett. 49 (2008) 948-951; Eur. J. Org. Chem. 2004, 695, 709; J. Org. Chem. 2007, 72, 2737- 2743; Heterocycles, 61, 2003, 505 - 512; Heterocycles, 48 (11), 1998, 2225; J. Am. Chem. Soc. 2002, 124, 11684-11688; J. Org. Chem. 2004, 69, 5578-5587; J. Org. Chem. 2007, 72, 8535-8538; Org. Lett. 2000, 2 (9), 1233-1236; Journal of Molecular Catalysis A: Chemical (2006), 256(1-2), 256-260; Acc.Chem. Res. (2008), 41(11), 1450-1460.
   Analoge Methoden für Palladium-induzierte C-N Kupplungen sind in der Literatur beschrieben, z.B. in J. Org. Chem. 2001, 66, 8677; J. Org. Chem. 1999, 64, 6019-6022; Angew. Chem. Int. Ed. 2005, 44, 1371 -1375; Heterocycles, 48, 11, 1998, 847*;* Tetrahedron 61 (2005) 2931-2939; Angew. Chem. Int. Ed. 2006, 45, 6523 - 6527.
   Analoge Methoden für Eisen-induzierte C-N Kupplungen sind in der Literatur beschrieben, z.B. in Angew. Chem. Int. Ed. 2007, 46, 934; Angew. Chem. Int. Ed. 2007, 46, 8862 -8865.
   Analoge Methoden für Kupfer/Eisen-induzierte C-N Kupplungen sind in der Literatur beschrieben, z.B. in Angew. Chem. Int. Ed. 2007, 46, 934; Tet. Lett. 39 (1998) 5617-5620.
   Analoge Methoden für Kupfer-induzierte C-N Kupplungen in einem Microwellenreaktor sind in der Literatur beschrieben, z.B. J. Comb. Chem. 358 2008, 10, 358-360.
   Verbindungen der allgemeinen Formel (XI) sind nach dem Fachmann bekannten Verfahren darstellbar, beispielsweise durch Umsetzung von einer Verbindung der allgemeine Formel (III), worin R¹, R², R³, R⁴ und R⁵ wie in Formel (I) definiert sind, mit Hydrazin(hydrat), wie beschrieben in Can. J. Chem. 2001, 79 (2), 183-194.
   Verbindungen der allgemeinen Formel (XIII) sind dem Fachmann bekannt und teilweise kommerziell erhältlich oder nach dem Fachmann bekannten Verfahren darstellbar, beispielsweise wie beschrieben in Science of Synthesis, Houben-Weyl (Methods of Molecular Transformations), Category 2, Volume 16, Ed. E. Schaumann.
i) Als Ausgangsstoff zur Herstellung einer Verbindung der genannten allgemeinen Formel (I), worin Het, R¹, R², R³, R⁴, R⁵ und R⁶, wie in Formel (I) definiert sind, kann beispielsweise auch eine Verbindung der allgemeinen Formel (XV) dienen, die durch Umsetzung einer Verbindung der Formel (XIII), worin R⁶ die gemäß Formel (I) zuvor angegebene Bedeutung hat, mit Benzophenonhydrazon (XIV), in Gegenwart eines geeigneten Katalysator/Liganden-Systems, mit einer geeigneten Base und in einem geeigneten Lösemittel hergestellt wird. Verbindungen der allgemeinen Formel (XV) ergeben mit einer Verbindung der allgemeinen Formel (III), in Gegenwart einer Säure, gegebenenfalls in einem Lösemittel, die Verbindung der allgemeinenFormel (I), wie in folgendem Schema dargestellt: 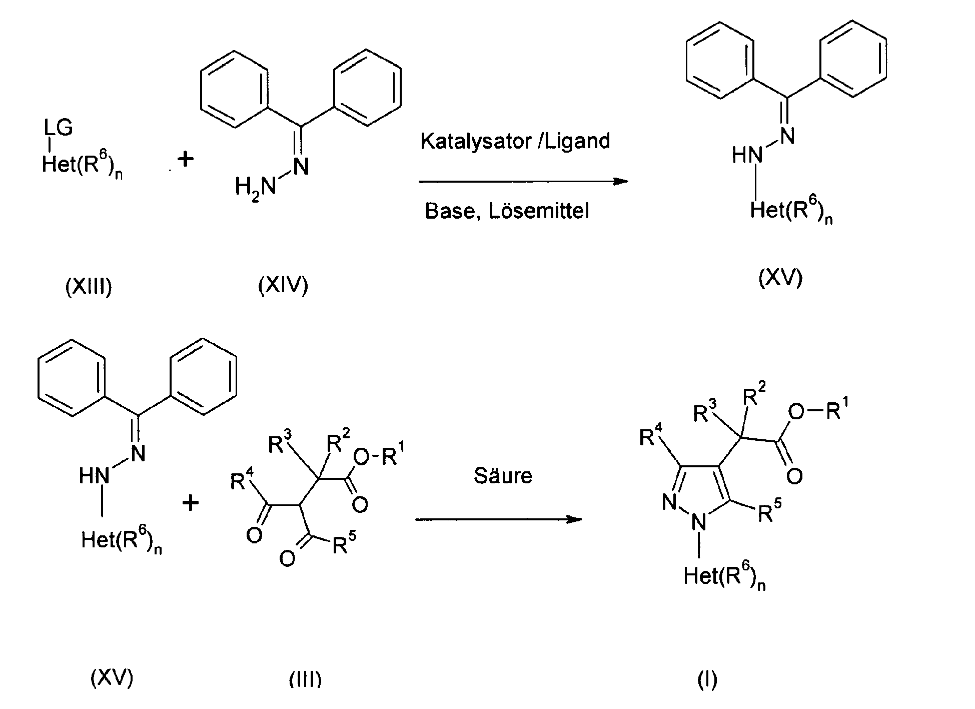 Hierin haben Het, R¹, R², R³, R⁴, R⁵, R⁶ die gemäß Formel (I) zuvor angegebenen Bedeutungen, LG bedeutet eine Abgangsgruppe, wobei als Abgangsgruppen beispielsweise Chlor, Brom, Iod, Phenylsulfonat, Tosylat oder Triflat fungieren können.
   Verbindungen der Formel (XIII) können mit Benzophenonhydrazon (XIV) in Gegenwart eines Katalysators und einem geeigneten Katalysator/Liganden-System zu Verbindungen der Formel (XV) umgesetzt werden. Die Reaktion wird bevorzugt unter Anwendung eines Palladium-Katalysators, z. B. Palladium(II)acetat, durchgeführt, mit einem Phosphin Liganden wie z. B. 2,2'-Bis(diphenylphospino)-1,1'-binaphthyl (BINAP), 1,1'-Diphenylphosphinoferrocene (DPPF), 2-Di-tert-butylphosphinobiphenyl (JohnPhos), 2-DiCyclohexylphosphino-2'-dimethylaminobiphenyl (DavePhos), 2-Dicyclohexylphosphino-2',4',6'-triisopropylbiphenyl (XPhos), 2-Dicyclohexylphosphino-2'-methylbiphenyl (MePhos), 4,5-Bis(diphenylphosphino)xanthene (XANTPHOS). Das Verwenden einer Base ist von Vorteil, z. B. Natrium-tert-butoxid. Die Reaktion wird in der Regel unter einer Inertgas-Atmosphäre, z.B. Stickstoff, unter Ausschluss von Wasser in einem geeigneten Lösemittel, z. B. Toluol, durchgeführt.
   Benzophenonhydrazon ist kommerziell erhältlich.
   Verbindungen der Formel (XV) können direkt weiter mit Verbindungen der Formel (III) zu Verbindungen der Formel (I) umgesetzt werden. Die Reaktion findet in Gegenwart einer geeigneten anorganischen oder organischen (nicht)wässrigen Säure statt, bevorzugt p-Toluolsulfonsäure, Schwefelsäure, besonders bevorzugt Salzsäure, wobei beispielsweise zwischen 1 und 10, bevorzugt zwischen 3 bis 7, besonders bevorzugt etwa 5 Äquivalente der Säure eingesetzt werden.
   Die Reaktion wird beispielsweise in einem geeigneten Lösemittel, z.B. Diethylether, Dioxan oder bevorzugt Tetrahydrofuran, in einem Temperaturbereich zwischen 0 und 80°C, bevorzugt 50°C und gegebenenfalls unter einer Inertgas-Atmosphäre, z.B. Stickstoff, durchgeführt.
   Analoge Zyklisierungsreaktionen eines Hydrazons mit einem 1,3-Diketon zu einem Pyrazol sind in der Literatur beschrieben, z.B. in WO-A-2001/32627; Angew. Chem. 110 (1998) 2249-2252; Tet. Lett. 43 (2002) 2171-2173; J.Am. Chem. Soc. 1981, 103, 7743 - 7752; Organic Process Research and Development 2004, 8, 1065 - 1071; Tet. Lett. 45 (2004) 5935-5937; WO-A-2007/064872, US 6489512, WO-A-2006/114213.
   Verbindungen der Formel (XV) können auch zu Verbindungen der Formel (II) umgesetzt werden, beispielsweise nach den dem Fachmann bekannten Verfahren, in Anwesenheit von Säure, bevorzugt unter teils wässrigen Bedingungen. Die Verbindungen der Formel (II) können weiter zu Verbindungen der Formel (I) nach obengenanntem Verfahren a) reagieren.
   Verbindungen der allgemeinen Formel (XIII) sind dem Fachmann bekannt und teilweise kommerziell erhältlich oder gemäß dem Fachmann bekannten Verfahren darstellbar, beispielsweise wie beschrieben in Science of Synthesis, Houben-Weyl (Methods of Molecular Transformations), Category 2, Volume 16, Ed. E. Schaumann.
   Verbindungen der allgemeinen Formel (XV) sind herstellbar wie z. B. beschrieben in Tet. Lett. 45 (2004) 5935-5937; Angew. Chem. Int. Ed. 2006, 45, 6523-6527; J. Am. Chem. Soc. (2003) 125, 13978-13980; J. Am. Chem. Soc. (2003), 125, 6653-6655; Org. Lett. 3 (9) (2001) 1351; Tet. Lett. 45 (2004) 5935-5937; Tetr. Lett. 43 (2002) 2171-2173; Angew. Chem. Int. Ed. 1998, 37 (15) 2090; WO2001/32627; J. Am. Chem. Soc. (1998) 120, 6621; WO-2007/064872; US-6489512; WO-2006/114213; US-2005/0192294, J. Am. Chem. Soc. 2003, 125, 6653 - 6655; Org. Lett. 2001, 3 (9), 1351 - 1354.
j) Zur Herstellung einer Verbindung der genannten Formel (I), worin Het, R¹, R², R³, R⁴, R⁵ und R⁶ wie in Formel (I) definiert sind, kann beispielsweise auch eine Verbindung der allgemeinen Formel (XVI), wobei R⁶ wie in Formel (I) definiert ist, mit Di-tert-butyl-azo-dicarboxylat (DBAD, XVII) in Gegenwart eines geeigneten Kupfer-Salzes in einem geeigneten Lösemittel zu einer Verbindung der allgemeinen Formel (XVIII) umgesetzt werden, worin R⁶ wie in Formel (I) definiert ist. Man erhält Verbindungen der Formel (II) oder deren Salze, worin R⁶ wie in Formel (I) definiert ist, die nach Verfahren a) zu Verbindungen der Formel (I), worin R¹, R², R³, R⁴, R⁵ und R⁶ wie in Formel (I) definiert sind, umgesetzt werden können, wie in folgenden Schema dargestellt: 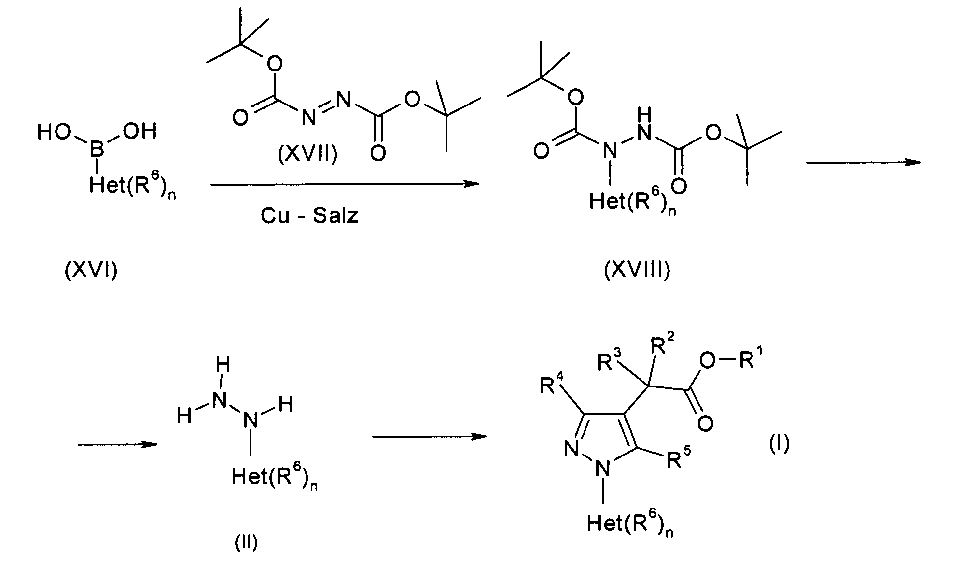 Die Umsetzung wird beispielsweise in Gegenwart eines geeigneten anorganischen Kupfer-Salzes, beispielsweise Cu(OAc)₂ (Kupferdiacetat) oder dessen Monohydrat Cu(OAc)₂.H₂O, in einem geeigneten Lösemittel, z.B. in einem Alkohol, wie Methanol, in einem Temperaturbereich zwischen 0 und 40°C, bevorzugt 20 - 25°C und gegebenenfalls unter einer Inertgas-Atmosphäre, z.B. Stickstoff, durchgeführt. Analoge Reaktionen unter Verwendung von kommerziell erhältlichem Di-tert-butyl-(E)-diazen-1,2-dicarboxylat (DTBAD) sind in der Literatur beschrieben, z.B.. Org. Lett. (2006) 8 (1), 43-45; J. Org. Chem. 2005, 70, 8631-8634.
   Verbindungen der allgemeinen Formel (XVI) sind kommerziell erhältlich und/oder nach dem Fachmann bekannten Verfahren darstellbar, z.B. wie beschrieben in a) Science of Synthesis, Houben-Weyl (Methods of Molecular Transformations), Category 2, Volume 6, Ed. E. Schaumann; b) Houben-Weyl (Methoden der organische Chemie), Band 13, Organoborverbindungen I-Teil 3a, Ed. E. Schaumann.
   Verbindungen der allgemeinen Formel (XVIII) können zu Verbindungen der allgemeinen Formel (II) nach dem Fachmann bekannten Verfahren umgesetzt werden, z. B wie beschrieben in J. Med. Chem. 1998, 41, 2858-2871; Tetrahedron 44 (17), 5525 (1988); J. Med. Chem. 1996, 39, 1172-1188; J. Org. Chem. 2004, 69, 5778-5781. Verbindungen der allgemeine Formel (II) oder deren Salze können nach obengenanntem Verfahren a) zu Verbindungen der Formel (I) umgesetzt werden.
k) Zur Herstellung einer Verbindung der genannten Formel (I), worin Het, R¹, R², R³, R⁴, R⁵ und R⁶ wie in Formel (I) definiert sind, kann beispielsweise auch eine Verbindung der allgemeinen Formel (XIX), worin R⁶ wie in Formel (I) definiert ist und LG' eine geeignete Gruppe bedeutet, wobei als geeignete Gruppen beispielsweise Brom und Iod fungieren können, mit einem geeigneten Metall oder einem geeigneten Transmetallierungsreagenz zu einer Verbindung der Formel (XX) umgesetzt werden, die wiederum, mit Di-tert-butyl-(E)-diazen-1,2-dicarboxylat (DTBAD, XVII) in Gegenwart eines geeigneten Lösemittels zu einer Verbindung der allgemeinen Formel (XVIII) umgesetzt wird, worin R⁶ wie in Formel (I) definiert ist. Man erhält Verbindungen der Formel (II) oder deren Salze, worin R⁶ wie in Formel (I) definiert ist, die nach Verfahren a) zu Verbindungen der Formel (I), R¹, R², R³, R⁴, R⁵ und R⁶ wie in Formel (I) definiert sind, umgesetzt werden können wie in folgenden Schema dargestellt:

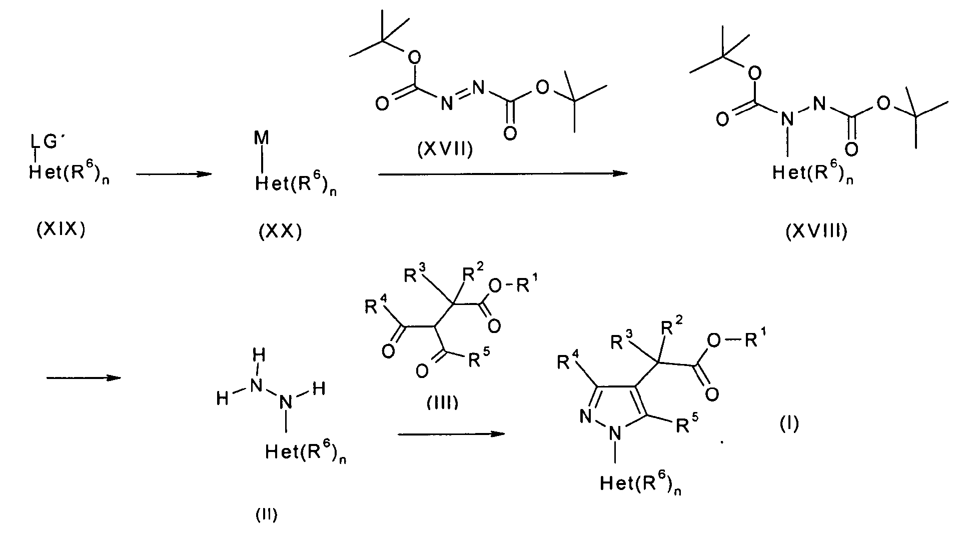

Die Umsetzung zu einer Verbindung der allgemeinen Formel (XX) wird beispielsweise in Gegenwart eines geeigneten Transmetallierungsreagenzes, z. B. einer Alkyllithium-Base, bevorzugt BuLi (Butyllithium); oder eines Metalls, bevorzugt Li, Mg oder Zn, durchgeführt. Das so entstandene Nucleophil wird mit Di-tert-butyl-(E)-diazen-1,2-dicarboxylat (DTBAD, XVIII) zu einer Verbindung der allgemeine Formel (XVIII) weiter umgesetzt. Analoge Reaktionen unter Verwendung von kommerziell erhältlichem Di-tert-butyl-azo-dicarboxylat (DBAD) sind in der Literatur beschrieben, z.B.. Tet. Lett. 1987, 28 (42), 4933; Tet. Lett. 39 (1998) 9157-9160.

Verbindungen der allgemeinen Formel (XIX) sind kommerziell erhältlich und/oder nach dem Fachmann bekannten Verfahren darstellbar, z.B. wie beschrieben in Science of Synthesis, Houben-Weyl (Methods of Molecular Transformations), Category 2, Volume 16, Ed. E. Schaumann.

Verbindungen der allgemeinen Formel (XVIII) können zu Verbindungen der allgemeine Formel (II) nach dem Fachmann bekannten Verfahren umgesetzt werden, z.B wie beschrieben in J. Med. Chem. 1998, 41, 2858-2871; Tetrahedron 44 (17), 5525 (1988); J. Med. Chem. 1996, 39, 1172-1188; J. Org. Chem. 2004, 69, 5778-5781. Verbindungen der allgemeine Formel (II) oder deren Salze können nach obengenannten Verfahren a) zu Verbindungen der Formel (I) umgesetzt werden.

Die Ausgangsstoffe der allgemeinen Formel (III) können nach allgemein bekannten Verfahren durch Alkylierung entsprechender 1,3-Diketone mit 2-halogenierten Essigsäurederivaten, beispielsweise Bromessigsäure-Derivaten erhalten werden (vgl. z.B. DE-OS 1946370, S. 13). Die hierfür als Ausgangsstoffe verwendeten 1,3-Diketone (V) sind nach obengenannten Verfahren f) herstellbar oder kommerziell erhältlich oder bekannt und/oder können nach bekannten Verfahren hergestellt werden (siehe z.B. US 4146721, DE2141124, DOS1946370 oder J. Am. Chem. Soc., 1948, 70, 4023; Justus Liebigs Ann. Chem. 1973, 1919; Justus Liebigs Ann. Chem. 1976, 13; J. Chem. Soc. Perkin Trans. 2, 1993, 6, 1067; Heteroatom Chemistry, 1997, 8, 147).

Für die Herstellung von Enantiomeren der Verbindungen (I) kommen auch übliche Racemattrennungsmethoden in Frage (vgl. Handbücher der Stereochemie), z. B. im Anschluss an Verfahren zur Trennung von Gemischen in Diastereomere, z. B. physikalische Verfahren wie Kristallisation, Chromatographieverfahren, vor allem Säulenchromatographie und Hochdruckflüssigchromatographie, Destillation, gegebenenfalls unter reduziertem Druck, Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Verbindungen (I) mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-Phenylethylamin und andere analoge Basen in Frage.

Die Kristallisationen werden dann meist in wässrigen oder wässrig-organischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH⁻.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen), den Boden, in dem oder auf dem die Pflanzen wachsen, (z. B. den Boden von Kulturland oder Nicht-Kulturland) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)

- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Weiter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie ÖI-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen 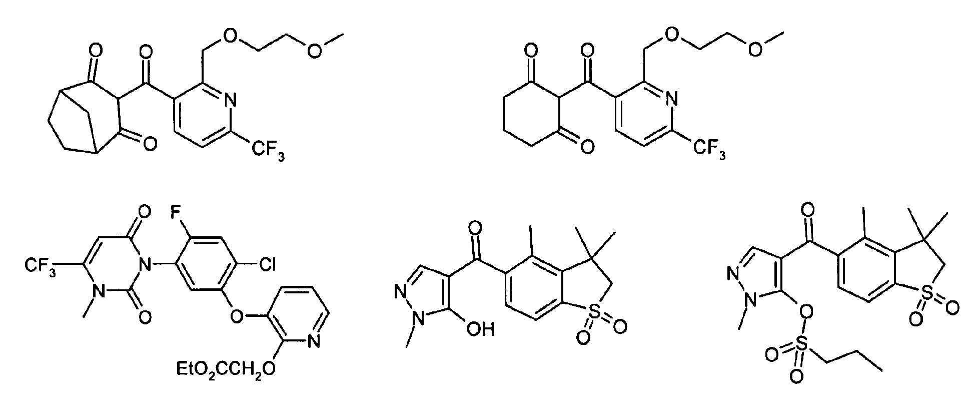 Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
A) Verbindungen der Formel (S-I), 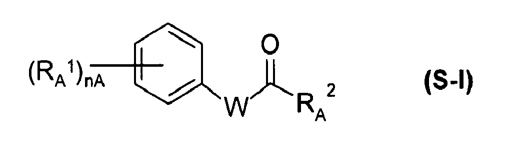 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴), 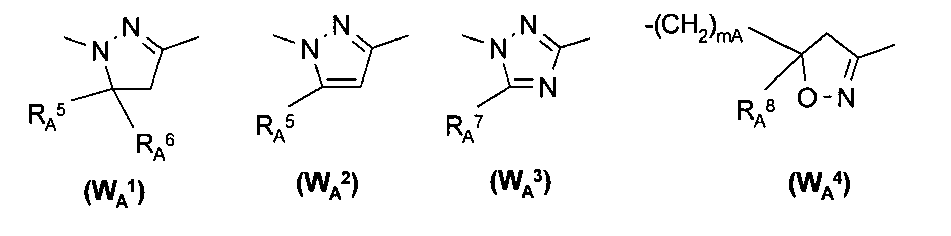
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁- C₄)Alkoxy-(C₁-C₄)alkyl, (C₁-C₆)Hydroxyalkyl, (C₃-C₁₂)Cycloalkyl oder Tri-(C₁- C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäure-ethylester (S1-1) ("Mefenpyr-diethyl", siehe Pestic. Man.), und verwandte Verbindungen, wie sie in der WO 91/07874 beschrieben sind;
   b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
   c) Verbindungen vom Typ der Triazolcarbonsäuren, vorzugsweise Verbindungen wie Fenchlorazol(-ethylester), d.h.
      1-(2,4-Dichlorphenyl)-5-trichlormethyl-(1H)-1,2,4-triazol-3-carbonsäure-ethylester (S1-6), und verwandte Verbindungen wie sie in EP-A-174 562 und EP-A-346 620 beschrieben sind;
   d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.
B) Chinolinderivate der Formel (S-II), 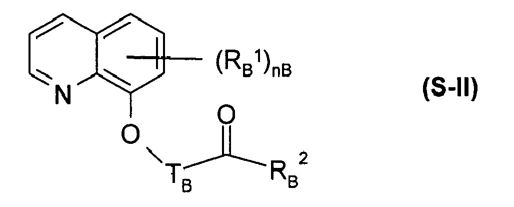 wobei die Symbole und Indizes folgende Bedeutungen haben:
   - R_{B}¹: Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
   b) Verbindungen vom Typ der (5-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
C) Verbindungen der Formel (S-III) 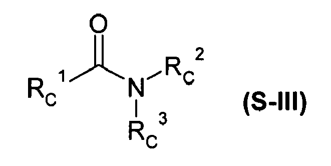 wobei die Symbole und Indizes folgende Bedeutungen haben:
   - R_{c}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{c}², R_{c}³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl- (C₁-C₄)alkyl, (C₂-C₄)Alkenylcarbamoyl-(C₁-C₄)alkyl, (C₁-C₄)Alkoxy-(C₁- C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{c}² und R_{c}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B. "Dichlormid" (siehe Pestic.Man.) (= N,N-Diallyl-2,2-dichloracetamid),
   "R-29148" (= 3-Dichloracetyl-2,2,5-trimethyl-1,3-oxazolidin von der Firma Stauffer),
   "R-28725" (= 3-Dichloracetyl-2,2,-dimethyl-1,3-oxazolidin von der Firma Stauffer),
   "Benoxacor" (siehe Pestic. Man.) (= 4-Dichloracetyl-3,4-dihydro-3-methyl-2H-1,4-benzoxazin),
   "PPG-1292" (= N-Allyl-N-[(1,3-dioxolan-2-yl)methyl]-dichloracetamid von der Firma PPG Industries),
   "DKA-24" (= N-Allyl-N-[(allylaminocarbonyl)methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "TI-35" (= 1-Dichloracetyl-azepan von der Firma TRI-Chemical RT) "Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 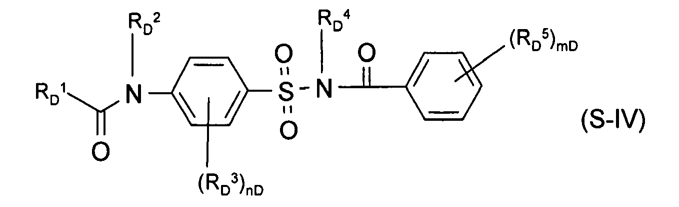 worin
   - R_{D}¹: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist, wobei jeder Kohlenwasserstoffteil vorzugsweise 1 bis 20 C-Atome aufweist und ein C-haltiger Rest R_{D}¹ inklusive Substituenten vorzugsweise 1 bis 30 C-Atome aufweist;
   - R_{D}²: Wasserstoff oder (C₁-C₄)Alkyl, vorzugsweise Wasserstoff, oder
   - R_{D}¹ und R_{D}²: zusammen mit der Gruppe der Formel -CO-N- den Rest eines 3- bis 8-gliedrigen gesättigten oder ungesättigten Rings;
   - R_{D}³: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R_{D}⁴: Wasserstoff oder (C₁-C₄)Alkyl, vorzugsweise H;
   - R_{D}⁵: gleich oder verschieden Halogen, Cyano, Nitro, Amino, Hydroxy, Carboxy, CHO, CONH₂, SO₂NH₂ oder einen Rest der Formel -Z^{c}-R^{c} ;
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b},R^{c}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen- (C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)- alkyl bedeuten;
   - Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten;
   - n_{D}: eine ganze Zahl von 0 bis 4, vorzugsweise 0, 1 oder 2, insbesondere 0 oder 1, und
   - m_{D}: eine ganze Zahl von 0 bis 5, vorzugsweise 0, 1, 2 oder 3, insbesondere 0, 1 oder 2;
   bedeuten;
E) Acylsulfamoylbenzoesäureamide der allgemeinen Formel (S-V), gegebenenfalls auch in Salzform, 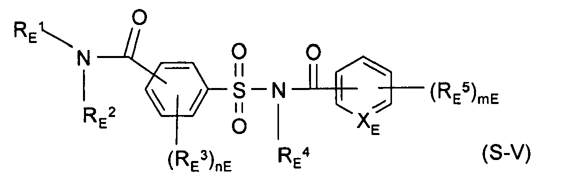 worin
   - X_{E}: CH oder N;
   - R_{E}¹: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R_{E}²: Wasserstoff, Hydroxy, (C₁-C₆)Alkyl, (C₂₋C₆)Alkenyl, (C₂-C₆)Alkinyl, (C₁- C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁- C₄)Alkylthio substituiert sind, oder
   - R_{E}¹ und R_{E}²: zusammen mit dem sie tragenden Stickstoffatom einen 3- bis 8- gliedrigen gesättigten oder ungesättigten Ring;
   - R_{E}³: Halogen, Cyano, Nitro, Amino, Hydroxy, Carboxy, CHO, CONH₂, SO₂NH₂ oder Z^{b}-R^{b};
   - R_{E}⁴: Wasserstoff, (C₁-C₄)Alkyl, (C₂-C₄)Alkenyl oder (C₂-C₄)Alkinyl;
   - R_{E}⁵: Halogen, Cyano, Nitro, Amino, Hydroxy, Carboxy, Phosphoryl, CHO, CONH₂, SO₂NH₂ oder Z^{c}-R^{c};
   - R^{a}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind; R^{b}, R^{c} gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)- Haloalkoxy, Mono- und Di-[(C₁-C₄)-alkyl]-amino substituiert sind;
   - z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)-Alkyl oder (C₁-C₄)-Haloalkyl;
   - n_{E}: eine ganze Zahl von 0 bis 4, und
   - m_{E}: für den Fall, daß X für CH steht, eine ganze Zahl von 0 bis 5, und für den Fall, daß X für N steht, eine ganze Zahl von 0 bis 4
   bedeuten;
   davon bevorzugt sind Verbindungen (auch in Form ihrer Salze) vom Typ der Acylsulfamoylbenzoesäureamide, z.B. der nachfolgenden Formel (S-VI), die z.B. bekannt sind aus WO 99/16744, 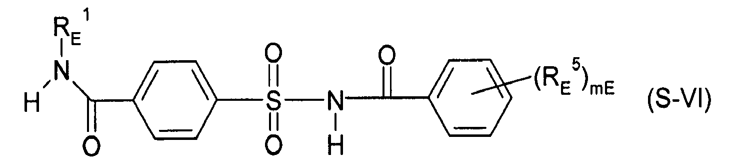 z.B. solche worin
   R_{E}¹ = Cyclopropyl und R_{E}⁵ = 2-OMe ist ("Cyprosulfamide", S3-1),
   R_{E}¹ = Cyclopropyl und R_{E}⁵ = 5-Cl-2-OMe ist (S3-2),
   R_{E}¹ = Ethyl und R_{E}⁵ = 2-OMe ist (S3-3),
   R_{E}¹ = Isopropyl und R_{E}⁵ = 5-Cl-2-OMe ist (S3-4) und
   R_{E}¹ = Isopropyl und R_{E}⁵ = 2-OMe ist (S3-5);
F) Verbindungen vom Typ der N-Acylsulfamoylphenylharnstoffe der Formel (S-VII), die z.B. bekannt sind aus der EP-A-365484, 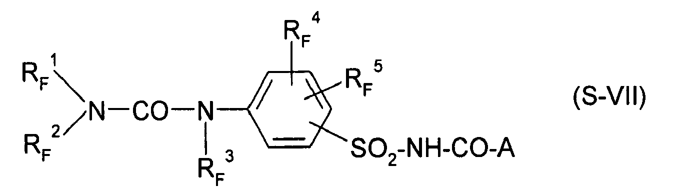 worin
   A für einen Rest aus der Gruppe 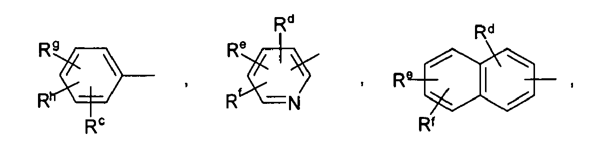 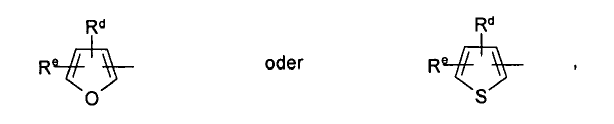
   - R_{F}¹ und R_{F}²: unabhängig voneinander für Wasserstoff, (C₁-C₈)Alkyl, (C₃-C₈)Cycloalkyl, (C₃-C₆)Alkenyl, (C₃-C₆)Alkinyl, 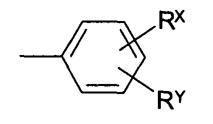 oder durch (C₁-C₄)Alkoxy oder 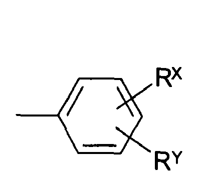 substituiertes (C₁-C₄)Alkoxy, oder
   - R_{F}¹ und R_{F}²: gemeinsam für eine (C₄-C₆)Alkylenbrücke oder eine durch Sauerstoff, Schwefel, SO, SO₂, NH oder -N(C₁-C₄-Alkyl)- unterbrochene (C₄- C₆)Alkylenbrücke,
   - R_{F}³: für Wasserstoff oder (C₁-C₄)Alkyl,
   - R_{F}⁴ und R_{F}⁵: unabhängig voneinander für Wasserstoff, Halogen, Cyan, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, -COOR^{j}, -CONR^{k}R^{m}, -CORⁿ, -SO₂NR^{k}R^{m} oder -OSO₂-C₁- C₄-Alkyl, oder R^{a} und R^{b} gemeinsam für eine (C₃-C₄)Alkylenbrücke, die durch Halogen oder C₁-C₄-Alkyl substituiert sein kann, oder eine (C₃- C₄)Alkenylenbrücke, die durch Halogen oder (C₁-C₄)Alkyl substituiert sein kann, oder eine C₄-Alkadienylenbrücke, die durch Halogen oder (C₁-C₄)Alkyl substituiert sein kann, und
   - R^{g} und R^{h}: unabhängig voneinander für Wasserstoff, Halogen, C₁-C₄-Alkyl, Trifluormethyl, Methoxy, Methylthio oder -COOR^{j} stehen, wobei
   - R^{c}: Wasserstoff, Halogen, (C₁-C₄)Alkyl oder Methoxy,
   - R^{d}: Wasserstoff, Halogen, Nitro, (C₁-C₄)Alkyl, (C₁-C₄)Alkoxy, (C₁-C₄)Alkylthio, (C₁- C₄)Alkylsulfinyl, (C₁-C₄)Alkylsulfonyl, -COOR^{j} oder -CONR^{k}R^{m},
   - R^{e}: Wasserstoff, Halogen, C₁-C₄-Alkyl, -COOR^{j}, Trifluormethyl oder Methoxy, oder R^{d} und R^{e} gemeinsam für eine (C₃-C₄)Alkylenbrücke,
   - R^{f}: Wasserstoff, Halogen oder (C₁-C₄)Alkyl,
   - R^{X} und R^{Y}: unabhängig voneinander Wasserstoff, Halogen, (C₁-C₄)Alkyl, (C₁- C₄)Alkoxy, (C₁-C₄)Alkylthio, -COOR⁴, Trifluormethyl, Nitro oder Cyan,
   - R^{j}, R^{k} und R^{m}: unabhängig voneinander Wasserstoff oder (C₁-C₄)Alkyl,
   - R^{k} und R^{m}: gemeinsam eine (C₄-C₆)Alkylenbrücke oder eine durch Sauerstoff, NH oder -N(C₁-C₄-Alkyl)- unterbrochene C₄-C₆-Alkylenbrücke, und
   - Rⁿ: (C₁-C₄)Alkyl, Phenyl oder durch Halogen, (C₁-C₄)Alkyl, Methoxy, Nitro oder Trifluormethyl substituiertes Phenyl bedeuten,
   davon bevorzugt sind:
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff, einschließlich der Stereoisomeren und der in der Landwirtschaft gebräuchlichen Salze,
G) Wirkstoffe aus der Klasse der Hydroxyaromaten und der aromatischaliphatischen Carbonsäurederivate, z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
K) Verbindungen der Formel (S-IX), wie sie in der WO-A-1998/38856 beschrieben sind 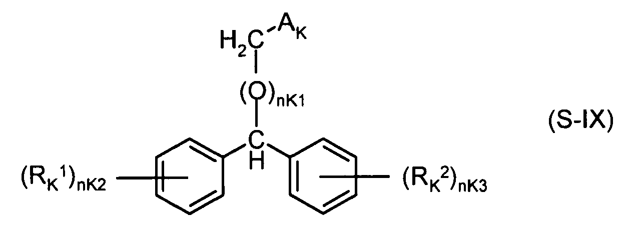 worin die Symbole und Indizes folgende Bedeutungen haben:
   - R_{K}¹, R_{K}²: unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{K}: COOR_{K}³ oder COOR_{K}⁴
   - R_{K}³, R_{K}⁴: unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{K}¹: 0 oder 1 und
   - n_{K}², n_{K}³: unabhängig voneinander 0, 1 oder 2;
   vorzugsweise:
   Methyl-(diphenylmethoxy)acetat (CAS-Regno: 41858-19-9),
L) Verbindungen der Formel (S-X),
   wie sie in der WO A-98/27049 beschrieben sind 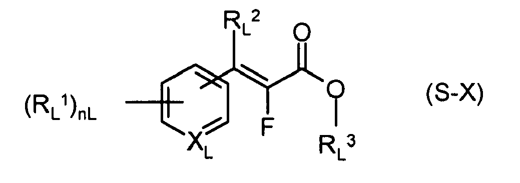 worin die Symbole und Indizes folgende Bedeutungen haben:
   - X_{L}: CH oder N,
   - n_{L}: für den Fall, dass X=N ist, eine ganze Zahl von 0 bis 4 und für den Fall, dass X=CH ist, eine ganze Zahl von 0 bis 5 ,
   - R_{L}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)Alkylsulfonyl, (C₁-C₄)Alkoxycarbonyl, ggf. substituiertes. Phenyl, ggf. substituiertes Phenoxy,
   - R_{L}²: Wasserstoff oder (C₁-C₄)Alkyl
   - R_{L}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)-2-chinolone, z.B.
   1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolyl-carbonyl)-2-chinolon (CAS-Regno: 95855-00-8), wie sie in der WO-A-1999000020 beschrieben sind,
N) Verbindungen der Formeln (S-XI) oder (S-XII)
   wie sie in der WO-A-2007023719 und WO-A-2007023764 beschrieben sind 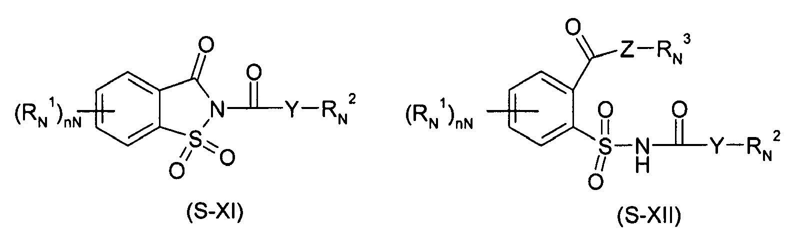 worin
   - R_{N}¹: Halogen, (C₁-C₄)Alkyl, Methoxy, Nitro, Cyano, CF₃, OCF₃
   - Y, Z: unabhängig voneinander O oder S,
   - n_{N}: eine ganze Zahl von 0 bis 4,
   - R_{N}²: (C₁-C₁₆)Alkyl, (C₂-C₆)Alkenyl, (C₃-C₆)Cycloalkyl, Aryl; Benzyl, Halobenzyl,
   - R_{N}³: Wasserstoff, (C₁-C₆)Alkyl bedeuten;
O) eine oder mehreren Verbindungen aus Gruppe:
   1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole), 2-Dichlormethyl-2-methyl-1,3-dioxolan (MG-191),
   einschließlich der Stereoisomeren und der in der Landwirtschaft gebräuchlichen Salze.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. Dies gilt sowohl für die Anwendung im Vorauflauf oder im Nachauflauf, wobei die Nachauflaufbehandlung in der Regel bevorzugt ist.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben. In den Beispielen beziehen sich Mengenangaben (auch Prozentangaben) auf das Gewicht, sofern nichts anderes speziell angegeben ist. Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold- Prelog-Regel, wenn nichts anderes näher definiert ist.

### (A) Synthesebeispiele

### Beispiel A1 [5-(4-Chlorphenyl)-3-methyl-1-(1,3-thiazol-5-yl)-1H-pyrazol-4-yl]essigsäure (siehe Tabelle 7, Beispiel 7-29)

a) Herstellung von Methyl-[5-(4-chlorphenyl)-3-methyl-1H-pyrazol-4-yl]acetat Zu einer Lösung von 10 g (37 mmol) Methyl-3-[(4-chlorphenyl)carbonyl]-4-oxopentanoat in Ethanol (100 ml) gab man 2,236 g (45 mmol) Hydrazinhydrat. Die Mischung wurde 6 Stunden unter Rückfluss erhitzt, dann auf Wasser gegeben und mit Dichlormethan extrahiert. Die vereinigten organischen Phasen wurden mit Magnesiumsulfat getrocknet, filtriert und eingeengt. Man erhielt 6,9 g Produkt (63 % d. Th.); NMR (CDCl₃, 400 MHz): 2.28 (s, 3H); 3.5 (s, 2H); 3.7 (s, 3H); 7.39 (d, 2H); 7.5 (d, 2H).
b) Herstellung von Methyl-[5-(4-chlorphenyl)-3-methyl-1-(1,3-thiazol-5-yl)-1H-pyrazol-4-yl]acetat
   Methyl-[5-(4-chlorphenyl)-3-methyl-1H-pyrazol-4-yl]acetat (1,0 g, 3,77 mmol), 5-Bromthiazol (0,526 g, 3,21 mmol), Salicylaldoxim (0,103 g, 0,75 mmol), Cu₂O (0,027 g, 0,18 mmol), Cs₂CO₃ (1,477 g, 4,53 mmol) und Acetonitril (4,8 ml) wurden in einem ausgeheitzen und über Argonstrom abgekühlten Gefäß nacheinander eingefüllt. Das geschlossene Gefäß wurde im Mikrowellenofen für 6 Stunden auf 85°C erhitzt. Danach wurde das Reaktionsgemisch auf Eiswasser gegossen, mit Dichloromethan extrahiert, die organische Phase getrocknet und eingeengt. Nach Chromatographie des Rückstands erhielt man 0,096 g Produkt (6,5 % d. Th.); NMR (CDCl₃, 400 MHz): 2.31 (s, 3H); 3.35 (s, 2H); 3.70 (s, 3H); 7.22 (d, 2H); 7.29 (br s, 1H); 7.42 (d, 2H); 8.53 (br s, 1H).
c) Herstellung von [5-(4-Chlorphenyl)-3-methyl-1-(1,3-thiazol-5-yl)-1H-pyrazol-4-yl]essigsäure
   Zu 0,289 g (0,83 mmol) Methyl-[5-(4-chlorphenyl)-3-methyl-1-(1,3-thiazol-5-yl)-1H-pyrazol-4-yl]acetat in 10 ml Methanol gab man 1 ml 2 molare wässrige Natronlauge, rührte vier Stunden bei Raumtemperatur und ließ über Nacht stehen. Der Ansatz wurde dann eingeengt. Zum Rückstand gab man Eiswasser, stellte das Gemisch durch Zugabe von 2 molarer wässriger Salzsäure auf pH 3-4 und extrahierte mit Dichloromethan. Die vereinigten organischen Phasen wurden mit Magnesiumsulfat getrocknet, filtriert und eingeengt. Das Rohprodukt wurde am Hochvakuum getrocknet. Man erhielt 0,187 g Produkt (60,7 % d. Th.); NMR (CDCl₃, 400 MHz): 2.33 (s, 3H); 3.39 (s, 2H); 7.24 (d, 2H); 7.31 (br s, 1H); 7.42 (d, 2H); 8.56 (br s, 1H).

### Beispiel A2 Cyclopropylmethyl-[5-(4-chlorphenyl)-3-methyl-1-(1,3-thiazol-5-yl)-1H-pyrazol-4-yl]acetat (siehe Tabelle 9, Beispiel 9-123)

In einen Mikrowellenreaktor wurden nacheinander 0,40 g (0,41 mmol) [5-(4-Chlorphenyl)-3-methyl-1-(1,3-thiazol-5-yl)-1H-pyrazol-4-yl]essigsäure, 3 ml Cyclopropylcarbinol und 2 Tropfen konzentrierte Schwelsäure gegeben.
Das geschlossenen Gefäß wurde im Mikrowellenofen für 4 Stunden auf 100°C erhitzt. Danach wurde der Ansatz am Hochvakuum eingeengt. Man gab Eiswasser und Dichloromethan zu und stellte mit ges. NaHCO₃-Lsg auf pH 8. Die vereinigten organischen Phasen wurden mit Magnesiumsulfat getrocknet, filtriert und eingeengt. Nach Chromatographie des Rückstands erhielt man 0,047 g Produkt (26 % d. Th.). NMR (CDCl₃, 400 MHz): 0.28 (m, 2H); 0.58 (m, 2H); 1.10 (m, 1H); 2.33 (s, 3H); 3.37 (s, 2H); 3.92 (d, 2H); 7.27 (d, 2H); 7.29 (br s, 1H); 7.42 (d, 2H); 8.52 (br s, 1H).

### Beispiel A3 Methyl-4-oxo-3-(pyridin-2-ylcarbonyl)pentanoat (siehe Tabelle 10, Beispiel 10-68)

### a) Herstellung von 1-(Pyridin-2-yl)butan-1,3-dion

Zu einer Mischung von 10 g (73 mmol) Picolinsäuremethylester und 25 ml (124 mmol) Aceton in 150 ml Tetrahydrofuran wurde eine Natriummethylat Lösung (28% in Methanol) zugetropft. Man rührte 3 Stunden bei 20°C und entfernte das Lösemittel im Vakuum. Der Rückstand wurde in Wasser aufgenommen, mit 2 molarer wässriger Salzsäure angesäuert und mit Dichlormethan extrahiert. Nach Trocknen der vereinigten organischen Phasen und Entfernen des Lösemittels im Vakuum erhielt man 7,940 g Produkt (60% d. Th.); NMR (CDCl₃, 400 MHz): 2.23 (s, 3H); 6.81 (s, 1H); 7.41 (m, 1H); 7.83 (m, 1H); 8.09 (m, 1H); 8.68 (m, 1H); 15.7 (br, 1H).

### b) Methyl-4-oxo-3-(pyridin-2-ylcarbonyl)pentanoat

Zu 0,911 g (22,78 mmol) Natriumhydrid in 25 ml Dimethylsulfoxid tropfte man eine Lösung von 3,38 g (20,7 mmol) 1-(Pyridin-2-yl)butan-1,3-dion gelöst in Dimethylsulfoxid so langsam zu, dass die Temperatur nicht höher als 30°C anstieg. Es wurde 30 Minuten bei 20°C weitergerührt. Dann wurden 3,486 g (22,78 mmol) Bromessigsäuremethylester in etwas Dimethylsulfoxid bei 0°C langsam zugetropft. Man rührte weitere 4 Stunden bei 20°C. Die Reaktionsmischung wurde auf Eiswasser gegossen, und mit Dichlormethan extrahiert. Die organische Phase wurde mehrmals mit Wasser gewaschen. Nach Trocknen der vereinigten organischen Phasen, Entfernen des Lösemittels im Vakuum und Chromatographie des Rückstands erhielt man 3,48 g Produkt (71,4% d. Th.); NMR (CDCl₃, 400 MHz): 2.4 (s, 3H); 2.9 (d, 1H); 3.05 (d, 1H); 3.69 (s, 3H); 5.49 (dd, 1H); 7.50 (m, 1H); 7.86 (m, 1H); 8.07 (m, 1H); 8.7 (m, 1H).

### Beispiel A4 Ethyl-5-[4-(2-methoxy-2-oxoethyl)-3-methyl-5-(pyridin-2-yl)-1H-pyrazol-1-yl]-1,3-thiazol-4-carboxylat (s. Tab. 8, Beispiel 8-241)

a) Herstellung von Methyl-4-oxo-3-(pyridin-2-ylcarbonyl)pentanoat Die Herstellung verläuft gemäß Beispiel A3.
b) Herstellung von Ethyl-5-hydrazinyl-1,3-thiazol-4-carboxylat Ethyl-5-hydrazinyl-1,3-thiazol-4-carboxylat wurde nach dem Fachmann bekannten Methoden via Diazotierung von Ethyl-5-amino-1,3-thiazol-4-carboxylat und anschliessende Reduktion hergestellt.
c) Herstellung von Ethyl-5-[4-(2-methoxy-2-oxoethyl)-3-methyl-5-(pyridin-2-yl)-1H-pyrazol-1-yl]-1,3-thiazol-4-carboxylat

In einem Mikrowellenreaktor wurden 0.450 g (1.91 mmol) Methyl-4-oxo-3-(pyridin-2-ylcarbonyl)pentanoat in 4 ml Methanol mit 0.358 g (1.91 mmol) Ethyl-5-hydrazinyl-1,3-thiazol-4-carboxylat versetzt. Das geschlossene Gefäß wurde im Mikrowellenofen für 2 Stunden auf 90°C erhitzt. Nach Entfernen des Lösemittels extrahierte man mehrfach mit Dichlormethan / Wasser. Die vereinigten organischen Phasen wurden über Natriumsulfat getrocknet und das Lösemittel im Vakuum entfernt. Nach Chromatographie des Rückstands erhielt man 0,508 g Produkt (61,8% d. Th.), NMR (CDCl₃, 300 MHz): 1.12 (t, 3H); 2.35 (s, 3H); 3.66 (s, 2H); 3.69 (s, 3H); 4.08 (s, 3H); 7.19 (m, 1H); 7.32 (m, 1H); 7.66 (m, 1H); 8.51 (m, 1H); 8.72 (s, 1H).

### Beispiel A5 Methyl-3-[(4-chlorphenyl)carbonyl]-4-oxopentanoat (siehe Tabelle 10, Beispiel 10-13)

Zu 2,237 g (56 mmol) Natriumhydrid in 200 ml Dimethylsulfoxid tropfte man eine Lösung von 10 g (51 mmol) 1-(4-Chlorphenyl)butan-1,3-dion (kommerziell erhältlich) gelöst in Dimethylsulfoxid so langsam zu, dass die Temperatur nicht höher als 30°C anstieg. Es wurde 30 Minuten bei 20°C weitergerührt. Dann wurden 8,558 g (56 mmol) Bromessigsäuremethylester in etwas Dimethylsulfoxid bei 0°C langsam zugetropft. Man rührte 4 Stunden bei 20°C. Die Reaktionsmischung wurde auf Eiswasser gegossen, und mit Dichlormethan extrahiert. Die organische Phase wurde mehrmals mit Wasser gewaschen. Nach Trocknen der vereinigten organischen Phasen, Entfernen des Lösemittels im Vakuum und Chromatographie des Rückstands erhielt man 7,750 g Produkt (56,7% d. Th.); NMR (CDCl₃, 400 MHz): 2.19 (s, 3H); 2.99 (d, 1H); 3.03 (d, 1H); 3.69 (s, 3H); 4.95 (dd, 1H); 7.49 (d, 2H); 7.98 (d, 2H).

### Beispiel A6 Methyl-[5-(4-chlorphenyl)-3-methyl-1-(3-methylisothiazol-

5-yl)-1H-pyrazol-4-yl]acetat (siehe Tabelle 2, Beispiel 2-147)
a) Herstellung von Methyl-3-[(4-chlorphenyl)carbonyl]-4-oxopentanoat Die Herstellung verläuft gemäß Beispiel A5.
b) Herstellung von 5-Hydrazinyl-3-methylisothiazol 5-Hydrazinyl-3-methylisothiazol wurde nach dem Fachmann bekannten Methoden via Diazotierung von 5-Amino-3-methylisothiazol und anschliessende Reduktion hergestellt.
c) Herstellung von Methyl-[5-(4-chlorphenyl)-3-methyl-1-(3-methylisothiazol-5-yl)-1H-pyrazol-4-yl]acetat

Zu 0.500 g (2.0 mmol) Methyl-3-[(4-chlorphenyl)carbonyl]-4-oxopentanoat in 10.00 ml Methanol gab man 0,301 g (2.0 mmol) 5-Hydrazinyl-3-methylisothiazol und 0.099 ml (2.0 mmol) konzentrierte Schwefelsäure und rührte 6 Stunden unter Rückfluss. Der Ansatz wurde auf Wasser gegossen und mit Ethylacetat extrahiert.
Nach Trocknen der vereinigten organischen Phasen, Entfernen des Lösemittels im Vakuum und HPLC Trennung des Rückstands erhielt man 0.106 g Produkt (14.9% d. Th.); NMR (CDCl3, 400 MHz): 2.29 (s, 3H); 2.30 (s, 3H); 3.29 (s, 2H); 3.68 (s, 3H); 6.30 (s, 1H); 7.31 (d, 2H); 7.49 (d, 2H).

Die in den nachfolgenden Tabellen 1 bis 10 beschriebenen Verbindungen erhält man gemäß den oder analog zu den oben beschriebenen Beispielen.
In der Tabelle 10 sind Zwischenprodukte der Formel (III) aufgeführt, die gemäß den oben beschriebenen Verfahren eingesetzt werden können.

In den Tabellen 1 bis 10 bedeuten:
- F, Cl, Br, J =: Fluor, Chlor, Brom bzw. Jod entsprechend den üblichen chemischen Atomsymbolen
- Me =: Methyl
- MeO oder OMe =: Methoxy
- 3,5-Me₂ =: 3,5-Dimethyl (z. B. als Substitution am Phenylring)
- 4,5-Cl₂ =: 4,5-Dichloro (z. B. als Substitution am Phenylring)
- Et =: Ethyl
- Pr =: n-Propyl
- iPr =: Isopropyl
- iOPr =: Isopropyloxy
- cyPr =: Cyclopropyl
- tBu =: tertiär-Butyl
- Ph =: Phenyl
- PhO =: Phenoxy
- Ac =: COCH₃ = Acetyl
- Allyl =: Prop-2-en-1-yl
- COOH =: Carboxy
- COOEt =: Ethoxycarbonyl
- COOMe =: Methoxycarbonyl
- 3,5-(COOMe)₂: = 3,5-Dimethoxycarbonyl
- "(R⁶)ₙ = H" =: unsubstituierter Cyclus (n = 0)

Im Übrigen gelten die üblichen chemischen Symbole, wie z. B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

Physikalische Daten ("Daten") der Verbindungen zu den Tabellen sind gegebenenfalls in den ausführlichen Herstellungsbeispielen (siehe oben) oder am Ende der Tabellen angegeben. Dabei bedeuten:
- "NMR" =: Daten gemäß ¹H-NMR-Spektum (¹H-Kernresonanz-Daten)
- "Schmp." =: Schmelzpunkt

**Tabelle 1: Verbindungen der Formel (la")**

| | | | | | |
|---|---|---|---|---|---|
| 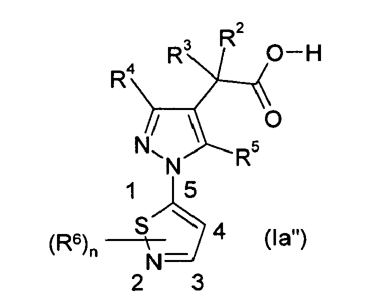 | | | | | |

| Nr. | R² | R³ | R⁴ | R⁵ | (R⁶)ₙ |
|---|---|---|---|---|---|
| 1-1 | H | H | Ph | Ph | H |
| 1-2 | H | H | Me | Ph | H |
| 1-3 | H | H | Me | 5-J-2-thienyl | H |
| 1-4 | H | H | Me | 2-Furyl | H |
| 1-5 | H | H | Me | Ph | 3-OMe |
| 1-6 | Me | H | Me | Ph | 4-Me |
| 1-7 | H | H | Me | Ph | 3-Cl |
| 1-8 | H | H | Me | Ph | 4-CF₃ |
| 1-9 | H | H | Me | Ph | 3-CF₃ |
| 1-10 | H | H | Me | Ph | 4-Me |
| 1-11 | H | H | Me | Ph | 3,4-Me₂ |
| 1-12 | H | H | Me | Ph | 3,4-Cl₂ |
| 1-13 | H | H | Me | 4-MeO-Ph | 4-Me |
| 1-14 | H | H | Me | 4-MeO-Ph | H |
| 1-15 | Me | H | Me | Ph | H |
| 1-16 | H | H | Me | 4-Me-Ph | 4-Me |
| 1-17 | H | H | Me | 4-Me-Ph | 4-Cl |
| 1-18 | H | H | Me | 4-Me-Ph | H |
| 1-19 | H | H | Me | 3-Cl-Ph | H |
| 1-20 | H | H | Me | 3-CF₃-Ph | H |
| 1-21 | H | H | Me | 3-CF₃-Ph | 4-Me |
| 1-22 | H | H | Me | 3,4-Cl₂-Ph | 4-Me |
| 1-23 | H | H | Me | 3-Cl-Ph | 4-Me |
| 1-24 | H | H | Me | 2-Cl-Ph | 4-Me |
| 1-25 | H | H | Me | 2,4-Cl₂-Ph | 4-Me |
| 1-26 | H | H | Me | 4-CF₃-Ph | 4-Me |
| 1-27 | H | H | Me | 4-Cl-Ph | 4-Me |
| 1-28 | H | H | Me | 4-Cl-Ph | H |
| 1-29 | H | H | Me | 3,4-Cl₂-Ph | H |
| 1-30 | H | H | Me | 4-CF₃-Ph | H |
| 1-31 | H | H | Me | 4-Cl-Ph | 4-Cl |
| 1-32 | H | H | Me | Ph | 4-Cl |
| 1-33 | H | H | Me | 2-Cl-Ph | H |
| 1-34 | H | H | Me | 4-tBu-Ph | 4-Me |
| 1-35 | H | H | Me | 3,5-Me₂-Ph | 4-Me |
| 1-36 | H | H | Me | Ph | 4-OMe |
| 1-37 | H | H | Me | 4-Cl-Ph | 4-OMe |
| 1-38 | H | H | Me | 4-Me-Ph | 4-Me |
| 1-39 | H | H | Me | 4-F-Ph | 4-Cl |
| 1-40 | H | H | Me | 4-F-Ph | 4-Me |
| 1-41 | H | H | Me | 3-Me-Ph | 4-Me |
| 1-42 | H | H | Me | 4-(COOH)-Ph | 4-Me |
| 1-43 | H | H | Me | 3-Br-Ph | 4-Me |
| 1-44 | H | H | Me | 4-Ph-Ph | 4-Me |
| 1-45 | H | H | Me | 4-(COOH)-Ph | H |
| 1-46 | H | H | Me | 3,5-Me₂-Ph | H |
| 1-47 | H | H | Me | Ph | 4-SMe |
| 1-48 | H | H | Me | 4-Cl-Ph | 4-SMe |
| 1-49 | H | H | Me | 3-Cl-4-Me-Ph | H |
| 1-50 | H | H | Me | 3-CF₃-4-Cl-Ph | H |
| 1-51 | H | H | Me | 3-CF₃-4-Cl-Ph | 4-Me |
| 1-52 | H | H | Me | 3-Cl-4-Me-Ph | 4-Me |
| 1-53 | H | H | Me | 2-Pyridyl | 4-Cl |
| 1-54 | H | H | Me | 4-Cl-Ph | 4-F |
| 1-55 | H | H | Me | 2-Thienyl | 4-Me |
| 1-56 | H | H | Me | 3-Me-2-thienyl | 4-Me |
| 1-57 | H | H | Me | 4-Me-2-thienyl | 4-Me |
| 1-58 | H | H | Me | 5-Cl-2-thienyl | 4-Me |
| 1-59 | H | H | Me | 5-Cl-2-thienyl | 4-Cl |
| 1-60 | H | H | Me | 3-Thienyl | 4-Me |
| 1-61 | H | H | Me | 2-Thienyl | H |
| 1-62 | H | H | Me | 3-Me-2-thienyl | H |
| 1-63 | H | H | Me | 4-Me-2-thienyl | H |
| 1-64 | H | H | Me | 5-Cl-2-thienyl | H |
| 1-65 | H | H | Me | 5-Me-2-thienyl | H |
| 1-66 | H | H | Me | 6-MeO-pyridin-3-yl | H |
| 1-67 | H | H | Me | 5-Br-2-thienyl | H |
| 1-68 | H | H | Me | 5-Br-2-thienyl | 4-Me |
| 1-69 | H | H | Me | 3-Thienyl | H |
| 1-70 | H | H | Me | 4-Cl-Ph | 4-S(O)Me |
| 1-71 | H | H | Me | 4-Br-Ph | 4-Me |
| 1-72 | H | H | Me | 1,3-Benzodioxol-5-yl | 4-Me |
| 1-73 | H | H | Me | 4-J-Ph | 4-Me |
| 1-74 | H | H | Me | 3,5-Cl₂-Ph | 4-Me |
| 1-75 | H | H | Me | 4-PhO-Ph | 4-Me |
| 1-76 | H | H | Me | 6-OH-pyridin-3-yl | H |
| 1-77 | H | H | Me | Ph | 4-S(O)Me |
| 1-78 | H | H | H | Ph | H |
| 1-79 | H | H | H | Ph | 4-Me |
| 1-80 | H | H | Et | Ph | H |
| 1-81 | H | H | n-Pr | Ph | H |
| 1-82 | H | H | CH₂Cl | Ph | H |
| 1-83 | H | H | CHCl₂ | Ph | H |
| 1-84 | H | H | CH₂F | Ph | H |
| 1-85 | H | H | CHF₂ | Ph | H |
| 1-86 | H | H | Cl | Ph | H |
| 1-87 | H | H | Et | Ph | 4-Me |
| 1-88 | H | H | n-Pr | Ph | 4-Me |
| 1-89 | H | H | CH₂Cl | Ph | 4-Me |
| 1-90 | H | H | CHCl₂ | Ph | 4-Me |
| 1-91 | H | H | CH₂F | Ph | 4-Me |
| 1-92 | H | H | CHF₂ | Ph | 4-Me |
| 1-93 | H | H | Cl | Ph | 4-Me |
| 1-94 | H | H | Et | 4-Cl-Ph | H |
| 1-95 | H | H | n-Pr | 4-Cl-Ph | H |
| 1-96 | H | H | CH₂Cl | 4-Cl-Ph | H |
| 1-97 | H | H | CHCl₂ | 4-Cl-Ph | H |
| 1-98 | H | H | CH₂F | 4-Cl-Ph | H |
| 1-99 | H | H | CHF₂ | 4-Cl-Ph | H |
| 1-100 | H | H | Cl | 4-Cl-Ph | H |
| 1-101 | H | H | Et | 4-Me-Ph | H |
| 1-102 | H | H | n-Pr | 4-Me-Ph | H |
| 1-103 | H | H | CH₂Cl | 4-Me-Ph | H |
| 1-104 | H | H | CHCl₂ | 4-Me-Ph | H |
| 1-105 | H | H | CH₂F | 4-Me-Ph | H |
| 1-106 | H | H | CHF₂ | 4-Me-Ph | H |
| 1-107 | H | H | Cl | 4-Me-Ph | H |
| 1-108 | H | H | Et | 2-Pyridyl | H |
| 1-109 | H | H | n-Pr | 2-Pyridyl | H |
| 1-110 | H | H | CH₂Cl | 2-Pyridyl | H |
| 1-111 | H | H | CHCl₂ | 2-Pyridyl | H |
| 1-112 | H | H | CH₂F | 2-Pyridyl | H |
| 1-113 | H | H | CHF₂ | 2-Pyridyl | H |
| 1-114 | H | H | Cl | 2-Pyridyl | H |
| 1-115 | H | H | Me | 2-Pyridyl | H |
| 1-116 | H | H | Me | 5-Cl-pyridin-2-yl | H |
| 1-117 | H | H | Me | 5-Cl-pyridin-2-yl | 4-Cl |
| 1-118 | H | H | Me | 5-Cl-pyridin-2-yl | 4-Me |
| 1-119 | H | H | Me | 5-Br-pyridin-2-yl | H |
| 1-120 | H | H | Me | 5-Br-pyridin-2-yl | 4-Cl |
| 1-121 | H | H | Me | 5-Br-pyridin-2-yl | 4-Me |
| 1-122 | H | H | Me | 5-F-pyridin-2-yl | H |
| 1-123 | H | H | Me | 5-Me-pyridin-2-yl | H |
| 1-124 | H | H | Me | 5-Me-pyridin-2-yl | 4-Me |
| 1-125 | H | H | Me | 2,4-Cl₂-Ph | H |
| 1-126 | H | H | Me | 4-CH₂COOH-Ph | 4-Me |
| 1-127 | H | H | Me | 3,4-Me₂-Ph | 4-Me |
| 1-128 | H | H | Me | 4-Br-Ph | H |
| 1-129 | H | H | Me | 3,4-Me₂-Ph | H |
| 1-130 | H | H | Me | 3-Me-Ph | H |
| 1-131 | H | H | Me | 4-F-Ph | H |
| 1-132 | H | H | Me | 4-(Me-CO)-Ph | H |
| 1-133 | H | H | Me | 4-tBu-Ph | H |
| 1-134 | H | H | Me | 4-Cl-3-Me-Ph | H |
| 1-135 | H | H | n-Pr | 4-Cl-Ph | 4-Me |
| 1-136 | H | H | Me | 3-Pyridyl | H |
| 1-137 | H | H | Me | 4-Pyridyl | H |
| 1-138 | H | H | C(O)OMe | Ph | H |
| 1-139 | H | H | Me | 6-Me-pyridin-3-yl | H |
| 1-140 | H | H | Me | 4-Cl-Ph | 4-SO₂Me |
| 1-141 | H | H | Me | 3-Pyridyl | 4-Me |
| 1-142 | H | H | Me | 2,3-Cl₂-Ph | 4-Me |
| 1-143 | H | H | Me | 2-Pyridyl | 4-Me |
| 1-144 | H | H | H | 4-Cl-Ph | 4-Me |
| 1-145 | H | H | Me | 6-Cl-pyridin-3-yl | H |
| 1-146 | H | H | Me | 4-Cl-Ph | 3-Me |
| 1-147 | H | H | Me | Ph | 3-Me |
| 1-148 | H | H | Me | 4-Me-pyridin-2-yl | H |
| 1-149 | H | H | Me | 4-Me-pyridin-2-yl | 4-Me |
| 1-150 | H | H | Me | 4-Me-pyridin-2-yl | 4-Cl |
| 1-151 | H | H | Me | 4-Me-pyridin-2-yl | 4-F |
| 1-152 | H | H | Me | 4-F-pyridin-2-yl | H |
| 1-153 | H | H | Me | 4-Cl-pyridin-2-yl | H |
| 1-154 | H | H | Me | 4-Br-pyridin-2-yl | H |
| 1-155 | H | H | Me | 4-OMe-pyridin-2-yl | H |
| 1-156 | H | H | Me | 5-CF₃-pyridin-2-yl | H |
| 1-157 | H | H | Me | 6-OMe-pyridin-2-yl | H |
| 1-158 | H | H | cyPr | 4-Cl-Ph | H |
| 1-159 | H | H | CN | 4-Cl-Ph | H |
| 1-160 | H | H | CN | 4-Cl-Ph | 4-Me |
| 1-161 | H | H | CN | 4-Me-Ph | H |
| 1-162 | H | H | CN | 4-Me-Ph | 4-Me |
| 1-163 | H | H | CN | Ph | H |
| 1-164 | H | H | CN | Ph | 4-Me |
| 1-165 | H | H | CN | 2-pyridyl | H |
| 1-166 | H | H | CN | 3-pyridyl | H |
| 1-167 | H | H | CN | 5-Cl-pyridin-2-yl | H |
| 1-168 | H | H | CN | 5-Br-pyridin-2-yl | H |
| 1-169 | H | H | CN | 5-F-pyridin-2-yl | H |
| 1-170 | H | H | CN | 5-Me-pyridin-2-yl | H |
| 1-171 | H | H | CN | 6-Me-pyridin-3-yl | H |
| 1-172 | H | H | CN | 4-Me-pyridin-2-yl | H |
| 1-173 | H | H | CN | 4-F-pyridin-2-yl | H |
| 1-174 | H | H | CN | 4-Cl-pyridin-2-yl | H |
| 1-175 | H | H | CN | 4-Br-pyridin-2-yl | H |
| 1-176 | H | H | CN | 4-OMe-pyridin-2-yl | H |
| 1-177 | H | H | formyl | 4-Cl-Ph | H |
| 1-178 | H | H | formyl | 4-Cl-Ph | 4-Me |
| 1-179 | H | H | formyl | 4-Me-Ph | H |
| 1-180 | H | H | formyl | 4-Me-Ph | 4-Me |
| 1-181 | H | H | formyl | Ph | H |
| 1-182 | H | H | formyl | Ph | 4-Me |
| 1-183 | H | H | formyl | 2-pyridyl | H |
| 1-184 | H | H | formyl | 3-pyridyl | H |
| 1-185 | H | H | formyl | 5-Cl-pyridin-2-yl | H |
| 1-186 | H | H | formyl | 5-Br-pyridin-2-yl | H |
| 1-187 | H | H | formyl | 5-F-pyridin-2-yl | H |
| 1-188 | H | H | formyl | 5-Me-pyridin-2-yl | H |
| 1-189 | H | H | formyl | 6-Me-pyridin-3-yl | H |
| 1-190 | H | H | formyl | 4-Me-pyridin-2-yl | H |
| 1-191 | H | H | formyl | 4-F-pyridin-2-yl | H |
| 1-192 | H | H | formyl | 4-Cl-pyridin-2-yl | H |
| 1-193 | H | H | formyl | 4-Br-pyridin-2-yl | H |
| 1-194 | H | H | formyl | 4-OMe-pyridin-2-yl | H |
| 1-195 | H | H | CH₂OH | 5-Me-pyridin-2-yl | H |
| 1-196 | H | H | CH₂OH | 4-Cl-Ph | H |
| 1-197 | H | H | CH₂OH | 4-Me-pyridin-2-yl | H |
| 1-198 | H | H | CH₂OH | 4-Me-Ph | H |
| 1-199 | H | H | CH₂OH | Ph | H |
| 1-200 | H | H | CH₂OH | 2-Pyridyl | H |
| 1-201 | H | H | Me | 2-Thiazolyl | H |
| 1-202 | H | H | Me | 2-Thiazolyl | 4-Cl |
| 1-203 | H | H | Me | 2-Thiazolyl | 4-Me |
| 1-204 | H | H | Me | 4-Me-thiazol-2-yl | H |
| 1-205 | H | H | Me | 4-Me-thiazol-2-yl | 4-Cl |
| 1-206 | H | H | Me | 4-Me-thiazol-2-yl | 4-Me |
| 1-207 | H | H | Me | 5-Me-thiazol-2-yl | H |
| 1-208 | H | H | Me | 5-Br-thiazol-2-yl | H |
| 1-209 | H | H | Me | 5-Br-thiazol-2-yl | 4-Me |
| 1-210 | H | H | Me | 5-Cl-thiazol-2-yl | H |
| 1-211 | H | H | Me | 4,6-Me₂-pyridin-2-yl | H |
| 1-212 | H | H | Me | 4,6-Me₂-pyridin-2-yl | 4-Me |
| 1-213 | H | H | Me | 2-Pyridyl | 4-F |
| 1-214 | H | H | Me | 2-Pyrazinyl | H |
| 1-215 | H | H | Me | 5-Me-Pyrazin-2-yl | H |
| 1-216 | H | H | Me | 2-Pyrazinyl | 4-Me |
| 1-217 | H | H | Me | 2-(1,3-Benzothiazolyl) | H |
| 1-218 | H | H | Me | 2-(1,3-Benzothiazolyl) | 4-Me |
| 1-219 | H | H | Me | 7-Cl-(1,3-benzothiazol-2-yl) | H |
| 1-220 | H | H | Me | 3-(1,5-Me₂-pyrazolyl) | H |
| 1-221 | H | H | Me | 3-(1,5-Me₂-pyrazolyl) | 4-Me |
| 1-222 | H | H | Me | 4,5-Me₂-thiazol-2-yl | H |
| 1-223 | H | H | Me | 4,5-Cl₂-thiazol-2-yl | H |
| 1-224 | H | H | Me | 2-Pyrimidinyl | H |
| 1-225 | H | H | Me | 2-Pyrimidinyl | 4-Me |
| 1-226 | H | H | Me | 5-F-pyrimidin-2-yl | H |
| 1-227 | H | H | Me | 5-Cl-pyrimidin-2-yl | H |
| 1-228 | H | H | Me | 5-Br-pyrimidin-2-yl | H |
| 1-229 | H | H | Me | 5-Me-pyrimidin-2-yl | H |
| 1-230 | H | H | Me | 5-Me-pyrimidin-2-yl | 4-Me |
| 1-231 | H | H | Me | 4,6-Me₂-pyrimidin-2-yl | H |
| 1-232 | H | H | Me | 4,6-Me₂-pyrimidin-2-yl | 4-Me |
| 1-233 | H | H | Me | 3-Pyridazinyl | H |
| 1-234 | H | H | Me | 6-Me-Pyridazin-3-yl | H |
| 1-235 | H | H | Me | 3-(1,2,4)-Triazinyl | H |
| 1-236 | H | H | Me | 6-Me-(1,2,4)-Triazin-3-yl | H |
| 1-237 | H | H | Me | Chinolin-2-yl | H |
| 1-238 | H | H | Me | Isochinolin-3-yl | H |

**Tabelle 2: Verbindungen der Formel (la"')**

| | | | | | |
|---|---|---|---|---|---|
| 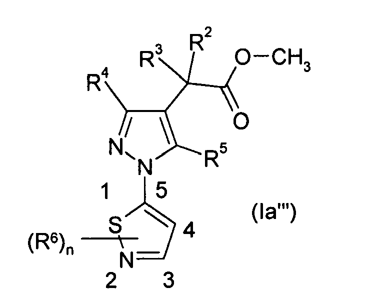 | | | | | |

| Nr. | R² | R³ | R⁴ | R⁵ | (R⁶)ₙ |
|---|---|---|---|---|---|
| 2-1 | H | H | Ph | Ph | H |
| 2-2 | H | H | Me | Ph | H |
| 2-3 | H | H | Me | 5-J-2-thienyl | H |
| 2-4 | H | H | Me | 2-Furyl | H |
| 2-5 | H | H | Me | Ph | 3-OMe |
| 2-6 | Me | H | Me | Ph | 4-Me |
| 2-7 | H | H | Me | Ph | 3-Cl |
| 2-8 | H | H | Me | Ph | 4-CF₃ |
| 2-9 | H | H | Me | Ph | 3-CF₃ |
| 2-10 | H | H | Me | Ph | 4-Me |
| 2-11 | H | H | Me | Ph | 3,4-Me₂ |
| 2-12 | H | H | Me | Ph | 3,4-Cl₂ |
| 2-13 | H | H | Me | 4-MeO-Ph | 4-Me |
| 2-14 | H | H | Me | 4-MeO-Ph | H |
| 2-15 | Me | H | Me | Ph | H |
| 2-16 | H | H | Me | 4-Me-Ph | H |
| 2-17 | H | H | Me | 4-Me-Ph | 4-Me |
| 2-18 | H | H | Me | 4-Me-Ph | 4-Cl |
| 2-19 | H | H | Me | 3-Cl-Ph | H |
| 2-20 | H | H | Me | 3-CF₃-Ph | H |
| 2-21 | H | H | Me | 3-CF₃-Ph | 4-Me |
| 2-22 | H | H | Me | 3,4-Cl₂-Ph | 4-Me |
| 2-23 | H | H | Me | 3-Cl-Ph | 4-Me |
| 2-24 | H | H | Me | 2-Cl-Ph | 4-Me |
| 2-25 | H | H | Me | 2,4-Cl₂-Ph | 4-Me |
| 2-26 | H | H | Me | 4-CF₃-Ph | 4-Me |
| 2-27 | H | H | Me | 4-Cl-Ph | 4-Me |
| 2-28 | H | H | Me | 4-Cl-Ph | H |
| 2-29 | H | H | Me | 4-Cl-Ph | 3-Cl |
| 2-30 | H | H | Me | 3,4-Cl₂-Ph | H |
| 2-31 | H | H | Me | 4-CF₃-Ph | H |
| 2-32 | H | H | Me | 4-Cl-Ph | 4-Cl |
| 2-33 | H | H | Me | Ph | 4-Cl |
| 2-34 | H | H | Me | 2-Cl-Ph | H |
| 2-35 | H | H | Me | 4-tBu-Ph | 4-Me |
| 2-36 | H | H | Me | 3,5-Me₂-Ph | 4-Me |
| 2-37 | H | H | Me | Ph | 4-OMe |
| 2-38 | H | H | Me | 4-Cl-Ph | 4-OMe |
| 2-39 | H | H | Me | 4-Me-Ph | 4-Me |
| 2-40 | H | H | Me | 4-F-Ph | 4-Me |
| 2-41 | H | H | Me | 4-F-Ph | 4-Cl |
| 2-42 | H | H | Me | 3-Me-Ph | 4-Me |
| 2-43 | H | H | Me | 4-COOH-Ph | 4-Me |
| 2-44 | H | H | Me | 3-Br-Ph | 4-Me |
| 2-45 | H | H | Me | 4-Ph-Ph | 4-Me |
| 2-46 | H | H | Me | 4-COOH-Ph | H |
| 2-47 | H | H | Me | 3,5-Me₂-Ph | H |
| 2-48 | H | H | Me | Ph | 4-SMe |
| 2-49 | H | H | Me | 4-Cl-Ph | 4-SMe |
| 2-50 | H | H | Me | 3-Cl-4-Me-Ph | H |
| 2-51 | H | H | Me | 3-CF₃-4-Cl-Ph | H |
| 2-52 | H | H | Me | 3-CF₃-4-Cl-Ph | 4-Me |
| 2-53 | H | H | Me | 3-Cl-4-Me-Ph | 4-Me |
| 2-54 | H | H | Me | 2-Pyridyl | 4-Cl |
| 2-55 | H | H | Me | 4-Cl-Ph | 4-F |
| 2-56 | H | H | Me | 2-Thienyl | 4-Me |
| 2-57 | H | H | Me | 3-Me-2-thienyl | 4-Me |
| 2-58 | H | H | Me | 4-Me-2-thienyl | 4-Me |
| 2-59 | H | H | Me | 5-Cl-2-thienyl | 4-Me |
| 2-60 | H | H | Me | 5-Cl-2-thienyl | 4-Cl |
| 2-61 | H | H | Me | 3-Thienyl | 4-Me |
| 2-62 | H | H | Me | 2-Thienyl | H |
| 2-63 | H | H | Me | 3-Me-2-thienyl | H |
| 2-64 | H | H | Me | 4-Me-2-thienyl | H |
| 2-65 | H | H | Me | 5-Cl-2-thienyl | H |
| 2-66 | H | H | Me | 5-Me-2-thienyl | H |
| 2-67 | H | H | Me | 6-MeO-pyridin-3-yl | H |
| 2-68 | H | H | Me | 5-Br-2-thienyl | H |
| 2-69 | H | H | Me | 5-Br-2-thienyl | 4-Me |
| 2-70 | H | H | Me | 3-Thienyl | H |
| 2-71 | H | H | Me | 4-Cl-Ph | 4-S(O)Me |
| 2-72 | H | H | Me | 4-Br-Ph | 4-Me |
| 2-73 | H | H | Me | 1,3-Benzodioxol-5-yl | 4-Me |
| 2-74 | H | H | Me | 4-J-Ph | 4-Me |
| 2-75 | H | H | Me | 3,5-Cl₂-Ph | 4-Me |
| 2-76 | H | H | Me | 4-PhO-Ph | 4-Me |
| 2-77 | H | H | Me | 6-OH-pyridin-3-yl | H |
| 2-78 | H | H | Me | Ph | 4-S(O)Me |
| 2-79 | H | H | H | Ph | H |
| 2-80 | H | H | H | Ph | 4-Me |
| 2-81 | H | H | Et | Ph | H |
| 2-82 | H | H | n-Pr | Ph | H |
| 2-83 | H | H | CH₂Cl | Ph | H |
| 2-84 | H | H | CHCl₂ | Ph | H |
| 2-85 | H | H | CH₂F | Ph | H |
| 2-86 | H | H | CHF₂ | Ph | H |
| 2-87 | H | H | Cl | Ph | H |
| 2-88 | H | H | Et | Ph | 4-Me |
| 2-89 | H | H | n-Pr | Ph | 4-Me |
| 2-90 | H | H | CH₂Cl | Ph | 4-Me |
| 2-91 | H | H | CHCl₂ | Ph | 4-Me |
| 2-92 | H | H | CH₂F | Ph | 4-Me |
| 2-93 | H | H | CHF₂ | Ph | 4-Me |
| 2-94 | H | H | Cl | Ph | 4-Me |
| 2-95 | H | H | Et | 4-Cl-Ph | H |
| 2-96 | H | H | n-Pr | 4-Cl-Ph | H |
| 2-97 | H | H | CH₂Cl | 4-Cl-Ph | H |
| 2-98 | H | H | CHCl₂ | 4-Cl-Ph | H |
| 2-99 | H | H | CH₂F | 4-Cl-Ph | H |
| 2-100 | H | H | CHF₂ | 4-Cl-Ph | H |
| 2-101 | H | H | Cl | 4-Cl-Ph | H |
| 2-102 | H | H | Et | 4-Me-Ph | H |
| 2-103 | H | H | n-Pr | 4-Me-Ph | H |
| 2-104 | H | H | CH₂Cl | 4-Me-Ph | H |
| 2-105 | H | H | CHCl₂ | 4-Me-Ph | H |
| 2-106 | H | H | CH₂F | 4-Me-Ph | H |
| 2-107 | H | H | CHF₂ | 4-Me-Ph | H |
| 2-108 | H | H | Cl | 4-Me-Ph | H |
| 2-109 | H | H | Et | 2-Pyridyl | H |
| 2-110 | H | H | n-Pr | 2-Pyridyl | H |
| 2-111 | H | H | CH₂Cl | 2-Pyridyl | H |
| 2-112 | H | H | CHCl₂ | 2-Pyridyl | H |
| 2-113 | H | H | CH₂F | 2-Pyridyl | H |
| 2-114 | H | H | CHF₂ | 2-Pyridyl | H |
| 2-115 | H | H | Cl | 2-Pyridyl | H |
| 2-116 | H | H | Me | 2-Pyridyl | H |
| 2-117 | H | H | Me | 5-Cl-pyridin-2-yl | H |
| 2-118 | H | H | Me | 5-Cl-pyridin-2-yl | 4-Cl |
| 2-119 | H | H | Me | 5-Cl-pyridin-2-yl | 4-Me |
| 2-120 | H | H | Me | 5-Br-pyridin-2-yl | H |
| 2-121 | H | H | Me | 5-Br-pyridin-2-yl | 4-Cl |
| 2-122 | H | H | Me | 5-Br-pyridin-2-yl | 4-Me |
| 2-123 | H | H | Me | 5-F-pyridin-2-yl | H |
| 2-124 | H | H | Me | 5-Me-pyridin-2-yl | H |
| 2-125 | H | H | Me | 5-Me-pyridin-2-yl | 4-Me |
| 2-126 | H | H | Me | 2,4-Cl₂-Ph | H |
| 2-127 | H | H | Me | 4-(CH₂COOH)-Ph | 4-Me |
| 2-128 | H | H | Me | 3,4-Me₂-Ph | 4-Me |
| 2-129 | H | H | Me | 4-Br-Ph | 4-Me |
| 2-130 | H | H | Me | 3,4-Me₂-Ph | H |
| 2-131 | H | H | Me | 3-Me-Ph | H |
| 2-132 | H | H | Me | 4-F-Ph | H |
| 2-133 | H | H | Me | 4-(Me-CO)-Ph | H |
| 2-134 | H | H | Me | 4-tBu-Ph | H |
| 2-135 | H | H | Me | 4-Cl-3-Me-Ph | H |
| 2-136 | H | H | n-Pr | 4-Cl-Ph | 4-Me |
| 2-137 | H | H | Me | 3-Pyridyl | H |
| 2-138 | H | H | Me | 4-Pyridyl | H |
| 2-139 | H | H | C(O)OMe | Ph | H |
| 2-140 | H | H | Me | 6-Me-pyridin-3-yl | H |
| 2-141 | H | H | Me | 4-Cl-Ph | 4-SO₂Me |
| 2-142 | H | H | Me | 3-Pyridyl | 4-Me |
| 2-143 | H | H | Me | 2,3-Cl₂-Ph | 4-Me |
| 2-144 | H | H | Me | 2-Pyridyl | 4-Me |
| 2-145 | H | H | H | 4-Cl-Ph | 4-Me |
| 2-146 | H | H | Me | 6-Cl-pyridin-3-yl | H |
| 2-147 | H | H | Me | 4-Cl-Ph | 3-Me |
| 2-148 | H | H | Me | Ph | 3-Me |
| 2-149 | H | H | Me | 4-Me-pyridin-2-yl | H |
| 2-150 | H | H | Me | 4-Me-pyridin-2-yl | 4-Me |
| 2-151 | H | H | Me | 4-Me-pyridin-2-yl | 4-Cl |
| 2-152 | H | H | Me | 4-Me-pyridin-2-yl | 4-F |
| 2-153 | H | H | Me | 4-F-pyridin-2-yl | H |
| 2-154 | H | H | Me | 4-Cl-pyridin-2-yl | H |
| 2-155 | H | H | Me | 4-Br-pyridin-2-yl | H |
| 2-156 | H | H | Me | 4-OMe-pyridin-2-yl | H |
| 2-157 | H | H | Me | 5-CF₃-pyridin-2-yl | H |
| 2-158 | H | H | Me | 6-OMe-pyridin-2-yl | H |
| 2-159 | H | H | cyPr | 4-Cl-Ph | H |
| 2-160 | H | H | CN | 4-Cl-Ph | H |
| 2-161 | H | H | CN | 4-Cl-Ph | 4-Me |
| 2-162 | H | H | CN | 4-Me-Ph | H |
| 2-163 | H | H | CN | 4-Me-Ph | 4-Me |
| 2-164 | H | H | CN | Ph | H |
| 2-165 | H | H | CN | Ph | 4-Me |
| 2-166 | H | H | CN | 2-pyridyl | H |
| 2-167 | H | H | CN | 3-pyridyl | H |
| 2-168 | H | H | CN | 5-Cl-pyridin-2-yl | H |
| 2-169 | H | H | CN | 5-Br-pyridin-2-yl | H |
| 2-170 | H | H | CN | 5-F-pyridin-2-yl | H |
| 2-171 | H | H | CN | 5-Me-pyridin-2-yl | H |
| 2-172 | H | H | CN | 6-Me-pyridin-3-yl | H |
| 2-173 | H | H | CN | 4-Me-pyridin-2-yl | H |
| 2-174 | H | H | CN | 4-F-pyridin-2-yl | H |
| 2-175 | H | H | CN | 4-Cl-pyridin-2-yl | H |
| 2-176 | H | H | CN | 4-Br-pyridin-2-yl | H |
| 2-177 | H | H | CN | 4-OMe-pyridin-2-yl | H |
| 2-178 | H | H | formyl | 4-Cl-Ph | H |
| 2-179 | H | H | formyl | 4-Cl-Ph | 4-Me |
| 2-180 | H | H | formyl | 4-Me-Ph | H |
| 2-181 | H | H | formyl | 4-Me-Ph | 4-Me |
| 2-182 | H | H | formyl | Ph | H |
| 2-183 | H | H | formyl | Ph | 4-Me |
| 2-184 | H | H | formyl | 2-pyridyl | H |
| 2-185 | H | H | formyl | 3-pyridyl | H |
| 2-186 | H | H | formyl | 5-Cl-pyridin-2-yl | H |
| 2-187 | H | H | formyl | 5-Br-pyridin-2-yl | H |
| 2-188 | H | H | formyl | 5-F-pyridin-2-yl | H |
| 2-189 | H | H | formyl | 5-Me-pyridin-2-yl | H |
| 2-190 | H | H | formyl | 6-Me-pyridin-3-yl | H |
| 2-191 | H | H | formyl | 4-Me-pyridin-2-yl | H |
| 2-192 | H | H | formyl | 4-F-pyridin-2-yl | H |
| 2-193 | H | H | formyl | 4-Cl-pyridin-2-yl | H |
| 2-194 | H | H | formyl | 4-Br-pyridin-2-yl | H |
| 2-195 | H | H | formyl | 4-OMe-pyridin-2-yl | H |
| 2-196 | H | H | CH₂OH | 5-Me-pyridin-2-yl | H |
| 2-197 | H | H | CH₂OH | 4-Cl-Ph | H |
| 2-198 | H | H | CH₂OH | 4-Me-pyridin-2-yl | H |
| 2-199 | H | H | CH₂OH | 4-Me-Ph | H |
| 2-200 | H | H | CH₂OH | Ph | H |
| 2-201 | H | H | CH₂OH | 2-Pyridyl | H |
| 2-202 | H | H | Me | 2-Thiazolyl | H |
| 2-203 | H | H | Me | 2-Thiazolyl | 4-Cl |
| 2-204 | H | H | Me | 2-Thiazolyl | 4-Me |
| 2-205 | H | H | Me | 4-Me-thiazol-2-yl | H |
| 2-206 | H | H | Me | 4-Me-thiazol-2-yl | 4-Cl |
| 2-207 | H | H | Me | 4-Me-thiazol-2-yl | 4-Me |
| 2-208 | H | H | Me | 5-Me-thiazol-2-yl | H |
| 2-209 | H | H | Me | 5-Br-thiazol-2-yl | H |
| 2-210 | H | H | Me | 5-Br-thiazol-2-yl | 4-Me |
| 2-211 | H | H | Me | 5-Cl-thiazol-2-yl | H |
| 2-212 | H | H | Me | 4,6-Me₂-pyridin-2-yl | H |
| 2-213 | H | H | Me | 4,6-Me₂-pyridin-2-yl | 4-Me |
| 2-214 | H | H | Me | 2-Pyridyl | 4-F |
| 2-215 | H | H | Me | 2-Pyrazinyl | H |
| 2-216 | H | H | Me | 5-Me-Pyrazin-2-yl | H |
| 2-217 | H | H | Me | 2-Pyrazinyl | 4-Me |
| 2-218 | H | H | Me | 2-(1,3-Benzothiazolyl) | H |
| 2-219 | H | H | Me | 2-(1,3-Benzothiazolyl) | 4-Me |
| 2-220 | H | H | Me | 7-Cl-(1,3-benzothiazol-2-yl) | H |
| 2-221 | H | H | Me | 3-(1,5-Me₂-pyrazolyl) | H |
| 2-222 | H | H | Me | 3-(1,5-Me₂-pyrazolyl) | 4-Me |
| 2-223 | H | H | Me | 4,5-Me₂-thiazol-2-yl | H |
| 2-224 | H | H | Me | 4,5-Cl₂-thiazol-2-yl | H |
| 2-225 | H | H | Me | 2-Pyrimidinyl | H |
| 2-226 | H | H | Me | 2-Pyrimidinyl | 4-Me |
| 2-227 | H | H | Me | 5-F-pyrimidin-2-yl | H |
| 2-228 | H | H | Me | 5-Cl-pyrimidin-2-yl | H |
| 2-229 | H | H | Me | 5-Br-pyrimidin-2-yl | H |
| 2-230 | H | H | Me | 5-Me-pyrimidin-2-yl | H |
| 2-231 | H | H | Me | 5-Me-pyrimidin-2-yl | 4-Me |
| 2-232 | H | H | Me | 4,6-Me₂-pyrimidin-2-yl | H |
| 2-233 | H | H | Me | 4,6-Me₂-pyrimidin-2-yl | 4-Me |
| 2-234 | H | H | Me | 3-Pyridazinyl | H |
| 2-235 | H | H | Me | 6-Me-Pyridazin-3-yl | H |
| 2-236 | H | H | Me | 3-(1,2,4)-Triazinyl | H |
| 2-237 | H | H | Me | 6-Me-(1,2,4)-Triazin-3-yl | H |
| 2-238 | H | H | Me | Chinolin-2-yl | H |
| 2-239 | H | H | Me | Isochinolin-3-yl | H |

**Tabelle 3: Verbindungen der Formel (Ia"")**

| | | | | |
|---|---|---|---|---|
| 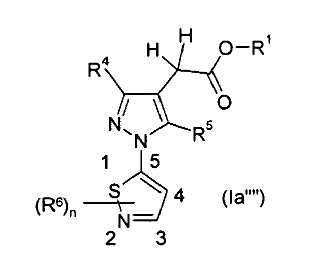 | | | | |

| Nr. | R¹ | R⁴ | R⁵ | (R⁶)ₙ |
|---|---|---|---|---|
| 3-1 | Et | Me | Ph | H |
| 3-2 | Et | Me | Ph | 4-Me |
| 3-3 | Et | Me | 3-Cl-Ph | H |
| 3-4 | Et | Me | 4-Cl-Ph | H |
| 3-5 | Et | Me | 4-Cl-Ph | 4-Me |
| 3-6 | Et | Me | 2-Thienyl | H |
| 3-7 | Et | Me | 3-Thienyl | H |
| 3-8 | Et | Me | 3-Me-2-thienyl | H |
| 3-9 | Et | Me | 4-Me-2-thienyl | H |
| 3-10 | Et | Me | 5-Br-2-thienyl | H |
| 3-11 | Et | Me | 5-Br-2-thienyl | 4-Me |
| 3-12 | Et | Me | 5-Cl-2-thienyl | H |
| 3-13 | Et | Me | 5-Cl-2-thienyl | 4-Me |
| 3-14 | Et | Me | 5-J-2-thienyl | H |
| 3-15 | Et | Me | 5-Me-2-thienyl | H |
| 3-16 | Et | Me | 3-Pyridyl | H |
| 3-17 | Et | Me | 6-MeO-pyridin-3-yl | H |
| 3-18 | Et | Me | 6-OH-pyridin-3-yl | H |
| 3-19 | Et | Me | 6-Me-pyridin-3-yl | H |
| 3-20 | Et | Me | 4-Me-Ph | H |
| 3-21 | Et | Me | 4-Me-Ph | 4-Me |
| 3-22 | Et | Me | 4-Br-Ph | H |
| 3-23 | Et | Me | 4-F-Ph | H |
| 3-24 | Et | Me | 4-F-Ph | 4-Me |
| 3-25 | Et | Me | 5-Cl-pyridin-2-yl | H |
| 3-26 | Et | Me | 5-Br-pyridin-2-yl | H |
| 3-27 | Et | Me | 5-F-pyridin-2-yl | H |
| 3-28 | Et | Me | 5-F-pyridin-2-yl | 4-Me |
| 3-29 | Et | Me | 5-Cl-pyridin-2-yl | 4-Me |
| 3-30 | Et | Me | 5-Br-pyridin-2-yl | 4-Me |
| 3-31 | Et | Me | 5-Me-pyridin-2-yl | H |
| 3-32 | Et | Me | 5-Me-pyridin-2-yl | 4-Me |
| 3-33 | Et | Me | 2-Pyridyl | 4-Me |
| 3-34 | Et | Me | 2-Pyridyl | H |
| 3-35 | Et | Me | 4-Pyridyl | H |
| 3-36 | Et | Me | 4-Me-pyridin-2-yl | H |
| 3-37 | Et | Me | 4-Me-pyridin-2-yl | 4-Me |
| 3-38 | Et | Me | 2-Thiazolyl | H |
| 3-39 | Et | Me | 4-Me-thiazol-2-yl | H |
| 3-40 | Et | Me | 5-Br-thiazol-2-yl | H |
| 3-41 | Et | Me | 5-Cl-thiazol-2-yl | H |
| 3-42 | Et | Me | 5-Me-thiazol-2-yl | H |
| 3-43 | Et | Me | 4,5-Me₂-thiazol-2-yl | H |
| 3-44 | Et | Me | 4,5-Cl₂-thiazol-2-yl | H |
| 3-45 | Et | Me | 4,6-Me₂-pyridin-2-yl | H |
| 3-46 | Et | Me | 2-Pyrazinyl | H |
| 3-47 | Et | Me | 2-Pyrimidinyl | H |
| 3-48 | Et | Me | 2-Pyrimidinyl | 4-Me |
| 3-49 | Et | Me | 5-Cl-pyrimidin-2-yl | H |
| 3-50 | Et | Me | 5-Br-pyrimidin-2-yl | H |
| 3-51 | Et | Me | 5-Me-pyrimidin-2-yl | H |
| 3-52 | Et | Me | 5-Me-pyrimidin-2-yl | 4-Me |
| 3-53 | Et | Me | 4,6-Me₂-pyrimidin-2-yl | H |
| 3-54 | Et | Me | 4,6-Me₂-pyrimidin-2-yl | 4-Me |
| 3-55 | Et | Me | 2-(1,3-Benzothiazolyl) | H |
| 3-56 | Et | Me | 7-Cl-(1,3-benzothiazol-2-yl) | H |
| 3-57 | Et | Me | 3-(1,5-Me₂-pyrazolyl) | H |
| 3-58 | Et | Me | 5-Me-Pyrazin-2-yl | H |
| 3-59 | Et | Me | 5-F-pyrimidin-2-yl | H |
| 3-60 | Et | Me | 4,6-Me₂-pyrimidin-2-yl | H |
| 3-61 | Et | Me | 3-Pyridazinyl | H |
| 3-62 | Et | Me | 6-Me-Pyridazin-3-yl | H |
| 3-63 | Et | Me | 3-(1,2,4)-Triazinyl | H |
| 3-64 | Et | Me | 6-Me-(1,2,4)-Triazin-3-yl | H |
| 3-65 | Et | Me | Chinolin-2-yl | H |
| 3-66 | Et | Me | Isochinolin-3-yl | H |
| 3-67 | Pr | Me | Ph | H |
| 3-68 | Pr | Me | 4-Cl-Ph | H |
| 3-69 | Pr | Me | 2-Thienyl | H |
| 3-70 | Pr | Me | 3-Pyridyl | H |
| 3-71 | Pr | Me | 6-Me-pyridin-3-yl | H |
| 3-72 | Pr | Me | 4-Me-Ph | H |
| 3-73 | Pr | Me | 4-Br-Ph | H |
| 3-74 | Pr | Me | 4-F-Ph | H |
| 3-75 | Pr | Me | 5-Cl-pyridin-2-yl | H |
| 3-76 | Pr | Me | 5-Br-pyridin-2-yl | H |
| 3-77 | Pr | Me | 5-F-pyridin-2-yl | H |
| 3-78 | Pr | Me | 5-Me-pyridin-2-yl | H |
| 3-79 | Pr | Me | 2-Pyridyl | H |
| 3-80 | Pr | Me | 4-Pyridyl | H |
| 3-81 | i-Pr | Me | Ph | H |
| 3-82 | i-Pr | Me | 4-Cl-Ph | H |
| 3-83 | i-Pr | Me | 2-Thienyl | H |
| 3-84 | i-Pr | Me | 3-Pyridyl | H |
| 3-85 | i-Pr | Me | 6-Me-pyridin-3-yl | H |
| 3-86 | i-Pr | Me | 4-Me-Ph | H |
| 3-87 | i-Pr | Me | 4-Br-Ph | H |
| 3-88 | i-Pr | Me | 4-F-Ph | H |
| 3-89 | i-Pr | Me | 5-Cl-pyridin-2-yl | H |
| 3-90 | i-Pr | Me | 5-Br-pyridin-2-yl | H |
| 3-91 | i-Pr | Me | 5-F-pyridin-2-yl | H |
| 3-92 | i-Pr | Me | 5-Me-pyridin-2-yl | H |
| 3-93 | i-Pr | Me | 2-Pyridyl | H |
| 3-94 | i-Pr | Me | 4-Pyridyl | H |
| 3-95 | CH₂Ph | Me | Ph | H |
| 3-96 | CH₂Ph | Me | 4-Cl-Ph | H |
| 3-97 | CH₂Ph | Me | 2-Thienyl | H |
| 3-98 | CH₂Ph | Me | 2-Pyridyl | H |
| 3-99 | Prop-2-in-1-yl | Me | Ph | H |
| 3-100 | Prop-2-in-1-yl | Me | 4-Cl-Ph | H |
| 3-101 | Prop-2-in-1-yl | Me | 2-Thienyl | H |
| 3-102 | Prop-2-in-1-yl | Me | 3-Thienyl | H |
| 3-103 | Prop-2-in-1-yl | Me | 3-Me-2-thienyl | H |
| 3-104 | Prop-2-in-1-yl | Me | 4-Me-2-thienyl | H |
| 3-105 | Prop-2-in-1-yl | Me | 5-Cl-2-thienyl | H |
| 3-106 | Prop-2-in-1-yl | Me | 5-Me-2-thienyl | H |
| 3-107 | Prop-2-in-1-yl | Me | 3-Pyridyl | H |
| 3-108 | Prop-2-in-1-yl | Me | 6-MeO-pyridin-3-yl | H |
| 3-109 | Prop-2-in-1-yl | H | Ph | H |
| 3-110 | Prop-2-in-1-yl | Me | 6-Me-pyridin-3-yl | H |
| 3-111 | Prop-2-in-1-yl | Me | 4-Me-Ph | H |
| 3-112 | Prop-2-in-1-yl | Me | 4-Br-Ph | H |
| 3-113 | Prop-2-in-1-yl | Me | 4-F-Ph | H |
| 3-114 | Prop-2-in-1-yl | Me | 5-Cl-pyridin-2-yl | H |
| 3-115 | Prop-2-in-1-yl | Me | 5-Br-pyridin-2-yl | H |
| 3-116 | Prop-2-in-1-yl | Me | 5-F-pyridin-2-yl | H |
| 3-117 | Prop-2-in-1-yl | Me | 5-Me-pyridin-2-yl | H |
| 3-118 | Prop-2-in-1-yl | Me | 2-Pyridyl | H |
| 3-119 | Prop-2-in-1-yl | Me | 4-Pyridyl | H |
| 3-120 | Prop-2-in-1-yl | Me | 4-Cl-Ph | 4-Me |
| 3-121 | Prop-2-in-1-yl | Me | Ph | 4-Me |
| 3-122 | Cyclopropylmethyl | Me | Ph | H |
| 3-123 | Cyclopropylmethyl | Me | 4-Cl-Ph | H |
| 3-124 | Cyclopropylmethyl | Me | 2-Thienyl | H |
| 3-125 | Cyclopropylmethyl | Me | 3-Thienyl | H |
| 3-126 | Cyclopropylmethyl | Me | 3-Me-2-thienyl | H |
| 3-127 | Cyclopropylmethyl | Me | 3-Pyridyl | H |
| 3-128 | Cyclopropylmethyl | Me | 5-Cl-2-thienyl | H |
| 3-129 | Cyclopropylmethyl | Me | 5-Me-2-thienyl | H |
| 3-130 | Cyclopropylmethyl | Me | 4-Me-2-thienyl | H |
| 3-131 | Cyclopropylmethyl | Me | 6-MeO-pyridin-3-yl | H |
| 3-132 | Cyclopropylmethyl | Me | 6-OH-pyridin-3-yl | H |
| 3-133 | Cyclopropylmethyl | Me | 6-Me-pyridin-3-yl | H |
| 3-134 | Cyclopropylmethyl | Me | 4-Me-Ph | H |
| 3-135 | Cyclopropylmethyl | Me | 4-Br-Ph | H |
| 3-136 | Cyclopropylmethyl | Me | 4-F-Ph | H |
| 3-137 | Cyclopropylmethyl | Me | 5-Cl-pyridin-2-yl | H |
| 3-138 | Cyclopropylmethyl | Me | 5-Br-pyridin-2-yl | H |
| 3-139 | Cyclopropylmethyl | Me | 5-F-pyridin-2-yl | H |
| 3-140 | Cyclopropylmethyl | Me | 5-Me-pyridin-2-yl | H |
| 3-141 | Cyclopropylmethyl | Me | 2-Pyridyl | H |
| 3-142 | Cyclopropylmethyl | Me | 4-Pyridyl | H |
| 3-143 | Cyclopropylmethyl | Me | 4-Cl-Ph | 4-Me |
| 3-144 | Cyclopropylmethyl | Me | Ph | 4-Me |
| 3-145 | Cyclopropylmethyl | H | Ph | H |
| 3-146 | Cyclopropylmethyl | H | Chinolin-2-yl | H |
| 3-147 | Cyclopropylmethyl | H | Isochinolin-3-yl | H |
| 3-148 | 3,3-Dichlor-2-fluorprop-2-en-1-yl | Me | Ph | H |
| 3-149 | 3,3-Dichlor-2-fluorprop-2-en-1-yl | Me | 4-Cl-Ph | H |
| 3-150 | 3,3-Dichlor-2-fluorprop-2-en-1-yl | Me | 2-Thienyl | H |
| 3-151 | 3,3-Dichlor-2-fluorprop-2-en-1-yl | Me | 2-Pyridyl | H |
| 3-152 | (1-Methylcyclopropyl)-methyl | Me | Ph | H |
| 3-153 | (1-Methylcyclopropyl)-methyl | Me | 4-Cl-Ph | H |
| 3-154 | (1-Methylcyclopropyl)-methyl | Me | 2-Thienyl | H |
| 3-155 | (1-Methylcyclopropyl)-methyl | Me | 2-Pyridyl | H |
| 3-156 | 4-Chlorbut-2-in-1-yl | Me | Ph | H |
| 3-157 | 4-Chlorbut-2-in-1-yl | Me | 4-Cl-Ph | H |
| 3-158 | 4-Chlorbut-2-in-1-yl | Me | 2-Thienyl | H |
| 3-159 | 4-Chlorbut-2-in-1-yl | Me | 2-Pyridyl | H |
| 3-160 | (2,2-Dichlorcyclopropyl)-methyl | Me | Ph | H |
| 3-161 | (2,2-Dichlorcyclopropyl)-methyl | Me | 4-Cl-Ph | H |
| 3-162 | (2,2-Dichlorcyclopropyl)-methyl | Me | 2-Thienyl | H |
| 3-163 | (2,2-Dichlorcyclopropyl)-methyl | Me | 2-Pyridyl | H |
| 3-164 | But-2-in-1-yl | Me | Ph | H |
| 3-165 | But-2-in-1-yl | Me | 4-Cl-Ph | H |
| 3-166 | But-2-in-1-yl | Me | 2-Thienyl | H |
| 3-167 | But-2-in-1-yl | Me | 3-Thienyl | H |
| 3-168 | But-2-in-1-yl | Me | 3-Me-2-thienyl | H |
| 3-169 | But-2-in-1-yl | Me | 4-Me-2-thienyl | H |
| 3-170 | But-2-in-1-yl | Me | 5-Cl-2-thienyl | H |
| 3-171 | But-2-in-1-yl | Me | 5-Me-2-thienyl | H |
| 3-172 | But-2-in-1-yl | Me | 3-Pyridyl | H |
| 3-173 | But-2-in-1-yl | Me | 6-MeO-pyridin-3-yl | H |
| 3-174 | But-2-in-1-yl | H | Ph | H |
| 3-175 | But-2-in-1-yl | Me | 6-Me-pyridin-3-yl | H |
| 3-176 | But-2-in-1-yl | Me | 4-Me-Ph | H |
| 3-177 | But-2-in-1-yl | Me | 4-Br-Ph | H |
| 3-178 | But-2-in-1-yl | Me | 4-F-Ph | H |
| 3-179 | But-2-in-1-yl | Me | 5-Cl-pyridin-2-yl | H |
| 3-180 | But-2-in-1-yl | Me | 5-Br-pyridin-2-yl | H |
| 3-181 | But-2-in-1-yl | Me | 5-F-pyridin-2-yl | H |
| 3-182 | But-2-in-1-yl | Me | 5-Me-pyridin-2-yl | H |
| 3-183 | But-2-in-1-yl | Me | 2-Pyridyl | H |
| 3-184 | But-2-in-1-yl | Me | 4-Pyridyl | H |
| 3-185 | But-2-in-1-yl | Me | 4-Cl-Ph | 4-Me |
| 3-186 | But-2-in-1-yl | Me | Ph | 4-Me |
| 3-187 | 1-Methylprop-2-in-1-yl | Me | Ph | H |
| 3-188 | 1-Methylprop-2-in-1-yl | Me | 4-Cl-Ph | H |
| 3-189 | 1-Methylprop-2-in-1-yl | Me | 2-Thienyl | H |
| 3-190 | 1-Methylprop-2-in-1-yl | Me | 2-Pyridyl | H |
| 3-191 | 1-Cyclopropylethyl | Me | Ph | H |
| 3-192 | 1-Cyclopropylethyl | Me | 4-Cl-Ph | H |
| 3-193 | 1-Cyclopropylethyl | Me | 2-Thienyl | H |
| 3-194 | 1-Cyclopropylethyl | Me | 2-Pyridyl | H |
| 3-195 | Allyl | Me | Ph | H |
| 3-196 | Allyl | Me | 4-Cl-Ph | H |
| 3-197 | Allyl | Me | 2-Thienyl | H |
| 3-198 | Allyl | Me | 2-Pyridyl | H |
| 3-199 | 3-Methylbut-2-en-1-yl | Me | Ph | H |
| 3-200 | 3-Methylbut-2-en-1-yl | Me | 4-Cl-Ph | H |
| 3-201 | 3-Methylbut-2-en-1-yl | Me | 2-Thienyl | H |
| 3-202 | 3-Methylbut-2-en-1-yl | Me | 2-Pyridyl | H |
| 3-203 | 2-Methylprop-2-en-1-yl | Me | Ph | H |
| 3-204 | 2-Methylprop-2-en-1-yl | Me | 4-Cl-Ph | H |
| 3-205 | 2-Methylprop-2-en-1-yl | Me | 2-Thienyl | H |
| 3-206 | 2-Methylprop-2-en-1-yl | Me | 2-Pyridyl | H |
| 3-207 | (2E)-1-Methylbut-2-en-1-yl | Me | Ph | H |
| 3-208 | (2E)-1-Methylbut-2-en-1-yl | Me | 4-Cl-Ph | H |
| 3-209 | (2E)-1-Methylbut-2-en-1-yl | Me | 2-Thienyl | H |
| 3-210 | (2E)-1-Methylbut-2-en-1-yl | Me | 2-Pyridyl | H |
| 3-211 | 3-Phenylprop-2-in-1-yl | Me | Ph | H |
| 3-212 | 3-Phenylprop-2-in-1-yl | Me | 4-Cl-Ph | H |
| 3-213 | 3-Phenylprop-2-in-1-yl | Me | 2-Thienyl | H |
| 3-214 | 3-Phenylprop-2-in-1-yl | Me | 2-Pyridyl | H |
| 3-215 | Cyclobutylmethyl | Me | Ph | H |
| 3-216 | Cyclobutylmethyl | Me | 4-Cl-Ph | H |
| 3-217 | Cyclobutylmethyl | Me | 2-Thienyl | H |
| 3-218 | Cyclobutylmethyl | Me | 2-Pyridyl | H |
| 3-219 | Cyclopentylmethyl | Me | Ph | H |
| 3-220 | Cyclopentylmethyl | Me | 4-Cl-Ph | H |
| 3-221 | Cyclopentylmethyl | Me | 2-Thienyl | H |
| 3-222 | Cyclopentylmethyl | Me | 2-Pyridyl | H |
| 3-223 | Cyclohexylmethyl | Me | Ph | H |
| 3-224 | Cyclohexylmethyl | Me | 4-Cl-Ph | H |
| 3-225 | Cyclohexylmethyl | Me | 2-Thienyl | H |
| 3-226 | Cyclohexylmethyl | Me | 2-Pyridyl | H |
| 3-227 | But-3-en-1-yl | Me | Ph | H |
| 3-228 | But-3-en-1-yl | Me | 4-Cl-Ph | H |
| 3-229 | But-3-en-1-yl | Me | 2-Thienyl | H |
| 3-230 | But-3-en-1-yl | Me | 2-Pyridyl | H |
| 3-231 | 2-Chloroprop-2-en-1-yl | Me | Ph | H |
| 3-232 | 2-Chloroprop-2-en-1-yl | Me | 4-Cl-Ph | H |
| 3-233 | 2-Chloroprop-2-en-1-yl | Me | 2-Thienyl | H |
| 3-234 | 2-Chloroprop-2-en-1-yl | Me | 3-Thienyl | H |
| 3-235 | 2-Chloroprop-2-en-1-yl | Me | 3-Me-2-thienyl | H |
| 3-236 | 2-Chloroprop-2-en-1-yl | Me | 4-Me-2-thienyl | H |
| 3-237 | 2-Chloroprop-2-en-1-yl | Me | 5-Cl-2-thienyl | H |
| 3-238 | 2-Chloroprop-2-en-1-yl | Me | 5-Me-2-thienyl | H |
| 3-239 | 2-Chloroprop-2-en-1-yl | Me | 3-Pyridyl | H |
| 3-240 | 2-Chloroprop-2-en-1-yl | Me | 6-MeO-pyridin-3-yl | H |
| 3-241 | 2-Chloroprop-2-en-1-yl | Me | 6-OH-pyridin-3-yl | H |
| 3-242 | 2-Chloroprop-2-en-1-yl | Me | 6-Me-pyridin-3-yl | H |
| 3-243 | 2-Chloroprop-2-en-1-yl | Me | 4-Me-Ph | H |
| 3-244 | 2-Chloroprop-2-en-1-yl | Me | 4-Br-Ph | H |
| 3-245 | 2-Chloroprop-2-en-1-yl | Me | 4-F-Ph | H |
| 3-246 | 2-Chloroprop-2-en-1-yl | Me | 5-Cl-pyridin-2-yl | H |
| 3-247 | 2-Chloroprop-2-en-1-yl | Me | 5-Br-pyridin-2-yl | H |
| 3-248 | 2-Chloroprop-2-en-1-yl | Me | 5-F-pyridin-2-yl | H |
| 3-249 | 2-Chloroprop-2-en-1-yl | Me | 5-Me-pyridin-2-yl | H |
| 3-250 | 2-Chloroprop-2-en-1-yl | Me | 2-Pyridyl | H |
| 3-251 | 2-Chloroprop-2-en-1-yl | Me | 4-Pyridyl | H |
| 3-252 | 2-Chloroprop-2-en-1-yl | Me | 4-Cl-Ph | 4-Me |
| 3-253 | 2-Chloroprop-2-en-1-yl | Me | Ph | 4-Me |
| 3-254 | 2-Chloroprop-2-en-1-yl | H | Ph | H |
| 3-255 | 2-Chloroprop-2-en-1-yl | H | Chinolin-2-yl | H |
| 3-256 | 2-Chloroprop-2-en-1-yl | H | Isochinolin-3-yl | H |
| 3-257 | 2-Methoxyethyl | Me | Ph | H |
| 3-258 | 2-Methoxyethyl | Me | 4-Cl-Ph | H |
| 3-259 | 2-Methoxyethyl | Me | 2-Thienyl | H |
| 3-260 | 2-Methoxyethyl | Me | 2-Pyridyl | H |
| 3-261 | Tetrahydrofuran-2-yl-methyl | Me | Ph | H |
| 3-262 | Tetrahydrofuran-2-yl-methyl | Me | 4-Cl-Ph | H |
| 3-263 | Tetrahydrofuran-2-yl-methyl | Me | 2-Thienyl | H |
| 3-264 | Tetrahydrofuran-2-yl-methyl | Me | 2-Pyridyl | H |
| 3-265 | 2-(Dimethylamino)ethyl | Me | Ph | H |
| 3-266 | 2-(Dimethylamino)ethyl | Me | 4-Cl-Ph | H |
| 3-267 | 2-(Dimethylamino)ethyl | Me | 2-Thienyl | H |
| 3-268 | 2-(Dimethylamino)ethyl | Me | 2-Pyridyl | H |
| 3-269 | Oxetan-3-yl | Me | Ph | H |
| 3-270 | Oxetan-3-yl | Me | 4-Cl-Ph | H |
| 3-271 | Oxetan-3-yl | Me | 2-Thienyl | H |
| 3-272 | Oxetan-3-yl | Me | 2-Pyridyl | H |
| 3-273 | (3-Methyloxetan-3-yl)methyl | Me | Ph | H |
| 3-274 | (3-Methyloxetan-3-yl)methyl | Me | 4-Cl-Ph | H |
| 3-275 | (3-Methyloxetan-3-yl)methyl | Me | 2-Thienyl | H |
| 3-276 | (3-Methyloxetan-3-yl)methyl | Me | 2-Pyridyl | H |
| 3-277 | 2,2,2-Trifluorethyl | Me | Ph | H |
| 3-278 | 2,2,2-Trifluorethyl | Me | 4-Cl-Ph | H |
| 3-279 | 2,2,2-Trifluorethyl | Me | 2-Thienyl | H |
| 3-280 | 2,2,2-Trifluorethyl | Me | 3-Pyridyl | H |
| 3-281 | 2,2,2-Trifluorethyl | Me | 6-Me-pyridin-3-yl | H |
| 3-282 | 2,2,2-Trifluorethyl | Me | 4-Me-Ph | H |
| 3-283 | 2,2,2-Trifluorethyl | Me | 4-Br-Ph | H |
| 3-284 | 2,2,2-Trifluorethyl | Me | 4-F-Ph | H |
| 3-285 | 2,2,2-Trifluorethyl | Me | 5-Cl-pyridin-2-yl | H |
| 3-286 | 2,2,2-Trifluorethyl | Me | 5-Br-pyridin-2-yl | H |
| 3-287 | 2,2,2-Trifluorethyl | Me | 5-F-pyridin-2-yl | H |
| 3-288 | 2,2,2-Trifluorethyl | Me | 5-Me-pyridin-2-yl | H |
| 3-289 | 2,2,2-Trifluorethyl | Me | 2-Pyridyl | H |
| 3-290 | 2,2,2-Trifluorethyl | Me | 4-Pyridyl | H |
| 3-291 | CH₂(4-Cl-Ph) | Me | Ph | H |
| 3-292 | CH₂(4-Cl-Ph) | Me | 4-Cl-Ph | H |
| 3-293 | CH₂(4-Cl-Ph) | Me | 2-Thienyl | H |
| 3-294 | CH₂(4-Cl-Ph) | Me | 3-Pyridyl | H |
| 3-295 | CH₂(4-Cl-Ph) | Me | 6-Me-pyridin-3-yl | H |
| 3-296 | CH₂(4-Cl-Ph) | Me | 4-Me-Ph | H |
| 3-297 | CH₂(4-Cl-Ph) | Me | 4-Br-Ph | H |
| 3-298 | CH₂(4-Cl-Ph) | Me | 4-F-Ph | H |
| 3-299 | CH₂(4-Cl-Ph) | Me | 5-Cl-pyridin-2-yl | H |
| 3-300 | CH₂(4-Cl-Ph) | Me | 5-Br-pyridin-2-yl | H |
| 3-301 | CH₂(4-Cl-Ph) | Me | 5-F-pyridin-2-yl | H |
| 3-302 | CH₂(4-Cl-Ph) | Me | 5-Me-pyridin-2-yl | H |
| 3-303 | CH₂(4-Cl-Ph) | Me | 2-Pyridyl | H |
| 3-304 | CH₂(4-Cl-Ph) | Me | 4-Pyridyl | H |
| 3-305 | CH₂(4-F-Ph) | Me | Ph | H |
| 3-306 | CH₂(4-F-Ph) | Me | 4-Cl-Ph | H |
| 3-307 | CH₂(4-F-Ph) | Me | 2-Thienyl | H |
| 3-308 | CH₂(4-F-Ph) | Me | 3-Pyridyl | H |
| 3-309 | CH₂(4-F-Ph) | Me | 6-Me-pyridin-3-yl | H |
| 3-310 | CH₂(4-F-Ph) | Me | 4-Me-Ph | H |
| 3-311 | CH₂(4-F-Ph) | Me | 4-Br-Ph | H |
| 3-312 | CH₂(4-F-Ph) | Me | 4-F-Ph | H |
| 3-313 | CH₂(4-F-Ph) | Me | 5-Cl-pyridin-2-yl | H |
| 3-314 | CH₂(4-F-Ph) | Me | 5-Br-pyridin-2-yl | H |
| 3-315 | CH₂(4-F-Ph) | Me | 5-F-pyridin-2-yl | H |
| 3-316 | CH₂(4-F-Ph) | Me | 5-Me-pyridin-2-yl | H |
| 3-317 | CH₂(4-F-Ph) | Me | 2-Pyridyl | H |
| 3-318 | CH₂(4-F-Ph) | Me | 4-Pyridyl | H |
| 3-319 | CH₂(4-OMe-Ph) | Me | Ph | H |
| 3-320 | CH₂(4-OMe-Ph) | Me | 4-Cl-Ph | H |
| 3-321 | CH₂(4-OMe-Ph) | Me | 2-Thienyl | H |
| 3-322 | CH₂(4-OMe-Ph) | Me | 3-Pyridyl | H |
| 3-323 | CH₂(4-OMe-Ph) | Me | 6-Me-pyridin-3-yl | H |
| 3-324 | CH₂(4-OMe-Ph) | Me | 4-Me-Ph | H |
| 3-325 | CH₂(4-OMe-Ph) | Me | 4-Br-Ph | H |
| 3-326 | CH₂(4-OMe-Ph) | Me | 4-F-Ph | H |
| 3-327 | CH₂(4-OMe-Ph) | Me | 5-Cl-pyridin-2-yl | H |
| 3-328 | CH₂(4-OMe-Ph) | Me | 5-Br-pyridin-2-yl | H |
| 3-329 | CH₂(4-OMe-Ph) | Me | 5-F-pyridin-2-yl | H |
| 3-330 | CH₂(4-OMe-Ph) | Me | 5-Me-pyridin-2-yl | H |
| 3-331 | CH₂(4-OMe-Ph) | Me | 2-Pyridyl | H |
| 3-332 | CH₂(4-OMe-Ph) | Me | 4-Pyridyl | H |
| 3-333 | 2,2-Difluorethyl | Me | Ph | H |
| 3-334 | 2,2-Difluorethyl | Me | 4-Cl-Ph | H |
| 3-335 | 2,2-Difluorethyl | Me | 2-Thienyl | H |
| 3-336 | 2,2-Difluorethyl | Me | 2-Pyridyl | H |
| 3-337 | Ph | Me | Ph | H |
| 3-338 | Ph | Me | 4-Cl-Ph | H |
| 3-339 | Ph | Me | 2-Thienyl | H |
| 3-340 | Ph | Me | 2-Pyridyl | H |
| 3-341 | 2-Fluorethyl | Me | Ph | H |
| 3-342 | 2-Fluorethyl | Me | 4-Cl-Ph | H |
| 3-343 | 2-Fluorethyl | Me | 2-Thienyl | H |
| 3-344 | 2-Fluorethyl | Me | 2-Pyridyl | H |
| 3-345 | 2,2,3,3,3-Pentafluorpropyl | Me | Ph | H |
| 3-346 | 2,2,3,3,3-Pentafluorpropyl | Me | 4-Cl-Ph | H |
| 3-347 | 2,2,3,3,3-Pentafluorpropyl | Me | 2-Thienyl | H |
| 3-348 | 2,2,3,3,3-Pentafluorpropyl | Me | 2-Pyridyl | H |
| 3-349 | 1-Ethyl-5-methyl-1H-pyrazol-4-methyl | Me | Ph | H |
| 3-350 | 1-Ethyl-5-methyl-1H-pyrazol-4-methyl | Me | 4-Cl-Ph | H |
| 3-351 | 1-Ethyl-5-methyl-1H-pyrazol-4-methyl | Me | 2-Thienyl | H |
| 3-352 | 1-Ethyl-5-methyl-1H-pyrazol-4-methyl | Me | 2-Pyridyl | H |

**Tabelle 4: Verbindungen der Formel (Ib")**

| | | | | | |
|---|---|---|---|---|---|
| 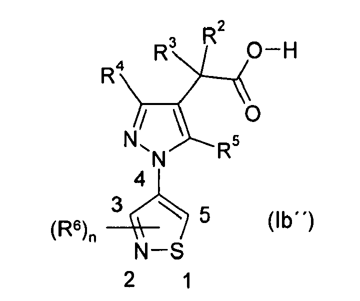 | | | | | |

| Nr. | R² | R³ | R⁴ | R⁵ | (R⁶)ₙ |
|---|---|---|---|---|---|
| 4-1 | H | H | Ph | Ph | H |
| 4-2 | H | H | Me | Ph | H |
| 4-3 | H | H | Me | 5-J-2-thienyl | H |
| 4-4 | H | H | Me | 2-Furyl | H |
| 4-5 | H | H | Me | Ph | 3-OMe |
| 4-6 | Me | H | Me | Ph | 5-Me |
| 4-7 | H | H | Me | Ph | 3-Cl |
| 4-8 | H | H | Me | Ph | 5-CF₃ |
| 4-9 | H | H | Me | Ph | 3-CF₃ |
| 4-10 | H | H | Me | Ph | 5-Me |
| 4-11 | H | H | Me | Ph | 3,5-Me₂ |
| 4-12 | H | H | Me | Ph | 3,5-Cl₂ |
| 4-13 | H | H | Me | 4-MeO-Ph | 5-Me |
| 4-14 | H | H | Me | 4-MeO-Ph | H |
| 4-15 | Me | H | Me | Ph | H |
| 4-16 | H | H | Me | 4-Me-Ph | 5-Me |
| 4-17 | H | H | Me | 4-Me-Ph | 5-Cl |
| 4-18 | H | H | Me | 4-Me-Ph | H |
| 4-19 | H | H | Me | 3-Cl-Ph | H |
| 4-20 | H | H | Me | 3-CF₃-Ph | H |
| 4-21 | H | H | Me | 3-CF₃-Ph | 4-Me |
| 4-22 | H | H | Me | 3,4-Cl₂-Ph | 5-Me |
| 4-23 | H | H | Me | 3-Cl-Ph | 5-Me |
| 4-24 | H | H | Me | 2-Cl-Ph | 5-Me |
| 4-25 | H | H | Me | 2,4-Cl₂-Ph | 5-Me |
| 4-26 | H | H | Me | 4-CF₃-Ph | 5-Me |
| 4-27 | H | H | Me | 4-Cl-Ph | 5-Me |
| 4-28 | H | H | Me | 4-Cl-Ph | H |
| 4-29 | H | H | Me | 3,4-Cl₂-Ph | H |
| 4-30 | H | H | Me | 4-CF₃-Ph | H |
| 4-31 | H | H | Me | 4-Cl-Ph | 5-Cl |
| 4-32 | H | H | Me | Ph | 5-Cl |
| 4-33 | H | H | Me | 2-Cl-Ph | H |
| 4-34 | H | H | Me | 4-tBu-Ph | 5-Me |
| 4-35 | H | H | Me | 3,5-Me₂-Ph | 5-Me |
| 4-36 | H | H | Me | Ph | 5-OMe |
| 4-37 | H | H | Me | 4-Cl-Ph | 5-OMe |
| 4-38 | H | H | Me | 4-Me-Ph | 5-Me |
| 4-39 | H | H | Me | 4-F-Ph | 5-Cl |
| 4-40 | H | H | Me | 4-F-Ph | 5-Me |
| 4-41 | H | H | Me | 3-Me-Ph | 5-Me |
| 4-42 | H | H | Me | 4-(COOH)-Ph | 5-Me |
| 4-43 | H | H | Me | 3-Br-Ph | 5-Me |
| 4-44 | H | H | Me | 4-Ph-Ph | 5-Me |
| 4-45 | H | H | Me | 4-(COOH)-Ph | H |
| 4-46 | H | H | Me | 3,5-Me₂-Ph | H |
| 4-47 | H | H | Me | Ph | 5-SMe |
| 4-48 | H | H | Me | 4-Cl-Ph | 5-SMe |
| 4-49 | H | H | Me | 3-Cl-4-Me-Ph | H |
| 4-50 | H | H | Me | 3-CF₃-4-Cl-Ph | H |
| 4-51 | H | H | Me | 3-CF₃-4-Cl-Ph | 5-Me |
| 4-52 | H | H | Me | 3-Cl-4-Me-Ph | 5-Me |
| 4-53 | H | H | Me | 2-Pyridyl | 5-Cl |
| 4-54 | H | H | Me | 4-Cl-Ph | 5-F |
| 4-55 | H | H | Me | 2-Thienyl | 5-Me |
| 4-56 | H | H | Me | 3-Me-2-thienyl | 5-Me |
| 4-57 | H | H | Me | 4-Me-2-thienyl | 5-Me |
| 4-58 | H | H | Me | 5-Cl-2-thienyl | 5-Me |
| 4-59 | H | H | Me | 5-Cl-2-thienyl | 5-Cl |
| 4-60 | H | H | Me | 3-Thienyl | 5-Me |
| 4-61 | H | H | Me | 2-Thienyl | H |
| 4-62 | H | H | Me | 3-Me-2-thienyl | H |
| 4-63 | H | H | Me | 4-Me-2-thienyl | H |
| 4-64 | H | H | Me | 5-Cl-2-thienyl | H |
| 4-65 | H | H | Me | 5-Me-2-thienyl | H |
| 4-66 | H | H | Me | 6-MeO-pyridin-3-yl | H |
| 4-67 | H | H | Me | 5-Br-2-thienyl | H |
| 4-68 | H | H | Me | 5-Br-2-thienyl | 5-Me |
| 4-69 | H | H | Me | 3-Thienyl | H |
| 4-70 | H | H | Me | 4-Cl-Ph | 5-S(O)Me |
| 4-71 | H | H | Me | 4-Br-Ph | 5-Me |
| 4-72 | H | H | Me | 1,3-Benzodioxol-5-yl | 5-Me |
| 4-73 | H | H | Me | 4-J-Ph | 5-Me |
| 4-74 | H | H | Me | 3,5-Cl₂-Ph | 5-Me |
| 4-75 | H | H | Me | 4-PhO-Ph | 5-Me |
| 4-76 | H | H | Me | 6-OH-pyridin-3-yl | H |
| 4-77 | H | H | Me | Ph | 5-S(O)Me |
| 4-78 | H | H | H | Ph | H |
| 4-79 | H | H | H | Ph | 5-Me |
| 4-80 | H | H | Et | Ph | H |
| 4-81 | H | H | n-Pr | Ph | H |
| 4-82 | H | H | CH₂Cl | Ph | H |
| 4-83 | H | H | CHCl₂ | Ph | H |
| 4-84 | H | H | CH₂F | Ph | H |
| 4-85 | H | H | CHF₂ | Ph | H |
| 4-86 | H | H | Cl | Ph | H |
| 4-87 | H | H | Et | Ph | 5-Me |
| 4-88 | H | H | n-Pr | Ph | 5-Me |
| 4-89 | H | H | CH₂Cl | Ph | 5-Me |
| 4-90 | H | H | CHCl₂ | Ph | 5-Me |
| 4-91 | H | H | CH₂F | Ph | 5-Me |
| 4-92 | H | H | CHF₂ | Ph | 5-Me |
| 4-93 | H | H | Cl | Ph | 5-Me |
| 4-94 | H | H | Et | 4-Cl-Ph | H |
| 4-95 | H | H | n-Pr | 4-Cl-Ph | H |
| 4-96 | H | H | CH₂Cl | 4-Cl-Ph | H |
| 4-97 | H | H | CHCl₂ | 4-Cl-Ph | H |
| 4-98 | H | H | CH₂F | 4-Cl-Ph | H |
| 4-99 | H | H | CHF₂ | 4-Cl-Ph | H |
| 4-100 | H | H | Cl | 4-Cl-Ph | H |
| 4-101 | H | H | Et | 4-Me-Ph | H |
| 4-102 | H | H | n-Pr | 4-Me-Ph | H |
| 4-103 | H | H | CH₂Cl | 4-Me-Ph | H |
| 4-104 | H | H | CHCl₂ | 4-Me-Ph | H |
| 4-105 | H | H | CH₂F | 4-Me-Ph | H |
| 4-106 | H | H | CHF₂ | 4-Me-Ph | H |
| 4-107 | H | H | Cl | 4-Me-Ph | H |
| 4-108 | H | H | Et | 2-Pyridyl | H |
| 4-109 | H | H | n-Pr | 2-Pyridyl | H |
| 4-110 | H | H | CH₂Cl | 2-Pyridyl | H |
| 4-111 | H | H | CHCl₂ | 2-Pyridyl | H |
| 4-112 | H | H | CH₂F | 2-Pyridyl | H |
| 4-113 | H | H | CHF₂ | 2-Pyridyl | H |
| 4-114 | H | H | Cl | 2-Pyridyl | H |
| 4-115 | H | H | Me | 2-Pyridyl | H |
| 4-116 | H | H | Me | 5-Cl-pyridin-2-yl | H |
| 4-117 | H | H | Me | 5-Cl-pyridin-2-yl | 5-Cl |
| 4-118 | H | H | Me | 5-Cl-pyridin-2-yl | 5-Me |
| 4-119 | H | H | Me | 5-Br-pyridin-2-yl | H |
| 4-120 | H | H | Me | 5-Br-pyridin-2-yl | 5-Cl |
| 4-121 | H | H | Me | 5-Br-pyridin-2-yl | 5-Me |
| 4-122 | H | H | Me | 5-F-pyridin-2-yl | H |
| 4-123 | H | H | Me | 5-Me-pyridin-2-yl | H |
| 4-124 | H | H | Me | 5-Me-pyridin-2-yl | 5-Me |
| 4-125 | H | H | Me | 2,4-Cl₂-Ph | H |
| 4-126 | H | H | Me | 4-CH₂COOH-Ph | 5-Me |
| 4-127 | H | H | Me | 3,4-Me₂-Ph | 5-Me |
| 4-128 | H | H | Me | 4-Br-Ph | H |
| 4-129 | H | H | Me | 3,4-Me₂-Ph | H |
| 4-130 | H | H | Me | 3-Me-Ph | H |
| 4-131 | H | H | Me | 4-F-Ph | H |
| 4-132 | H | H | Me | 4-(Me-CO)-Ph | H |
| 4-133 | H | H | Me | 4-tBu-Ph | H |
| 4-134 | H | H | Me | 4-Cl-3-Me-Ph | H |
| 4-135 | H | H | n-Pr | 4-Cl-Ph | 5-Me |
| 4-136 | H | H | Me | 3-Pyridyl | H |
| 4-137 | H | H | Me | 4-Pyridyl | H |
| 4-138 | H | H | C(O)OMe | Ph | H |
| 4-139 | H | H | Me | 6-Me-pyridin-3-yl | H |
| 4-140 | H | H | Me | 4-Cl-Ph | 5-SO₂Me |
| 4-141 | H | H | Me | 3-Pyridyl | 5-Me |
| 4-142 | H | H | Me | 2,3-Cl₂-Ph | 5-Me |
| 4-143 | H | H | Me | 2-Pyridyl | 5-Me |
| 4-144 | H | H | H | 4-Cl-Ph | 5-Me |
| 4-145 | H | H | Me | 6-Cl-pyridin-3-yl | H |
| 4-146 | H | H | Me | Ph | 5-Me |
| 4-147 | H | H | Me | 4-Me-pyridin-2-yl | H |
| 4-148 | H | H | Me | 4-Me-pyridin-2-yl | 5-Me |
| 4-149 | H | H | Me | 4-Me-pyridin-2-yl | 5-Cl |
| 4-150 | H | H | Me | 4-Me-pyridin-2-yl | 5-F |
| 4-151 | H | H | Me | 4-F-pyridin-2-yl | H |
| 4-152 | H | H | Me | 4-Cl-pyridin-2-yl | H |
| 4-153 | H | H | Me | 4-Br-pyridin-2-yl | H |
| 4-154 | H | H | Me | 4-OMe-pyridin-2-yl | H |
| 4-155 | H | H | Me | 5-CF₃-pyridin-2-yl | H |
| 4-156 | H | H | Me | 6-OMe-pyridin-2-yl | H |
| 4-157 | H | H | cyPr | 4-Cl-Ph | H |
| 4-158 | H | H | CN | 4-Cl-Ph | H |
| 4-159 | H | H | CN | 4-Cl-Ph | 5-Me |
| 4-160 | H | H | CN | 4-Me-Ph | H |
| 4-161 | H | H | CN | 4-Me-Ph | 5-Me |
| 4-162 | H | H | CN | Ph | H |
| 4-163 | H | H | CN | Ph | 5-Me |
| 4-164 | H | H | CN | 2-pyridyl | H |
| 4-165 | H | H | CN | 3-pyridyl | H |
| 4-166 | H | H | CN | 5-Cl-pyridin-2-yl | H |
| 4-167 | H | H | CN | 5-Br-pyridin-2-yl | H |
| 4-168 | H | H | CN | 5-F-pyridin-2-yl | H |
| 4-169 | H | H | CN | 5-Me-pyridin-2-yl | H |
| 4-170 | H | H | CN | 6-Me-pyridin-3-yl | H |
| 4-171 | H | H | CN | 4-Me-pyridin-2-yl | H |
| 4-172 | H | H | CN | 4-F-pyridin-2-yl | H |
| 4-173 | H | H | CN | 4-Cl-pyridin-2-yl | H |
| 4-174 | H | H | CN | 4-Br-pyridin-2-yl | H |
| 4-175 | H | H | CN | 4-OMe-pyridin-2-yl | H |
| 4-176 | H | H | formyl | 4-Cl-Ph | H |
| 4-177 | H | H | formyl | 4-Cl-Ph | 5-Me |
| 4-178 | H | H | formyl | 4-Me-Ph | H |
| 4-179 | H | H | formyl | 4-Me-Ph | 5-Me |
| 4-180 | H | H | formyl | Ph | H |
| 4-181 | H | H | formyl | Ph | 5-Me |
| 4-182 | H | H | formyl | 2-pyridyl | H |
| 4-183 | H | H | formyl | 3-pyridyl | H |
| 4-184 | H | H | formyl | 5-Cl-pyridin-2-yl | H |
| 4-185 | H | H | formyl | 5-Br-pyridin-2-yl | H |
| 4-186 | H | H | formyl | 5-F-pyridin-2-yl | H |
| 4-187 | H | H | formyl | 5-Me-pyridin-2-yl | H |
| 4-188 | H | H | formyl | 6-Me-pyridin-3-yl | H |
| 4-189 | H | H | formyl | 4-Me-pyridin-2-yl | H |
| 4-190 | H | H | formyl | 4-F-pyridin-2-yl | H |
| 4-191 | H | H | formyl | 4-Cl-pyridin-2-yl | H |
| 4-192 | H | H | formyl | 4-Br-pyridin-2-yl | H |
| 4-193 | H | H | formyl | 4-OMe-pyridin-2-yl | H |
| 4-194 | H | H | CH₂OH | 5-Me-pyridin-2-yl | H |
| 4-195 | H | H | CH₂OH | 4-Cl-Ph | H |
| 4-196 | H | H | CH₂OH | 4-Me-pyridin-2-yl | H |
| 4-197 | H | H | CH₂OH | 4-Me-Ph | H |
| 4-198 | H | H | CH₂OH | Ph | H |
| 4-199 | H | H | CH₂OH | 2-Pyridyl | H |
| 4-200 | H | H | Me | 2-Thiazolyl | H |
| 4-201 | H | H | Me | 2-Thiazolyl | 5-Cl |
| 4-202 | H | H | Me | 2-Thiazolyl | 5-Me |
| 4-203 | H | H | Me | 4-Me-thiazol-2-yl | H |
| 4-204 | H | H | Me | 4-Me-thiazol-2-yl | 5-Cl |
| 4-205 | H | H | Me | 4-Me-thiazol-2-yl | 5-Me |
| 4-206 | H | H | Me | 5-Me-thiazol-2-yl | H |
| 4-207 | H | H | Me | 5-Br-thiazol-2-yl | H |
| 4-208 | H | H | Me | 5-Br-thiazol-2-yl | 5-Me |
| 4-209 | H | H | Me | 5-Cl-thiazol-2-yl | H |
| 4-210 | H | H | Me | 4,6-Me₂-pyridin-2-yl | H |
| 4-211 | H | H | Me | 4,6-Me₂-pyridin-2-yl | 5-Me |
| 4-212 | H | H | Me | 2-Pyridyl | 5-F |
| 4-213 | H | H | Me | 2-Pyrazinyl | H |
| 4-214 | H | H | Me | 5-Me-Pyrazin-2-yl | H |
| 4-215 | H | H | Me | 2-Pyrazinyl | 5-Me |
| 4-216 | H | H | Me | 2-(1,3-Benzothiazolyl) | H |
| 4-217 | H | H | Me | 2-(1,3-Benzothiazolyl) | 5-Me |
| 4-218 | H | H | Me | 7-Cl-(1,3-benzothiazol-2-yl) | H |
| 4-219 | H | H | Me | 3-(1,5-Me₂-pyrazolyl) | H |
| 4-220 | H | H | Me | 3-(1,5-Me₂-pyrazolyl) | 5-Me |
| 4-221 | H | H | Me | 4,5-Me₂-thiazol-2-yl | H |
| 4-222 | H | H | Me | 4,5-Cl₂-thiazol-2-yl | H |
| 4-223 | H | H | Me | 2-Pyrimidinyl | H |
| 4-224 | H | H | Me | 2-Pyrimidinyl | 5-Me |
| 4-225 | H | H | Me | 5-F-pyrimidin-2-yl | H |
| 4-226 | H | H | Me | 5-Cl-pyrimidin-2-yl | H |
| 4-227 | H | H | Me | 5-Br-pyrimidin-2-yl | H |
| 4-228 | H | H | Me | 5-Me-pyrimidin-2-yl | H |
| 4-229 | H | H | Me | 5-Me-pyrimidin-2-yl | 5-Me |
| 4-230 | H | H | Me | 4,6-Me₂-pyrimidin-2-yl | H |
| 4-231 | H | H | Me | 4,6-Me₂-pyrimidin-2-yl | 5-Me |
| 4-232 | H | H | Me | 3-Pyridazinyl | H |
| 4-233 | H | H | Me | 6-Me-Pyridazin-3-yl | H |
| 4-234 | H | H | Me | 3-(1,2,4)-Triazinyl | H |
| 4-235 | H | H | Me | 6-Me-(1,2,4)-Triazin-3-yl | H |
| 4-236 | H | H | Me | 4-Cl-Ph | 3-COOMe |
| 4-237 | H | H | Me | 4-Cl-Ph | 3,5-(COOMe)₂ |
| 4-238 | H | H | Me | Chinolin-2-yl | H |
| 4-239 | H | H | Me | Isochinolin-3-yl | H |

**Tabelle 5: Verbindungen der Formel (Ib"')**

| | | | | | |
|---|---|---|---|---|---|
| 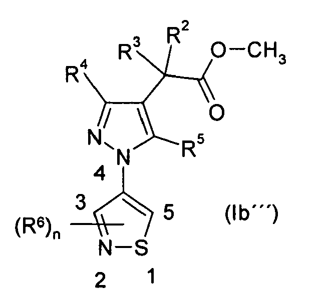 | | | | | |

| Nr. | R² | R³ | R⁴ | R⁵ | (R⁶)ₙ |
|---|---|---|---|---|---|
| 5-1 | H | H | Ph | Ph | H |
| 5-2 | H | H | Me | Ph | H |
| 5-3 | H | H | Me | 5-J-2-thienyl | H |
| 5-4 | H | H | Me | 2-Furyl | H |
| 5-5 | H | H | Me | Ph | 3-OMe |
| 5-6 | Me | H | Me | Ph | 5-Me |
| 5-7 | H | H | Me | Ph | 3-Cl |
| 5-8 | H | H | Me | Ph | 5-CF₃ |
| 5-9 | H | H | Me | Ph | 3-CF₃ |
| 5-10 | H | H | Me | Ph | 5-Me |
| 5-11 | H | H | Me | Ph | 3,5-Me₂ |
| 5-12 | H | H | Me | Ph | 3,5-Cl₂ |
| 5-13 | H | H | Me | 4-MeO-Ph | 5-Me |
| 5-14 | H | H | Me | 4-MeO-Ph | H |
| 5-15 | Me | H | Me | Ph | H |
| 5-16 | H | H | Me | 4-Me-Ph | H |
| 5-17 | H | H | Me | 4-Me-Ph | 5-Me |
| 5-18 | H | H | Me | 4-Me-Ph | 5-Cl |
| 5-19 | H | H | Me | 3-Cl-Ph | H |
| 5-20 | H | H | Me | 3-CF₃-Ph | H |
| 5-21 | H | H | Me | 3-CF₃-Ph | 5-Me |
| 5-22 | H | H | Me | 3,4-Cl₂-Ph | 5-Me |
| 5-23 | H | H | Me | 3-Cl-Ph | 5-Me |
| 5-24 | H | H | Me | 2-Cl-Ph | 5-Me |
| 5-25 | H | H | Me | 2,4-Cl₂-Ph | 5-Me |
| 5-26 | H | H | Me | 4-CF₃-Ph | 5-Me |
| 5-27 | H | H | Me | 4-Cl-Ph | 5-Me |
| 5-28 | H | H | Me | 4-Cl-Ph | H |
| 5-29 | H | H | Me | 3,4-Cl₂-Ph | H |
| 5-30 | H | H | Me | 4-CF₃-Ph | H |
| 5-31 | H | H | Me | 4-Cl-Ph | 5-Cl |
| 5-32 | H | H | Me | Ph | 5-Cl |
| 5-33 | H | H | Me | 2-Cl-Ph | H |
| 5-34 | H | H | Me | 4-tBu-Ph | 5-Me |
| 5-35 | H | H | Me | 3,5-Me₂-Ph | 5-Me |
| 5-36 | H | H | Me | Ph | 5-OMe |
| 5-37 | H | H | Me | 4-Cl-Ph | 5-OMe |
| 5-38 | H | H | Me | 4-Me-Ph | 5-Me |
| 5-39 | H | H | Me | 4-F-Ph | 5-Me |
| 5-40 | H | H | Me | 4-F-Ph | 5-Cl |
| 5-41 | H | H | Me | 3-Me-Ph | 5-Me |
| 5-42 | H | H | Me | 4-COOH-Ph | 5-Me |
| 5-43 | H | H | Me | 3-Br-Ph | 5-Me |
| 5-44 | H | H | Me | 4-Ph-Ph | 5-Me |
| 5-45 | H | H | Me | 4-COOH-Ph | H |
| 5-46 | H | H | Me | 3,5-Me₂-Ph | H |
| 5-47 | H | H | Me | Ph | 5-SMe |
| 5-48 | H | H | Me | 4-Cl-Ph | 5-SMe |
| 5-49 | H | H | Me | 3-Cl-4-Me-Ph | H |
| 5-50 | H | H | Me | 3-CF₃-4-Cl-Ph | H |
| 5-51 | H | H | Me | 3-CF₃-4-Cl-Ph | 5-Me |
| 5-52 | H | H | Me | 3-Cl-4-Me-Ph | 5-Me |
| 5-53 | H | H | Me | 2-Pyridyl | 5-Cl |
| 5-54 | H | H | Me | 4-Cl-Ph | 5-F |
| 5-55 | H | H | Me | 2-Thienyl | 5-Me |
| 5-56 | H | H | Me | 3-Me-2-thienyl | 5-Me |
| 5-57 | H | H | Me | 4-Me-2-thienyl | 5-Me |
| 5-58 | H | H | Me | 5-Cl-2-thienyl | 5-Me |
| 5-59 | H | H | Me | 5-Cl-2-thienyl | 5-Cl |
| 5-60 | H | H | Me | 3-Thienyl | 5-Me |
| 5-61 | H | H | Me | 2-Thienyl | H |
| 5-62 | H | H | Me | 3-Me-2-thienyl | H |
| 5-63 | H | H | Me | 4-Me-2-thienyl | H |
| 5-64 | H | H | Me | 5-Cl-2-thienyl | H |
| 5-65 | H | H | Me | 5-Me-2-thienyl | H |
| 5-66 | H | H | Me | 6-MeO-pyridin-3-yl | H |
| 5-67 | H | H | Me | 5-Br-2-thienyl | H |
| 5-68 | H | H | Me | 5-Br-2-thienyl | 5-Me |
| 5-69 | H | H | Me | 3-Thienyl | H |
| 5-70 | H | H | Me | 4-Cl-Ph | 5-S(O)Me |
| 5-71 | H | H | Me | 4-Br-Ph | 5-Me |
| 5-72 | H | H | Me | 1,3-Benzodioxol-5-yl | 5-Me |
| 5-73 | H | H | Me | 4-J-Ph | 5-Me |
| 5-74 | H | H | Me | 3,5-Cl₂-Ph | 5-Me |
| 5-75 | H | H | Me | 4-PhO-Ph | 5-Me |
| 5-76 | H | H | Me | 6-OH-pyridin-3-yl | H |
| 5-77 | H | H | Me | Ph | 5-S(O)Me |
| 5-78 | H | H | H | Ph | H |
| 5-79 | H | H | H | Ph | 5-Me |
| 5-80 | H | H | Et | Ph | H |
| 5-81 | H | H | n-Pr | Ph | H |
| 5-82 | H | H | CH₂Cl | Ph | H |
| 5-83 | H | H | CHCl₂ | Ph | H |
| 5-84 | H | H | CH₂F | Ph | H |
| 5-85 | H | H | CHF₂ | Ph | H |
| 5-86 | H | H | Cl | Ph | H |
| 5-87 | H | H | Et | Ph | 5-Me |
| 5-88 | H | H | n-Pr | Ph | 5-Me |
| 5-89 | H | H | CH₂Cl | Ph | 5-Me |
| 5-90 | H | H | CHCl₂ | Ph | 5-Me |
| 5-91 | H | H | CH₂F | Ph | 5-Me |
| 5-92 | H | H | CHF₂ | Ph | 5-Me |
| 5-93 | H | H | Cl | Ph | 5-Me |
| 5-94 | H | H | Et | 4-Cl-Ph | H |
| 5-95 | H | H | n-Pr | 4-Cl-Ph | H |
| 5-96 | H | H | CH₂Cl | 4-Cl-Ph | H |
| 5-97 | H | H | CHCl₂ | 4-Cl-Ph | H |
| 5-98 | H | H | CH₂F | 4-Cl-Ph | H |
| 5-99 | H | H | CHF₂ | 4-Cl-Ph | H |
| 5-100 | H | H | Cl | 4-Cl-Ph | H |
| 5-101 | H | H | Et | 4-Me-Ph | H |
| 5-102 | H | H | n-Pr | 4-Me-Ph | H |
| 5-103 | H | H | CH₂Cl | 4-Me-Ph | H |
| 5-104 | H | H | CHCl₂ | 4-Me-Ph | H |
| 5-105 | H | H | CH₂F | 4-Me-Ph | H |
| 5-106 | H | H | CHF₂ | 4-Me-Ph | H |
| 5-107 | H | H | Cl | 4-Me-Ph | H |
| 5-108 | H | H | Et | 2-Pyridyl | H |
| 5-109 | H | H | n-Pr | 2-Pyridyl | H |
| 5-110 | H | H | CH₂Cl | 2-Pyridyl | H |
| 5-111 | H | H | CHCl₂ | 2-Pyridyl | H |
| 5-112 | H | H | CH₂F | 2-Pyridyl | H |
| 5-113 | H | H | CHF₂ | 2-Pyridyl | H |
| 5-114 | H | H | Cl | 2-Pyridyl | H |
| 5-115 | H | H | Me | 2-Pyridyl | H |
| 5-116 | H | H | Me | 5-Cl-pyridin-2-yl | H |
| 5-117 | H | H | Me | 5-Cl-pyridin-2-yl | 5-Cl |
| 5-118 | H | H | Me | 5-Cl-pyridin-2-yl | 5-Me |
| 5-119 | H | H | Me | 5-Br-pyridin-2-yl | H |
| 5-120 | H | H | Me | 5-Br-pyridin-2-yl | 5-Cl |
| 5-121 | H | H | Me | 5-Br-pyridin-2-yl | 5-Me |
| 5-122 | H | H | Me | 5-F-pyridin-2-yl | H |
| 5-123 | H | H | Me | 5-Me-pyridin-2-yl | H |
| 5-124 | H | H | Me | 5-Me-pyridin-2-yl | 5-Me |
| 5-125 | H | H | Me | 2,4-Cl₂-Ph | H |
| 5-126 | H | H | Me | 4-(CH₂COOH)-Ph | 5-Me |
| 5-127 | H | H | Me | 3,4-Me₂-Ph | 5-Me |
| 5-128 | H | H | Me | 4-Br-Ph | 5-Me |
| 5-129 | H | H | Me | 3,4-Me₂-Ph | H |
| 5-130 | H | H | Me | 3-Me-Ph | H |
| 5-131 | H | H | Me | 4-F-Ph | H |
| 5-132 | H | H | Me | 4-(Me-CO)-Ph | H |
| 5-133 | H | H | Me | 4-tBu-Ph | H |
| 5-134 | H | H | Me | 4-Cl-3-Me-Ph | H |
| 5-135 | H | H | n-Pr | 4-Cl-Ph | 5-Me |
| 5-136 | H | H | Me | 3-Pyridyl | H |
| 5-137 | H | H | Me | 4-Pyridyl | H |
| 5-138 | H | H | C(O)OMe | Ph | H |
| 5-139 | H | H | Me | 6-Me-pyridin-3-yl | H |
| 5-140 | H | H | Me | 4-Cl-Ph | 5-SO₂Me |
| 5-141 | H | H | Me | 3-Pyridyl | 5-Me |
| 5-142 | H | H | Me | 2,3-Cl₂-Ph | 5-Me |
| 5-143 | H | H | Me | 2-Pyridyl | 5-Me |
| 5-144 | H | H | H | 4-Cl-Ph | 5-Me |
| 5-145 | H | H | Me | 6-Cl-pyridin-3-yl | H |
| 5-146 | H | H | Me | Ph | 5-Me |
| 5-147 | H | H | Me | 4-Me-pyridin-2-yl | H |
| 5-148 | H | H | Me | 4-Me-pyridin-2-yl | 5-Me |
| 5-149 | H | H | Me | 4-Me-pyridin-2-yl | 5-Cl |
| 5-150 | H | H | Me | 4-Me-pyridin-2-yl | 5-F |
| 5-151 | H | H | Me | 4-F-pyridin-2-yl | H |
| 5-152 | H | H | Me | 4-Cl-pyridin-2-yl | H |
| 5-153 | H | H | Me | 4-Br-pyridin-2-yl | H |
| 5-154 | H | H | Me | 4-OMe-pyridin-2-yl | H |
| 5-155 | H | H | Me | 5-CF₃-pyridin-2-yl | H |
| 5-156 | H | H | Me | 6-OMe-pyridin-2-yl | H |
| 5-157 | H | H | cyPr | 4-Cl-Ph | H |
| 5-158 | H | H | CN | 4-Cl-Ph | H |
| 5-159 | H | H | CN | 4-Cl-Ph | 5-Me |
| 5-160 | H | H | CN | 4-Me-Ph | H |
| 5-161 | H | H | CN | 4-Me-Ph | 5-Me |
| 5-162 | H | H | CN | Ph | H |
| 5-163 | H | H | CN | Ph | 5-Me |
| 5-164 | H | H | CN | 2-pyridyl | H |
| 5-165 | H | H | CN | 3-pyridyl | H |
| 5-166 | H | H | CN | 5-Cl-pyridin-2-yl | H |
| 5-167 | H | H | CN | 5-Br-pyridin-2-yl | H |
| 5-168 | H | H | CN | 5-F-pyridin-2-yl | H |
| 5-169 | H | H | CN | 5-Me-pyridin-2-yl | H |
| 5-170 | H | H | CN | 6-Me-pyridin-3-yl | H |
| 5-171 | H | H | CN | 4-Me-pyridin-2-yl | H |
| 5-172 | H | H | CN | 4-F-pyridin-2-yl | H |
| 5-173 | H | H | CN | 4-Cl-pyridin-2-yl | H |
| 5-174 | H | H | CN | 4-Br-pyridin-2-yl | H |
| 5-175 | H | H | CN | 4-OMe-pyridin-2-yl | H |
| 5-176 | H | H | formyl | 4-Cl-Ph | H |
| 5-177 | H | H | formyl | 4-Cl-Ph | 5-Me |
| 5-178 | H | H | formyl | 4-Me-Ph | H |
| 5-179 | H | H | formyl | 4-Me-Ph | 5-Me |
| 5-180 | H | H | formyl | Ph | H |
| 5-181 | H | H | formyl | Ph | 5-Me |
| 5-182 | H | H | formyl | 2-pyridyl | H |
| 5-183 | H | H | formyl | 3-pyridyl | H |
| 5-184 | H | H | formyl | 5-Cl-pyridin-2-yl | H |
| 5-185 | H | H | formyl | 5-Br-pyridin-2-yl | H |
| 5-186 | H | H | formyl | 5-F-pyridin-2-yl | H |
| 5-187 | H | H | formyl | 5-Me-pyridin-2-yl | H |
| 5-188 | H | H | formyl | 6-Me-pyridin-3-yl | H |
| 5-189 | H | H | formyl | 4-Me-pyridin-2-yl | H |
| 5-190 | H | H | formyl | 4-F-pyridin-2-yl | H |
| 5-191 | H | H | formyl | 4-Cl-pyridin-2-yl | H |
| 5-192 | H | H | formyl | 4-Br-pyridin-2-yl | H |
| 5-193 | H | H | formyl | 4-OMe-pyridin-2-yl | H |
| 5-194 | H | H | CH₂OH | 5-Me-pyridin-2-yl | H |
| 5-195 | H | H | CH₂OH | 4-Cl-Ph | H |
| 5-196 | H | H | CH₂OH | 4-Me-pyridin-2-yl | H |
| 5-197 | H | H | CH₂OH | 4-Me-Ph | H |
| 5-198 | H | H | CH₂OH | Ph | H |
| 5-199 | H | H | CH₂OH | 2-Pyridyl | H |
| 5-200 | H | H | Me | 2-Thiazolyl | H |
| 5-201 | H | H | Me | 2-Thiazolyl | 5-Cl |
| 5-202 | H | H | Me | 2-Thiazolyl | 5-Me |
| 5-203 | H | H | Me | 4-Me-thiazol-2-yl | H |
| 5-204 | H | H | Me | 4-Me-thiazol-2-yl | 5-Cl |
| 5-205 | H | H | Me | 4-Me-thiazol-2-yl | 5-Me |
| 5-206 | H | H | Me | 5-Me-thiazol-2-yl | H |
| 5-207 | H | H | Me | 5-Br-thiazol-2-yl | H |
| 5-208 | H | H | Me | 5-Br-thiazol-2-yl | 5-Me |
| 5-209 | H | H | Me | 5-Cl-thiazol-2-yl | H |
| 5-210 | H | H | Me | 4,6-Me₂-pyridin-2-yl | H |
| 5-211 | H | H | Me | 4,6-Me₂-pyridin-2-yl | 5-Me |
| 5-212 | H | H | Me | 2-Pyridyl | 5-F |
| 5-213 | H | H | Me | 2-Pyrazinyl | H |
| 5-214 | H | H | Me | 5-Me-Pyrazin-2-yl | H |
| 5-215 | H | H | Me | 2-Pyrazinyl | 5-Me |
| 5-216 | H | H | Me | 2-(1,3-Benzothiazolyl) | H |
| 5-217 | H | H | Me | 2-(1,3-Benzothiazolyl) | 5-Me |
| 5-218 | H | H | Me | 7-Cl-(1,3-benzothiazol-2-yl) | H |
| 5-219 | H | H | Me | 3-(1,5-Me₂-pyrazolyl) | H |
| 5-220 | H | H | Me | 3-(1,5-Me₂-pyrazolyl) | 5-Me |
| 5-221 | H | H | Me | 4,5-Me₂-thiazol-2-yl | H |
| 5-222 | H | H | Me | 4,5-Cl₂-thiazol-2-yl | H |
| 5-223 | H | H | Me | 2-Pyrimidinyl | H |
| 5-224 | H | H | Me | 2-Pyrimidinyl | 5-Me |
| 5-225 | H | H | Me | 5-F-pyrimidin-2-yl | H |
| 5-226 | H | H | Me | 5-Cl-pyrimidin-2-yl | H |
| 5-227 | H | H | Me | 5-Br-pyrimidin-2-yl | H |
| 5-228 | H | H | Me | 5-Me-pyrimidin-2-yl | H |
| 5-229 | H | H | Me | 5-Me-pyrimidin-2-yl | 5-Me |
| 5-230 | H | H | Me | 4,6-Me₂-pyrimidin-2-yl | H |
| 5-231 | H | H | Me | 4,6-Me₂-pyrimidin-2-yl | 5-Me |
| 5-232 | H | H | Me | 3-Pyridazinyl | H |
| 5-233 | H | H | Me | 6-Me-Pyridazin-3-yl | H |
| 5-234 | H | H | Me | 3-(1,2,4)-Triazinyl | H |
| 5-235 | H | H | Me | 6-Me-(1,2,4)-Triazin-3-yl | H |
| 5-236 | H | H | Me | 4-Cl-Ph | 3-COOMe |
| 5-237 | H | H | Me | 4-Cl-Ph | 3,5-(COOMe)₂ |
| 5-238 | H | H | Me | 2-Pyridyl | 3,5-(COOMe)₂ |
| 5-239 | H | H | Me | Chinolin-2-yl | H |
| 5-240 | H | H | Me | Isochinolin-3-yl | H |

**Tabelle 6: Verbindungen der Formel (Ib"")**

| | | | | |
|---|---|---|---|---|
| 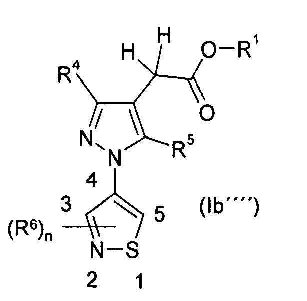 | | | | |

| Nr. | R¹ | R⁴ | R⁵ | (R⁶)ₙ |
|---|---|---|---|---|
| 6-1 | Et | Me | Ph | H |
| 6-2 | Et | Me | Ph | 5-Me |
| 6-3 | Et | Me | 3-Cl-Ph | H |
| 6-4 | Et | Me | 4-Cl-Ph | H |
| 6-5 | Et | Me | 4-Cl-Ph | 5-Me |
| 6-6 | Et | Me | 2-Thienyl | H |
| 6-7 | Et | Me | 3-Thienyl | H |
| 6-8 | Et | Me | 3-Me-2-thienyl | H |
| 6-9 | Et | Me | 4-Me-2-thienyl | H |
| 6-10 | Et | Me | 5-Br-2-thienyl | H |
| 6-11 | Et | Me | 5-Br-2-thienyl | 5-Me |
| 6-12 | Et | Me | 5-Cl-2-thienyl | H |
| 6-13 | Et | Me | 5-Cl-2-thienyl | 5-Me |
| 6-14 | Et | Me | 5-J-2-thienyl | H |
| 6-15 | Et | Me | 5-Me-2-thienyl | H |
| 6-16 | Et | Me | 3-Pyridyl | H |
| 6-17 | Et | Me | 6-MeO-pyridin-3-yl | H |
| 6-18 | Et | Me | 6-OH-pyridin-3-yl | H |
| 6-19 | Et | Me | 6-Me-pyridin-3-yl | H |
| 6-20 | Et | Me | 4-Me-Ph | H |
| 6-21 | Et | Me | 4-Me-Ph | 5-Me |
| 6-22 | Et | Me | 4-Br-Ph | H |
| 6-23 | Et | Me | 4-F-Ph | H |
| 6-24 | Et | Me | 4-F-Ph | 5-Me |
| 6-25 | Et | Me | 5-Cl-pyridin-2-yl | H |
| 6-26 | Et | Me | 5-Br-pyridin-2-yl | H |
| 6-27 | Et | Me | 5-F-pyridin-2-yl | H |
| 6-28 | Et | Me | 5-F-pyridin-2-yl | 5-Me |
| 6-29 | Et | Me | 5-Cl-pyridin-2-yl | 5-Me |
| 6-30 | Et | Me | 5-Br-pyridin-2-yl | 5-Me |
| 6-31 | Et | Me | 5-Me-pyridin-2-yl | H |
| 6-32 | Et | Me | 5-Me-pyridin-2-yl | 5-Me |
| 6-33 | Et | Me | 2-Pyridyl | 5-Me |
| 6-34 | Et | Me | 2-Pyridyl | H |
| 6-35 | Et | Me | 4-Pyridyl | H |
| 6-36 | Et | Me | 4-Me-pyridin-2-yl | H |
| 6-37 | Et | Me | 4-Me-pyridin-2-yl | 5-Me |
| 6-38 | Et | Me | 2-Thiazolyl | H |
| 6-39 | Et | Me | 4-Me-thiazol-2-yl | H |
| 6-40 | Et | Me | 5-Br-thiazol-2-yl | H |
| 6-41 | Et | Me | 5-Cl-thiazol-2-yl | H |
| 6-42 | Et | Me | 5-Me-thiazol-2-yl | H |
| 6-43 | Et | Me | 4,5-Me₂-thiazol-2-yl | H |
| 6-44 | Et | Me | 4,5-Cl₂-thiazol-2-yl | H |
| 6-45 | Et | Me | 4,6-Me₂-pyridin-2-yl | H |
| 6-46 | Et | Me | 2-Pyrazinyl | H |
| 6-47 | Et | Me | 2-Pyrimidinyl | H |
| 6-48 | Et | Me | 2-Pyrimidinyl | 5-Me |
| 6-49 | Et | Me | 5-Cl-pyrimidin-2-yl | H |
| 6-50 | Et | Me | 5-Br-pyrimidin-2-yl | H |
| 6-51 | Et | Me | 5-Me-pyrimidin-2-yl | H |
| 6-52 | Et | Me | 5-Me-pyrimidin-2-yl | 5-Me |
| 6-53 | Et | Me | 4,6-Me₂-pyrimidin-2-yl | H |
| 6-54 | Et | Me | 4,6-Me₂-pyrimidin-2-yl | 5-Me |
| 6-55 | Et | Me | 2-(1,3-Benzothiazolyl) | H |
| 6-56 | Et | Me | 7-Cl-(1,3-benzothiazol-2-yl) | H |
| 6-57 | Et | Me | 3-(1,5-Me₂-pyrazolyl) | H |
| 6-58 | Et | Me | 5-Me-Pyrazin-2-yl | H |
| 6-59 | Et | Me | 5-F-pyrimidin-2-yl | H |
| 6-60 | Et | Me | 4,6-Me₂-pyrimidin-2-yl | H |
| 6-61 | Et | Me | 3-Pyridazinyl | H |
| 6-62 | Et | Me | 6-Me-Pyridazin-3-yl | H |
| 6-63 | Et | Me | 3-(1,2,4)-Triazinyl | H |
| 6-64 | Et | Me | 6-Me-(1,2,4)-Triazin-3-yl | H |
| 6-65 | Et | Me | Chinolin-2-yl | H |
| 6-66 | Et | Me | Isochinolin-3-yl | H |
| 6-67 | Pr | Me | Ph | H |
| 6-68 | Pr | Me | 4-Cl-Ph | H |
| 6-69 | Pr | Me | 2-Thienyl | H |
| 6-70 | Pr | Me | 3-Pyridyl | H |
| 6-71 | Pr | Me | 6-Me-pyridin-3-yl | H |
| 6-72 | Pr | Me | 4-Me-Ph | H |
| 6-73 | Pr | Me | 4-Br-Ph | H |
| 6-74 | Pr | Me | 4-F-Ph | H |
| 6-75 | Pr | Me | 5-Cl-pyridin-2-yl | H |
| 6-76 | Pr | Me | 5-Br-pyridin-2-yl | H |
| 6-77 | Pr | Me | 5-F-pyridin-2-yl | H |
| 6-78 | Pr | Me | 5-Me-pyridin-2-yl | H |
| 6-79 | Pr | Me | 2-Pyridyl | H |
| 6-80 | Pr | Me | 4-Pyridyl | H |
| 6-81 | i-Pr | Me | Ph | H |
| 6-82 | i-Pr | Me | 4-Cl-Ph | H |
| 6-83 | i-Pr | Me | 2-Thienyl | H |
| 6-84 | i-Pr | Me | 3-Pyridyl | H |
| 6-85 | i-Pr | Me | 6-Me-pyridin-3-yl | H |
| 6-86 | i-Pr | Me | 4-Me-Ph | H |
| 6-87 | i-Pr | Me | 4-Br-Ph | H |
| 6-88 | i-Pr | Me | 4-F-Ph | H |
| 6-89 | i-Pr | Me | 5-Cl-pyridin-2-yl | H |
| 6-90 | i-Pr | Me | 5-Br-pyridin-2-yl | H |
| 6-91 | i-Pr | Me | 5-F-pyridin-2-yl | H |
| 6-92 | i-Pr | Me | 5-Me-pyridin-2-yl | H |
| 6-93 | i-Pr | Me | 2-Pyridyl | H |
| 6-94 | i-Pr | Me | 4-Pyridyl | H |
| 6-95 | CH₂Ph | Me | Ph | H |
| 6-96 | CH₂Ph | Me | 4-Cl-Ph | H |
| 6-97 | CH₂Ph | Me | 2-Thienyl | H |
| 6-98 | CH₂Ph | Me | 2-Pyridyl | H |
| 6-99 | Prop-2-in-1-yl | Me | Ph | H |
| 6-100 | Prop-2-in-1-yl | Me | 4-Cl-Ph | H |
| 6-101 | Prop-2-in-1-yl | Me | 2-Thienyl | H |
| 6-102 | Prop-2-in-1-yl | Me | 3-Thienyl | H |
| 6-103 | Prop-2-in-1-yl | Me | 3-Me-2-thienyl | H |
| 6-104 | Prop-2-in-1-yl | Me | 4-Me-2-thienyl | H |
| 6-105 | Prop-2-in-1-yl | Me | 5-Cl-2-thienyl | H |
| 6-106 | Prop-2-in-1-yl | Me | 5-Me-2-thienyl | H |
| 6-107 | Prop-2-in-1-yl | Me | 3-Pyridyl | H |
| 6-108 | Prop-2-in-1-yl | Me | 6-MeO-pyridin-3-yl | H |
| 6-109 | Prop-2-in-1-yl | H | Ph | H |
| 6-110 | Prop-2-in-1-yl | Me | 6-Me-pyridin-3-yl | H |
| 6-111 | Prop-2-in-1-yl | Me | 4-Me-Ph | H |
| 6-112 | Prop-2-in-1-yl | Me | 4-Br-Ph | H |
| 6-113 | Prop-2-in-1-yl | Me | 4-F-Ph | H |
| 6-114 | Prop-2-in-1-yl | Me | 5-Cl-pyridin-2-yl | H |
| 6-115 | Prop-2-in-1-yl | Me | 5-Br-pyridin-2-yl | H |
| 6-116 | Prop-2-in-1-yl | Me | 5-F-pyridin-2-yl | H |
| 6-117 | Prop-2-in-1-yl | Me | 5-Me-pyridin-2-yl | H |
| 6-118 | Prop-2-in-1-yl | Me | 2-Pyridyl | H |
| 6-119 | Prop-2-in-1-yl | Me | 4-Pyridyl | H |
| 6-120 | Prop-2-in-1-yl | Me | 4-Cl-Ph | 5-Me |
| 6-121 | Prop-2-in-1-yl | Me | Ph | 5-Me |
| 6-122 | Cyclopropylmethyl | Me | Ph | H |
| 6-123 | Cyclopropylmethyl | Me | 4-Cl-Ph | H |
| 6-124 | Cyclopropylmethyl | Me | 2-Thienyl | H |
| 6-125 | Cyclopropylmethyl | Me | 3-Thienyl | H |
| 6-126 | Cyclopropylmethyl | Me | 3-Me-2-thienyl | H |
| 6-127 | Cyclopropylmethyl | Me | 3-Pyridyl | H |
| 6-128 | Cyclopropylmethyl | Me | 5-Cl-2-thienyl | H |
| 6-129 | Cyclopropylmethyl | Me | 5-Me-2-thienyl | H |
| 6-130 | Cyclopropylmethyl | Me | 4-Me-2-thienyl | H |
| 6-131 | Cyclopropylmethyl | Me | 6-MeO-pyridin-3-yl | H |
| 6-132 | Cyclopropylmethyl | Me | 6-OH-pyridin-3-yl | H |
| 6-133 | Cyclopropylmethyl | Me | 6-Me-pyridin-3-yl | H |
| 6-134 | Cyclopropylmethyl | Me | 4-Me-Ph | H |
| 6-135 | Cyclopropylmethyl | Me | 4-Br-Ph | H |
| 6-136 | Cyclopropylmethyl | Me | 4-F-Ph | H |
| 6-137 | Cyclopropylmethyl | Me | 5-Cl-pyridin-2-yl | H |
| 6-138 | Cyclopropylmethyl | Me | 5-Br-pyridin-2-yl | H |
| 6-139 | Cyclopropylmethyl | Me | 5-F-pyridin-2-yl | H |
| 6-140 | Cyclopropylmethyl | Me | 5-Me-pyridin-2-yl | H |
| 6-141 | Cyclopropylmethyl | Me | 2-Pyridyl | H |
| 6-142 | Cyclopropylmethyl | Me | 4-Pyridyl | H |
| 6-143 | Cyclopropylmethyl | Me | 4-Cl-Ph | 5-Me |
| 6-144 | Cyclopropylmethyl | Me | Ph | 5-Me |
| 6-145 | Cyclopropylmethyl | H | Ph | H |
| 6-146 | Cyclopropylmethyl | H | Chinolin-2-yl | H |
| 6-147 | Cyclopropylmethyl | H | Isochinolin-3-yl | H |
| 6-148 | 3,3-Dichlor-2-fluorprop-2-en-1-yl | Me | Ph | H |
| 6-149 | 3,3-Dichlor-2-fluorprop-2-en-1-yl | Me | 4-Cl-Ph | H |
| 6-150 | 3,3-Dichlor-2-fluorprop-2-en-1-yl | Me | 2-Thienyl | H |
| 6-151 | 3,3-Dichlor-2-fluorprop-2-en-1-yl | Me | 2-Pyridyl | H |
| 6-152 | (1-Methylcyclopropyl)-methyl | Me | Ph | H |
| 6-153 | (1-Methylcyclopropyl)-methyl | Me | 4-Cl-Ph | H |
| 6-154 | (1-Methylcyclopropyl)-methyl | Me | 2-Thienyl | H |
| 6-155 | (1-Methylcyclopropyl)-methyl | Me | 2-Pyridyl | H |
| 6-156 | 4-Chlorbut-2-in-1-yl | Me | Ph | H |
| 6-157 | 4-Chlorbut-2-in-1-yl | Me | 4-Cl-Ph | H |
| 6-158 | 4-Chlorbut-2-in-1-yl | Me | 2-Thienyl | H |
| 6-159 | 4-Chlorbut-2-in-1-yl | Me | 2-Pyridyl | H |
| 6-160 | (2,2-Dichlorcyclopropyl)- methyl | Me | Ph | H |
| 6-161 | (2,2-Dichlorcyclopropyl)- methyl | Me | 4-CI-Ph | H |
| 6-162 | (2,2-Dichlorcyclopropyl)- methyl | Me | 2-Thienyl | H |
| 6-163 | (2,2-Dichlorcyclopropyl)- methyl | Me | 2-Pyridyl | H |
| 6-164 | But-2-in-1-yl | Me | Ph | H |
| 6-165 | But-2-in-1-yl | Me | 4-Cl-Ph | H |
| 6-166 | But-2-in-1-yl | Me | 2-Thienyl | H |
| 6-167 | But-2-in-1-yl | Me | 3-Thienyl | H |
| 6-168 | But-2-in-1-yl | Me | 3-Me-2-thienyl | H |
| 6-169 | But-2-in-1-yl | Me | 4-Me-2-thienyl | H |
| 6-170 | But-2-in-1-yl | Me | 5-Cl-2-thienyl | H |
| 6-171 | But-2-in-1-yl | Me | 5-Me-2-thienyl | H |
| 6-172 | But-2-in-1-yl | Me | 3-Pyridyl | H |
| 6-173 | But-2-in-1-yl | Me | 6-MeO-pyridin-3-yl | H |
| 6-174 | But-2-in-1-yl | H | Ph | H |
| 6-175 | But-2-in-1-yl | Me | 6-Me-pyridin-3-yl | H |
| 6-176 | But-2-in-1-yl | Me | 4-Me-Ph | H |
| 6-177 | But-2-in-1-yl | Me | 4-Br-Ph | H |
| 6-178 | But-2-in-1-yl | Me | 4-F-Ph | H |
| 6-179 | But-2-in-1-yl | Me | 5-Cl-pyridin-2-yl | H |
| 6-180 | But-2-in-1-yl | Me | 5-Br-pyridin-2-yl | H |
| 6-181 | But-2-in-1-yl | Me | 5-F-pyridin-2-yl | H |
| 6-182 | But-2-in-1-yl | Me | 5-Me-pyridin-2-yl | H |
| 6-183 | But-2-in-1-yl | Me | 2-Pyridyl | H |
| 6-184 | But-2-in-1-yl | Me | 4-Pyridyl | H |
| 6-185 | But-2-in-1-yl | Me | 4-Cl-Ph | 5-Me |
| 6-186 | But-2-in-1-yl | Me | Ph | 5-Me |
| 6-187 | 1-Methylprop-2-in-1-yl | Me | Ph | H |
| 6-188 | 1-Methylprop-2-in-1-yl | Me | 4-Cl-Ph | H |
| 6-189 | 1-Methylprop-2-in-1-yl | Me | 2-Thienyl | H |
| 6-190 | 1-Methylprop-2-in-1-yl | Me | 2-Pyridyl | H |
| 6-191 | 1-Cyclopropylethyl | Me | Ph | H |
| 6-192 | 1-Cyclopropylethyl | Me | 4-Cl-Ph | H |
| 6-193 | 1-Cyclopropylethyl | Me | 2-Thienyl | H |
| 6-194 | 1-Cyclopropylethyl | Me | 2-Pyridyl | H |
| 6-195 | Allyl | Me | Ph | H |
| 6-196 | Allyl | Me | 4-Cl-Ph | H |
| 6-197 | Allyl | Me | 2-Thienyl | H |
| 6-198 | Allyl | Me | 2-Pyridyl | H |
| 6-199 | 3-Methylbut-2-en-1-yl | Me | Ph | H |
| 6-200 | 3-Methylbut-2-en-1-yl | Me | 4-Cl-Ph | H |
| 6-201 | 3-Methylbut-2-en-1-yl | Me | 2-Thienyl | H |
| 6-202 | 3-Methylbut-2-en-1-yl | Me | 2-Pyridyl | H |
| 6-203 | 2-Methylprop-2-en-1-yl | Me | Ph | H |
| 6-204 | 2-Methylprop-2-en-1-yl | Me | 4-Cl-Ph | H |
| 6-205 | 2-Methylprop-2-en-1-yl | Me | 2-Thienyl | H |
| 6-206 | 2-Methylprop-2-en-1-yl | Me | 2-Pyridyl | H |
| 6-207 | (2E)-1-Methylbut-2-en-1-yl | Me | Ph | H |
| 6-208 | (2E)-1-Methylbut-2-en-1-yl | Me | 4-Cl-Ph | H |
| 6-209 | (2E)-1-Methylbut-2-en-1-yl | Me | 2-Thienyl | H |
| 6-210 | (2E)-1-Methylbut-2-en-1-yl | Me | 2-Pyridyl | H |
| 6-211 | 3-Phenylprop-2-in-1-yl | Me | Ph | H |
| 6-212 | 3-Phenylprop-2-in-1-yl | Me | 4-Cl-Ph | H |
| 6-213 | 3-Phenylprop-2-in-1-yl | Me | 2-Thienyl | H |
| 6-214 | 3-Phenylprop-2-in-1-yl | Me | 2-Pyridyl | H |
| 6-215 | Cyclobutylmethyl | Me | Ph | H |
| 6-216 | Cyclobutylmethyl | Me | 4-Cl-Ph | H |
| 6-217 | Cyclobutylmethyl | Me | 2-Thienyl | H |
| 6-218 | Cyclobutylmethyl | Me | 2-Pyridyl | H |
| 6-219 | Cyclopentylmethyl | Me | Ph | H |
| 6-220 | Cyclopentylmethyl | Me | 4-Cl-Ph | H |
| 6-221 | Cyclopentylmethyl | Me | 2-Thienyl | H |
| 6-222 | Cyclopentylmethyl | Me | 2-Pyridyl | H |
| 6-223 | Cyclohexylmethyl | Me | Ph | H |
| 6-224 | Cyclohexylmethyl | Me | 4-Cl-Ph | H |
| 6-225 | Cyclohexylmethyl | Me | 2-Thienyl | H |
| 6-226 | Cyclohexylmethyl | Me | 2-Pyridyl | H |
| 6-227 | But-3-en-1-yl | Me | Ph | H |
| 6-228 | But-3-en-1-yl | Me | 4-Cl-Ph | H |
| 6-229 | But-3-en-1-yl | Me | 2-Thienyl | H |
| 6-230 | But-3-en-1-yl | Me | 2-Pyridyl | H |
| 6-231 | 2-Chloroprop-2-en-1-yl | Me | Ph | H |
| 6-232 | 2-Chloroprop-2-en-1-yl | Me | 4-Cl-Ph | H |
| 6-233 | 2-Chloroprop-2-en-1-yl | Me | 2-Thienyl | H |
| 6-234 | 2-Chloroprop-2-en-1-yl | Me | 3-Thienyl | H |
| 6-235 | 2-Chloroprop-2-en-1-yl | Me | 3-Me-2-thienyl | H |
| 6-236 | 2-Chloroprop-2-en-1-yl | Me | 4-Me-2-thienyl | H |
| 6-237 | 2-Chloroprop-2-en-1-yl | Me | 5-Cl-2-thienyl | H |
| 6-238 | 2-Chloroprop-2-en-1-yl | Me | 5-Me-2-thienyl | H |
| 6-239 | 2-Chloroprop-2-en-1-yl | Me | 3-Pyridyl | H |
| 6-240 | 2-Chloroprop-2-en-1-yl | Me | 6-MeO-pyridin-3-yl | H |
| 6-241 | 2-Chloroprop-2-en-1-yl | Me | 6-OH-pyridin-3-yl | H |
| 6-242 | 2-Chloroprop-2-en-1-yl | Me | 6-Me-pyridin-3-yl | H |
| 6-243 | 2-Chloroprop-2-en-1-yl | Me | 4-Me-Ph | H |
| 6-244 | 2-Chloroprop-2-en-1-yl | Me | 4-Br-Ph | H |
| 6-245 | 2-Chloroprop-2-en-1-yl | Me | 4-F-Ph | H |
| 6-246 | 2-Chloroprop-2-en-1-yl | Me | 5-Cl-pyridin-2-yl | H |
| 6-247 | 2-Chloroprop-2-en-1-yl | Me | 5-Br-pyridin-2-yl | H |
| 6-248 | 2-Chloroprop-2-en-1-yl | Me | 5-F-pyridin-2-yl | H |
| 6-249 | 2-Chloroprop-2-en-1-yl | Me | 5-Me-pyridin-2-yl | H |
| 6-250 | 2-Chloroprop-2-en-1-yl | Me | 2-Pyridyl | H |
| 6-251 | 2-Chloroprop-2-en-1-yl | Me | 4-Pyridyl | H |
| 6-252 | 2-Chloroprop-2-en-1-yl | Me | 4-Cl-Ph | 5-Me |
| 6-253 | 2-Chloroprop-2-en-1-yl | Me | Ph | 5-Me |
| 6-254 | 2-Chloroprop-2-en-1-yl | H | Ph | H |
| 6-255 | 2-Chloroprop-2-en-1-yl | H | Chinolin-2-yl | H |
| 6-256 | 2-Chloroprop-2-en-1-yl | H | Isochinolin-3-yl | H |
| 6-257 | 2-Methoxyethyl | Me | Ph | H |
| 6-258 | 2-Methoxyethyl | Me | 4-Cl-Ph | H |
| 6-259 | 2-Methoxyethyl | Me | 2-Thienyl | H |
| 6-260 | 2-Methoxyethyl | Me | 2-Pyridyl | H |
| 6-261 | Tetrahydrofuran-2-yl-methyl | Me | Ph | H |
| 6-262 | Tetrahydrofuran-2-yl-methyl | Me | 4-Cl-Ph | H |
| 6-263 | Tetrahydrofuran-2-yl-methyl | Me | 2-Thienyl | H |
| 6-264 | Tetrahydrofuran-2-yl-methyl | Me | 2-Pyridyl | H |
| 6-265 | 2-(Dimethylamino)ethyl | Me | Ph | H |
| 6-266 | 2-(Dimethylamino)ethyl | Me | 4-Cl-Ph | H |
| 6-267 | 2-(Dimethylamino)ethyl | Me | 2-Thienyl | H |
| 6-268 | 2-(Dimethylamino)ethyl | Me | 2-Pyridyl | H |
| 6-269 | Oxetan-3-yl | Me | Ph | H |
| 6-270 | Oxetan-3-yl | Me | 4-Cl-Ph | H |
| 6-271 | Oxetan-3-yl | Me | 2-Thienyl | H |
| 6-272 | Oxetan-3-yl | Me | 2-Pyridyl | H |
| 6-273 | (3-Methyloxetan-3-yl)methyl | Me | Ph | H |
| 6-274 | (3-Methyloxetan-3-yl)methyl | Me | 4-Cl-Ph | H |
| 6-275 | (3-Methyloxetan-3-yl)methyl | Me | 2-Thienyl | H |
| 6-276 | (3-Methyloxetan-3-yl)methyl | Me | 2-Pyridyl | H |
| 6-277 | 2,2,2-Trifluorethyl | Me | Ph | H |
| 6-278 | 2,2,2-Trifluorethyl | Me | 4-Cl-Ph | H |
| 6-279 | 2,2,2-Trifluorethyl | Me | 2-Thienyl | H |
| 6-280 | 2,2,2-Trifluorethyl | Me | 3-Pyridyl | H |
| 6-281 | 2,2,2-Trifluorethyl | Me | 6-Me-pyridin-3-yl | H |
| 6-282 | 2,2,2-Trifluorethyl | Me | 4-Me-Ph | H |
| 6-283 | 2,2,2-Trifluorethyl | Me | 4-Br-Ph | H |
| 6-284 | 2,2,2-Trifluorethyl | Me | 4-F-Ph | H |
| 6-285 | 2,2,2-Trifluorethyl | Me | 5-Cl-pyridin-2-yl | H |
| 6-286 | 2,2,2-Trifluorethyl | Me | 5-Br-pyridin-2-yl | H |
| 6-287 | 2,2,2-Trifluorethyl | Me | 5-F-pyridin-2-yl | H |
| 6-288 | 2,2,2-Trifluorethyl | Me | 5-Me-pyridin-2-yl | H |
| 6-289 | 2,2,2-Trifluorethyl | Me | 2-Pyridyl | H |
| 6-290 | 2,2,2-Trifluorethyl | Me | 4-Pyridyl | H |
| 6-291 | CH₂(4-Cl-Ph) | Me | Ph | H |
| 6-292 | CH₂(4-Cl-Ph) | Me | 4-Cl-Ph | H |
| 6-293 | CH₂(4-Cl-Ph) | Me | 2-Thienyl | H |
| 6-294 | CH₂(4-Cl-Ph) | Me | 3-Pyridyl | H |
| 6-295 | CH₂(4-Cl-Ph) | Me | 6-Me-pyridin-3-yl | H |
| 6-296 | CH₂(4-Cl-Ph) | Me | 4-Me-Ph | H |
| 6-297 | CH₂(4-Cl-Ph) | Me | 4-Br-Ph | H |
| 6-298 | CH₂(4-Cl-Ph) | Me | 4-F-Ph | H |
| 6-299 | CH₂(4-Cl-Ph) | Me | 5-Cl-pyridin-2-yl | H |
| 6-300 | CH₂(4-Cl-Ph) | Me | 5-Br-pyridin-2-yl | H |
| 6-301 | CH₂(4-Cl-Ph) | Me | 5-F-pyridin-2-yl | H |
| 6-302 | CH₂(4-Cl-Ph) | Me | 5-Me-pyridin-2-yl | H |
| 6-303 | CH₂(4-Cl-Ph) | Me | 2-Pyridyl | H |
| 6-304 | CH₂(4-Cl-Ph) | Me | 4-Pyridyl | H |
| 6-305 | CH₂(4-F-Ph) | Me | Ph | H |
| 6-306 | CH₂(4-F-Ph) | Me | 4-Cl-Ph | H |
| 6-307 | CH₂(4-F-Ph) | Me | 2-Thienyl | H |
| 6-308 | CH₂(4-F-Ph) | Me | 3-Pyridyl | H |
| 6-309 | CH₂(4-F-Ph) | Me | 6-Me-pyridin-3-yl | H |
| 6-310 | CH₂(4-F-Ph) | Me | 4-Me-Ph | H |
| 6-311 | CH₂(4-F-Ph) | Me | 4-Br-Ph | H |
| 6-312 | CH₂(4-F-Ph) | Me | 4-F-Ph | H |
| 6-313 | CH₂(4-F-Ph) | Me | 5-Cl-pyridin-2-yl | H |
| 6-314 | CH₂(4-F-Ph) | Me | 5-Br-pyridin-2-yl | H |
| 6-315 | CH₂(4-F-Ph) | Me | 5-F-pyridin-2-yl | H |
| 6-316 | CH₂(4-F-Ph) | Me | 5-Me-pyridin-2-yl | H |
| 6-317 | CH₂(4-F-Ph) | Me | 2-Pyridyl | H |
| 6-318 | CH₂(4-F-Ph) | Me | 4-Pyridyl | H |
| 6-319 | CH₂(4-OMe-Ph) | Me | Ph | H |
| 6-320 | CH₂(4-OMe-Ph) | Me | 4-Cl-Ph | H |
| 6-321 | CH₂(4-OMe-Ph) | Me | 2-Thienyl | H |
| 6-322 | CH₂(4-OMe-Ph) | Me | 3-Pyridyl | H |
| 6-323 | CH₂(4-OMe-Ph) | Me | 6-Me-pyridin-3-yl | H |
| 6-324 | CH₂(4-OMe-Ph) | Me | 4-Me-Ph | H |
| 6-325 | CH₂(4-OMe-Ph) | Me | 4-Br-Ph | H |
| 6-326 | CH₂(4-OMe-Ph) | Me | 4-F-Ph | H |
| 6-327 | CH₂(4-OMe-Ph) | Me | 5-Cl-pyridin-2-yl | H |
| 6-328 | CH₂(4-OMe-Ph) | Me | 5-Br-pyridin-2-yl | H |
| 6-329 | CH₂(4-OMe-Ph) | Me | 5-F-pyridin-2-yl | H |
| 6-330 | CH₂(4-OMe-Ph) | Me | 5-Me-pyridin-2-yl | H |
| 6-331 | CH₂(4-OMe-Ph) | Me | 2-Pyridyl | H |
| 6-332 | CH₂(4-OMe-Ph) | Me | 4-Pyridyl | H |
| 6-333 | 2,2-Difluorethyl | Me | Ph | H |
| 6-334 | 2,2-Difluorethyl | Me | 4-Cl-Ph | H |
| 6-335 | 2,2-Difluorethyl | Me | 2-Thienyl | H |
| 6-336 | 2,2-Difluorethyl | Me | 2-Pyridyl | H |
| 6-337 | Ph | Me | Ph | H |
| 6-338 | Ph | Me | 4-Cl-Ph | H |
| 6-339 | Ph | Me | 2-Thienyl | H |
| 6-340 | Ph | Me | 2-Pyridyl | H |
| 6-341 | 2-Fluorethyl | Me | Ph | H |
| 6-342 | 2-Fluorethyl | Me | 4-Cl-Ph | H |
| 6-343 | 2-Fluorethyl | Me | 2-Thienyl | H |
| 6-344 | 2-Fluorethyl | Me | 2-Pyridyl | H |
| 6-345 | 2,2,3,3,3-Pentafluorpropyl | Me | Ph | H |
| 6-346 | 2,2,3,3,3-Pentafluorpropyl | Me | 4-Cl-Ph | H |
| 6-347 | 2,2,3,3,3-Pentafluorpropyl | Me | 2-Thienyl | H |
| 6-348 | 2,2,3,3,3-Pentafluorpropyl | Me | 2-Pyridyl | H |
| 6-349 | 1-Ethyl-5-methyl-1H-pyrazol-4-methyl | Me | Ph | H |
| 6-350 | 1-Ethyl-5-methyl-1H-pyrazol-4-methyl | Me | 4-Cl-Ph | H |
| 6-351 | 1-Ethyl-5-methyl-1H-pyrazol-4-methyl | Me | 2-Thienyl | H |
| 6-352 | 1-Ethyl-5-methyl-1H-pyrazol-4-methyl | Me | 2-Pyridyl | H |

**Tabelle 7: Verbindungen der Formel (Ic")**

| | | | | | |
|---|---|---|---|---|---|
| 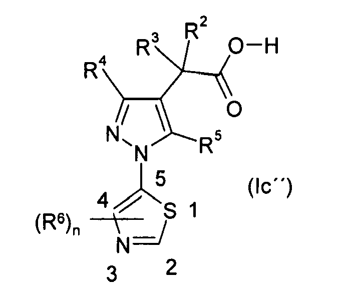 | | | | | |

| Nr. | R² | R³ | R⁴ | R⁵ | (R⁶)ₙ |
|---|---|---|---|---|---|
| 7-1 | H | H | Ph | Ph | H |
| 7-2 | H | H | Me | Ph | H |
| 7-3 | H | H | Me | 5-J-2-thienyl | H |
| 7-4 | H | H | Me | 2-Furyl | H |
| 7-5 | H | H | Me | Ph | 2-OMe |
| 7-6 | Me | H | Me | Ph | 4-Me |
| 7-7 | H | H | Me | Ph | 2-Cl |
| 7-8 | H | H | Me | Ph | 4-CF₃ |
| 7-9 | H | H | Me | Ph | 2-CF₃ |
| 7-10 | H | H | Me | Ph | 4-Me |
| 7-11 | H | H | Me | Ph | 2,4-Me₂ |
| 7-12 | H | H | Me | Ph | 2,4-Cl₂ |
| 7-13 | H | H | Me | 4-MeO-Ph | 4-Me |
| 7-14 | H | H | Me | 4-MeO-Ph | H |
| 7-15 | Me | H | Me | Ph | H |
| 7-16 | H | H | Me | 4-Me-Ph | 4-Me |
| 7-17 | H | H | Me | 4-Me-Ph | 4-Cl |
| 7-18 | H | H | Me | 4-Me-Ph | H |
| 7-19 | H | H | Me | 3-Cl-Ph | H |
| 7-20 | H | H | Me | 3-CF₃-Ph | H |
| 7-21 | H | H | Me | 3-CF₃-Ph | 4-Me |
| 7-22 | H | H | Me | 3,4-Cl₂-Ph | 4-Me |
| 7-23 | H | H | Me | 3-Cl-Ph | 4-Me |
| 7-24 | H | H | Me | 2-Cl-Ph | 4-Me |
| 7-25 | H | H | Me | 2,4-Cl₂-Ph | 4-Me |
| 7-26 | H | H | Me | 4-CF₃-Ph | 4-Me |
| 7-27 | H | H | Me | 4-Cl-Ph | 4-Me |
| 7-28 | H | H | Me | 4-Cl-Ph | 4-COOEt |
| 7-29 | H | H | Me | 4-Cl-Ph | H |
| 7-30 | H | H | Me | 3,4-Cl₂-Ph | H |
| 7-31 | H | H | Me | 4-CF₃-Ph | H |
| 7-32 | H | H | Me | 4-Cl-Ph | 4-Cl |
| 7-33 | H | H | Me | Ph | 4-Cl |
| 7-34 | H | H | Me | 2-Cl-Ph | H |
| 7-35 | H | H | Me | 4-tBu-Ph | 4-Me |
| 7-36 | H | H | Me | 3,5-Me₂-Ph | 4-Me |
| 7-37 | H | H | Me | Ph | 4-OMe |
| 7-38 | H | H | Me | 4-Cl-Ph | 4-OMe |
| 7-39 | H | H | Me | 4-Me-Ph | 4-Me |
| 7-40 | H | H | Me | 4-F-Ph | 4-Cl |
| 7-41 | H | H | Me | 4-F-Ph | 4-Me |
| 7-42 | H | H | Me | 3-Me-Ph | 4-Me |
| 7-43 | H | H | Me | 4-(COOH)-Ph | 4-Me |
| 7-44 | H | H | Me | 3-Br-Ph | 4-Me |
| 7-45 | H | H | Me | 4-Ph-Ph | 4-Me |
| 7-46 | H | H | Me | 4-(COOH)-Ph | H |
| 7-47 | H | H | Me | 3,5-Me₂-Ph | H |
| 7-48 | H | H | Me | Ph | 4-SMe |
| 7-49 | H | H | Me | 4-Cl-Ph | 4-SMe |
| 7-50 | H | H | Me | 3-Cl-4-Me-Ph | H |
| 7-51 | H | H | Me | 3-CF₃-4-Cl-Ph | H |
| 7-52 | H | H | Me | 3-CF₃-4-Cl-Ph | 4-Me |
| 7-53 | H | H | Me | 3-Cl-4-Me-Ph | 4-Me |
| 7-54 | H | H | Me | 2-Pyridyl | 4-Cl |
| 7-55 | H | H | Me | 4-Cl-Ph | 4-F |
| 7-56 | H | H | Me | 2-Thienyl | 4-Me |
| 7-57 | H | H | Me | 3-Me-2-thienyl | 4-Me |
| 7-58 | H | H | Me | 4-Me-2-thienyl | 4-Me |
| 7-59 | H | H | Me | 5-Cl-2-thienyl | 4-Me |
| 7-60 | H | H | Me | 5-Cl-2-thienyl | 4-Cl |
| 7-61 | H | H | Me | 3-Thienyl | 4-Me |
| 7-62 | H | H | Me | 2-Thienyl | H |
| 7-63 | H | H | Me | 3-Me-2-thienyl | H |
| 7-64 | H | H | Me | 4-Me-2-thienyl | H |
| 7-65 | H | H | Me | 5-Cl-2-thienyl | H |
| 7-66 | H | H | Me | 5-Me-2-thienyl | H |
| 7-67 | H | H | Me | 6-MeO-pyridin-3-yl | H |
| 7-68 | H | H | Me | 5-Br-2-thienyl | H |
| 7-69 | H | H | Me | 5-Br-2-thienyl | 4-Me |
| 7-70 | H | H | Me | 3-Thienyl | H |
| 7-71 | H | H | Me | 4-Cl-Ph | 4-S(O)Me |
| 7-72 | H | H | Me | 4-Br-Ph | 4-Me |
| 7-73 | H | H | Me | 1,3-Benzodioxol-5-yl | 4-Me |
| 7-74 | H | H | Me | 4-J-Ph | 4-Me |
| 7-75 | H | H | Me | 3,5-Cl₂-Ph | 4-Me |
| 7-76 | H | H | Me | 4-PhO-Ph | 4-Me |
| 7-77 | H | H | Me | 6-OH-pyridin-3-yl | H |
| 7-78 | H | H | Me | Ph | 4-S(O)Me |
| 7-79 | H | H | H | Ph | H |
| 7-80 | H | H | H | Ph | 4-Me |
| 7-81 | H | H | Et | Ph | H |
| 7-82 | H | H | n-Pr | Ph | H |
| 7-83 | H | H | CH₂Cl | Ph | H |
| 7-84 | H | H | CHCl₂ | Ph | H |
| 7-85 | H | H | CH₂F | Ph | H |
| 7-86 | H | H | CHF₂ | Ph | H |
| 7-87 | H | H | Cl | Ph | H |
| 7-88 | H | H | Et | Ph | 4-Me |
| 7-89 | H | H | n-Pr | Ph | 4-Me |
| 7-90 | H | H | CH₂Cl | Ph | 4-Me |
| 7-91 | H | H | CHCl₂ | Ph | 4-Me |
| 7-92 | H | H | CH₂F | Ph | 4-Me |
| 7-93 | H | H | CHF₂ | Ph | 4-Me |
| 7-94 | H | H | Cl | Ph | 4-Me |
| 7-95 | H | H | Et | 4-Cl-Ph | H |
| 7-96 | H | H | n-Pr | 4-Cl-Ph | H |
| 7-97 | H | H | CH₂Cl | 4-Cl-Ph | H |
| 7-98 | H | H | CHCl₂ | 4-Cl-Ph | H |
| 7-99 | H | H | CH₂F | 4-Cl-Ph | H |
| 7-100 | H | H | CHF₂ | 4-Cl-Ph | H |
| 7-101 | H | H | Cl | 4-Cl-Ph | H |
| 7-102 | H | H | Et | 4-Me-Ph | H |
| 7-103 | H | H | n-Pr | 4-Me-Ph | H |
| 7-104 | H | H | CH₂Cl | 4-Me-Ph | H |
| 7-105 | H | H | CHCl₂ | 4-Me-Ph | H |
| 7-106 | H | H | CH₂F | 4-Me-Ph | H |
| 7-107 | H | H | CHF₂ | 4-Me-Ph | H |
| 7-108 | H | H | Cl | 4-Me-Ph | H |
| 7-109 | H | H | Et | 2-Pyridyl | H |
| 7-110 | H | H | n-Pr | 2-Pyridyl | H |
| 7-111 | H | H | CH₂Cl | 2-Pyridyl | H |
| 7-112 | H | H | CHCl₂ | 2-Pyridyl | H |
| 7-113 | H | H | CH₂F | 2-Pyridyl | H |
| 7-114 | H | H | CHF₂ | 2-Pyridyl | H |
| 7-115 | H | H | Cl | 2-Pyridyl | H |
| 7-116 | H | H | Me | 2-Pyridyl | H |
| 7-117 | H | H | Me | 5-Cl-pyridin-2-yl | H |
| 7-118 | H | H | Me | 5-Cl-pyridin-2-yl | 4-Cl |
| 7-119 | H | H | Me | 5-Cl-pyridin-2-yl | 4-Me |
| 7-120 | H | H | Me | 5-Br-pyridin-2-yl | H |
| 7-121 | H | H | Me | 5-Br-pyridin-2-yl | 4-Cl |
| 7-122 | H | H | Me | 5-Br-pyridin-2-yl | 4-Me |
| 7-123 | H | H | Me | 5-F-pyridin-2-yl | H |
| 7-124 | H | H | Me | 5-Me-pyridin-2-yl | H |
| 7-125 | H | H | Me | 5-Me-pyridin-2-yl | 4-Me |
| 7-126 | H | H | Me | 2,4-Cl₂-Ph | H |
| 7-127 | H | H | Me | 4-CH₂COOH-Ph | 4-Me |
| 7-128 | H | H | Me | 3,4-Me₂-Ph | 4-Me |
| 7-129 | H | H | Me | 4-Br-Ph | H |
| 7-130 | H | H | Me | 3,4-Me₂-Ph | H |
| 7-131 | H | H | Me | 3-Me-Ph | H |
| 7-132 | H | H | Me | 4-F-Ph | H |
| 7-133 | H | H | Me | 4-(Me-CO)-Ph | H |
| 7-134 | H | H | Me | 4-tBu-Ph | H |
| 7-135 | H | H | Me | 4-Cl-3-Me-Ph | H |
| 7-136 | H | H | n-Pr | 4-Cl-Ph | 4-Me |
| 7-137 | H | H | Me | 3-Pyridyl | H |
| 7-138 | H | H | Me | 4-Pyridyl | H |
| 7-139 | H | H | C(O)OMe | Ph | H |
| 7-140 | H | H | Me | 6-Me-pyridin-3-yl | H |
| 7-141 | H | H | Me | 4-Cl-Ph | 4-SO₂Me |
| 7-142 | H | H | Me | 3-Pyridyl | 4-Me |
| 7-143 | H | H | Me | 2,3-Cl₂-Ph | 4-Me |
| 7-144 | H | H | Me | 2-Pyridyl | 4-Me |
| 7-145 | H | H | H | 4-Cl-Ph | 4-Me |
| 7-146 | H | H | Me | 6-Cl-pyridin-3-yl | H |
| 7-147 | H | H | Me | Ph | 2-Me |
| 7-148 | H | H | Me | 4-Me-pyridin-2-yl | H |
| 7-149 | H | H | Me | 4-Me-pyridin-2-yl | 4-Me |
| 7-150 | H | H | Me | 4-Me-pyridin-2-yl | 4-Cl |
| 7-151 | H | H | Me | 4-Me-pyridin-2-yl | 4-F |
| 7-152 | H | H | Me | 4-F-pyridin-2-yl | H |
| 7-153 | H | H | Me | 4-Cl-pyridin-2-yl | H |
| 7-154 | H | H | Me | 4-Br-pyridin-2-yl | H |
| 7-155 | H | H | Me | 4-OMe-pyridin-2-yl | H |
| 7-156 | H | H | Me | 5-CF₃-pyridin-2-yl | H |
| 7-157 | H | H | Me | 6-OMe-pyridin-2-yl | H |
| 7-158 | H | H | cyPr | 4-Cl-Ph | H |
| 7-159 | H | H | CN | 4-Cl-Ph | H |
| 7-160 | H | H | CN | 4-Cl-Ph | 4-Me |
| 7-161 | H | H | CN | 4-Me-Ph | H |
| 7-162 | H | H | CN | 4-Me-Ph | 4-Me |
| 7-163 | H | H | CN | Ph | H |
| 7-164 | H | H | CN | Ph | 4-Me |
| 7-165 | H | H | CN | 2-pyridyl | H |
| 7-166 | H | H | CN | 3-pyridyl | H |
| 7-167 | H | H | CN | 5-Cl-pyridin-2-yl | H |
| 7-168 | H | H | CN | 5-Br-pyridin-2-yl | H |
| 7-169 | H | H | CN | 5-F-pyridin-2-yl | H |
| 7-170 | H | H | CN | 5-Me-pyridin-2-yl | H |
| 7-171 | H | H | CN | 6-Me-pyridin-3-yl | H |
| 7-172 | H | H | CN | 4-Me-pyridin-2-yl | H |
| 7-173 | H | H | CN | 4-F-pyridin-2-yl | H |
| 7-174 | H | H | CN | 4-Cl-pyridin-2-yl | H |
| 7-175 | H | H | CN | 4-Br-pyridin-2-yl | H |
| 7-176 | H | H | CN | 4-OMe-pyridin-2-yl | H |
| 7-177 | H | H | formyl | 4-Cl-Ph | H |
| 7-178 | H | H | formyl | 4-Cl-Ph | 4-Me |
| 7-179 | H | H | formyl | 4-Me-Ph | H |
| 7-180 | H | H | formyl | 4-Me-Ph | 4-Me |
| 7-181 | H | H | formyl | Ph | H |
| 7-182 | H | H | formyl | Ph | 4-Me |
| 7-183 | H | H | formyl | 2-pyridyl | H |
| 7-184 | H | H | formyl | 3-pyridyl | H |
| 7-185 | H | H | formyl | 5-Cl-pyridin-2-yl | H |
| 7-186 | H | H | formyl | 5-Br-pyridin-2-yl | H |
| 7-187 | H | H | formyl | 5-F-pyridin-2-yl | H |
| 7-188 | H | H | formyl | 5-Me-pyridin-2-yl | H |
| 7-189 | H | H | formyl | 6-Me-pyridin-3-yl | H |
| 7-190 | H | H | formyl | 4-Me-pyridin-2-yl | H |
| 7-191 | H | H | formyl | 4-F-pyridin-2-yl | H |
| 7-192 | H | H | formyl | 4-Cl-pyridin-2-yl | H |
| 7-193 | H | H | formyl | 4-Br-pyridin-2-yl | H |
| 7-194 | H | H | formyl | 4-OMe-pyridin-2-yl | H |
| 7-195 | H | H | CH₂OH | 5-Me-pyridin-2-yl | H |
| 7-196 | H | H | CH₂OH | 4-Cl-Ph | H |
| 7-197 | H | H | CH₂OH | 4-Me-pyridin-2-yl | H |
| 7-198 | H | H | CH₂OH | 4-Me-Ph | H |
| 7-199 | H | H | CH₂OH | Ph | H |
| 7-200 | H | H | CH₂OH | 2-Pyridyl | H |
| 7-201 | H | H | Me | 2-Thiazolyl | H |
| 7-202 | H | H | Me | 2-Thiazolyl | 4-Cl |
| 7-203 | H | H | Me | 2-Thiazolyl | 4-Me |
| 7-204 | H | H | Me | 4-Me-thiazol-2-yl | H |
| 7-205 | H | H | Me | 4-Me-thiazol-2-yl | 4-Cl |
| 7-206 | H | H | Me | 4-Me-thiazol-2-yl | 4-Me |
| 7-207 | H | H | Me | 5-Me-thiazol-2-yl | H |
| 7-208 | H | H | Me | 5-Br-thiazol-2-yl | H |
| 7-209 | H | H | Me | 5-Br-thiazol-2-yl | 4-Me |
| 7-210 | H | H | Me | 5-Cl-thiazol-2-yl | H |
| 7-211 | H | H | Me | 4,6-Me₂-pyridin-2-yl | H |
| 7-212 | H | H | Me | 4,6-Me₂-pyridin-2-yl | 4-Me |
| 7-213 | H | H | Me | 2-Pyridyl | 4-F |
| 7-214 | H | H | Me | 2-Pyrazinyl | H |
| 7-215 | H | H | Me | 5-Me-Pyrazin-2-yl | H |
| 7-216 | H | H | Me | 2-Pyrazinyl | 4-Me |
| 7-217 | H | H | Me | 2-(1,3-Benzothiazolyl) | H |
| 7-218 | H | H | Me | 2-(1,3-Benzothiazolyl) | 4-Me |
| 7-219 | H | H | Me | 7-Cl-(1,3-benzothiazol-2-yl) | H |
| 7-220 | H | H | Me | 3-(1,5-Me₂-pyrazolyl) | H |
| 7-221 | H | H | Me | 3-(1,5-Me₂-pyrazolyl) | 4-Me |
| 7-222 | H | H | Me | 4,5-Me₂-thiazol-2-yl | H |
| 7-223 | H | H | Me | 4,5-Cl₂-thiazol-2-yl | H |
| 7-224 | H | H | Me | 2-Pyrimidinyl | H |
| 7-225 | H | H | Me | 2-Pyrimidinyl | 4-Me |
| 7-226 | H | H | Me | 5-F-pyrimidin-2-yl | H |
| 7-227 | H | H | Me | 5-Cl-pyrimidin-2-yl | H |
| 7-228 | H | H | Me | 5-Br-pyrimidin-2-yl | H |
| 7-229 | H | H | Me | 5-Me-pyrimidin-2-yl | H |
| 7-230 | H | H | Me | 5-Me-pyrimidin-2-yl | 4-Me |
| 7-231 | H | H | Me | 4,6-Me₂-pyrimidin-2-yl | H |
| 7-232 | H | H | Me | 4,6-Me₂-pyrimidin-2-yl | 4-Me |
| 7-233 | H | H | Me | 3-Pyridazinyl | H |
| 7-234 | H | H | Me | 6-Me-Pyridazin-3-yl | H |
| 7-235 | H | H | Me | 3-(1,2,4)-Triazinyl | H |
| 7-236 | H | H | Me | 6-Me-(1,2,4)-Triazin-3-yl | H |
| 7-237 | H | H | Me | Chinolin-2-yl | H |
| 7-238 | H | H | Me | Isochinolin-3-yl | H |

**Tabelle 8: Verbindungen der Formel (Ic"')**

| | | | | | |
|---|---|---|---|---|---|
| 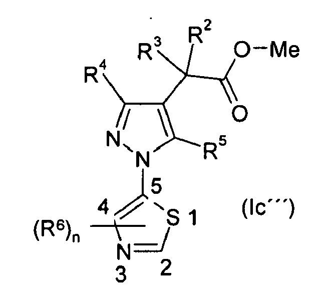 | | | | | |

| Nr. | R² | R³ | R⁴ | R⁵ | (R⁶)ₙ |
|---|---|---|---|---|---|
| 8-1 | H | H | Ph | Ph | H |
| 8-2 | H | H | Me | Ph | H |
| 8-3 | H | H | Me | 5-J-2-thienyl | H |
| 8-4 | H | H | Me | 2-Furyl | H |
| 8-5 | Me | H | Me | Ph | 2-OMe |
| 8-6 | Me | H | Me | Ph | 4-Me |
| 8-7 | Me | H | Me | Ph | 2-Cl |
| 8-8 | H | H | Me | Ph | 2-CF₃ |
| 8-9 | H | H | Me | Ph | 4-CF₃ |
| 8-10 | H | H | Me | Ph | 4-Me |
| 8-11 | H | H | Me | Ph | 2,4-Me₂ |
| 8-12 | H | H | Me | Ph | 2,4-Cl₂ |
| 8-13 | H | H | Me | 4-MeO-Ph | 4-Me |
| 8-14 | H | H | Me | 4-MeO-Ph | H |
| 8-15 | Me | H | Me | Ph | H |
| 8-16 | H | H | Me | 4-Me-Ph | H |
| 8-17 | H | H | Me | 4-Me-Ph | 4-Me |
| 8-18 | H | H | Me | 4-Me-Ph | 4-Cl |
| 8-19 | H | H | Me | 3-Cl-Ph | H |
| 8-20 | H | H | Me | 3-CF₃-Ph | H |
| 8-21 | H | H | Me | 3-CF₃-Ph | 4-Me |
| 8-22 | H | H | Me | 3,4-Cl₂-Ph | 4-Me |
| 8-23 | H | H | Me | 3-Cl-Ph | 4-Me |
| 8-24 | H | H | Me | 2-Cl-Ph | 4-Me |
| 8-25 | H | H | Me | 2,4-Cl₂-Ph | 4-Me |
| 8-26 | H | H | Me | 4-CF₃-Ph | 4-Me |
| 8-27 | H | H | Me | 4-Cl-Ph | 4-Me |
| 8-28 | H | H | Me | 4-Cl-Ph | H |
| 8-29 | H | H | Me | 4-Cl-Ph | 2-Cl |
| 8-30 | H | H | Me | 4-Cl-Ph | 4-COOEt |
| 8-31 | H | H | Me | 3,4-Cl₂-Ph | H |
| 8-32 | H | H | Me | 4-CF₃-Ph | H |
| 8-33 | H | H | Me | 4-Cl-Ph | 4-Cl |
| 8-34 | H | H | Me | Ph | 4-Cl |
| 8-35 | H | H | Me | 2-Cl-Ph | H |
| 8-36 | H | H | Me | 4-tBu-Ph | 4-Me |
| 8-37 | H | H | Me | 3,5-Me₂-Ph | 4-Me |
| 8-38 | H | H | Me | Ph | 4-OMe |
| 8-39 | H | H | Me | 4-Cl-Ph | 4-OMe |
| 8-40 | H | H | Me | 4-Me-Ph | 4-Me |
| 8-41 | H | H | Me | 4-F-Ph | 4-Me |
| 8-42 | H | H | Me | 4-F-Ph | 4-Cl |
| 8-43 | H | H | Me | 3-Me-Ph | 4-Me |
| 8-44 | H | H | Me | 4-COOH-Ph | 4-Me |
| 8-45 | H | H | Me | 3-Br-Ph | 4-Me |
| 8-46 | H | H | Me | 4-Ph-Ph | 4-Me |
| 8-47 | H | H | Me | 4-COOH-Ph | H |
| 8-48 | H | H | Me | 3,5-Me₂-Ph | H |
| 8-49 | H | H | Me | Ph | 4-SMe |
| 8-50 | H | H | Me | 4-Cl-Ph | 4-SMe |
| 8-51 | H | H | Me | 3-Cl-4-Me-Ph | H |
| 8-52 | H | H | Me | 3-CF₃-4-Cl-Ph | H |
| 8-53 | H | H | Me | 3-CF₃-4-Cl-Ph | 4-Me |
| 8-54 | H | H | Me | 3-Cl-4-Me-Ph | 4-Me |
| 8-55 | H | H | Me | 2-Pyridyl | 4-Cl |
| 8-56 | H | H | Me | 4-Cl-Ph | 4-F |
| 8-57 | H | H | Me | 2-Thienyl | 4-Me |
| 8-58 | H | H | Me | 3-Me-2-thienyl | 4-Me |
| 8-59 | H | H | Me | 4-Me-2-thienyl | 4-Me |
| 8-60 | H | H | Me | 5-Cl-2-thienyl | 4-Me |
| 8-61 | H | H | Me | 5-Cl-2-thienyl | 4-Cl |
| 8-62 | H | H | Me | 3-Thienyl | 4-Me |
| 8-63 | H | H | Me | 2-Thienyl | H |
| 8-64 | H | H | Me | 3-Me-2-thienyl | H |
| 8-65 | H | H | Me | 4-Me-2-thienyl | H |
| 8-66 | H | H | Me | 5-Cl-2-thienyl | H |
| 8-67 | H | H | Me | 5-Me-2-thienyl | H |
| 8-68 | H | H | Me | 6-MeO-pyridin-3-yl | H |
| 8-69 | H | H | Me | 5-Br-2-thienyl | H |
| 8-70 | H | H | Me | 5-Br-2-thienyl | 4-Me |
| 8-71 | H | H | Me | 3-Thienyl | H |
| 8-72 | H | H | Me | 4-Cl-Ph | 4-S(O)Me |
| 8-73 | H | H | Me | 4-Br-Ph | 4-Me |
| 8-74 | H | H | Me | 1,3-Benzodioxol-5-yl | 4-Me |
| 8-75 | H | H | Me | 4-J-Ph | 4-Me |
| 8-76 | H | H | Me | 3,5-Cl₂-Ph | 4-Me |
| 8-77 | H | H | Me | 4-PhO-Ph | 4-Me |
| 8-78 | H | H | Me | 6-OH-pyridin-3-yl | H |
| 8-79 | H | H | Me | Ph | 4-S(O)Me |
| 8-80 | H | H | H | Ph | H |
| 8-81 | H | H | H | Ph | 4-Me |
| 8-82 | H | H | Et | Ph | H |
| 8-83 | H | H | n-Pr | Ph | H |
| 8-84 | H | H | CH₂Cl | Ph | H |
| 8-85 | H | H | CHCl₂ | Ph | H |
| 8-86 | H | H | CH₂F | Ph | H |
| 8-87 | H | H | CHF₂ | Ph | H |
| 8-88 | H | H | Cl | Ph | H |
| 8-89 | H | H | Et | Ph | 4-Me |
| 8-90 | H | H | n-Pr | Ph | 4-Me |
| 8-91 | H | H | CH₂Cl | Ph | 4-Me |
| 8-92 | H | H | CHCl₂ | Ph | 4-Me |
| 8-93 | H | H | CH₂F | Ph | 4-Me |
| 8-94 | H | H | CHF₂ | Ph | 4-Me |
| 8-95 | H | H | Cl | Ph | 4-Me |
| 8-96 | H | H | Et | 4-Cl-Ph | H |
| 8-97 | H | H | n-Pr | 4-Cl-Ph | H |
| 8-98 | H | H | CH₂C1 | 4-Cl-Ph | H |
| 8-99 | H | H | CHCl₂ | 4-Cl-Ph | H |
| 8-100 | H | H | CH₂F | 4-Cl-Ph | H |
| 8-101 | H | H | CHF₂ | 4-Cl-Ph | H |
| 8-102 | H | H | Cl | 4-Cl-Ph | H |
| 8-103 | H | H | Et | 4-Me-Ph | H |
| 8-104 | H | H | n-Pr | 4-Me-Ph | H |
| 8-105 | H | H | CH₂Cl | 4-Me-Ph | H |
| 8-106 | H | H | CHCl₂ | 4-Me-Ph | H |
| 8-107 | H | H | CH₂F | 4-Me-Ph | H |
| 8-108 | H | H | CHF₂ | 4-Me-Ph | H |
| 8-109 | H | H | Cl | 4-Me-Ph | H |
| 8-110 | H | H | Et | 2-Pyridyl | H |
| 8-111 | H | H | n-Pr | 2-Pyridyl | H |
| 8-112 | H | H | CH₂Cl | 2-Pyridyl | H |
| 8-113 | H | H | CHCl₂ | 2-Pyridyl | H |
| 8-114 | H | H | CH₂F | 2-Pyridyl | H |
| 8-115 | H | H | CHF₂ | 2-Pyridyl | H |
| 8-116 | H | H | Cl | 2-Pyridyl | H |
| 8-117 | H | H | Me | 2-Pyridyl | H |
| 8-118 | H | H | Me | 5-Cl-pyridin-2-yl | H |
| 8-119 | H | H | Me | 5-Cl-pyridin-2-yl | 4-Cl |
| 8-120 | H | H | Me | 5-Cl-pyridin-2-yl | 4-Me |
| 8-121 | H | H | Me | 5-Br-pyridin-2-yl | H |
| 8-122 | H | H | Me | 5-Br-pyridin-2-yl | 4-Cl |
| 8-123 | H | H | Me | 5-Br-pyridin-2-yl | 4-Me |
| 8-124 | H | H | Me | 5-F-pyridin-2-yl | H |
| 8-125 | H | H | Me | 5-Me-pyridin-2-yl | H |
| 8-126 | H | H | Me | 5-Me-pyridin-2-yl | 4-Me |
| 8-127 | H | H | Me | 2,4-Cl₂-Ph | H |
| 8-128 | H | H | Me | 4-(CH₂COOH)-Ph | 4-Me |
| 8-129 | H | H | Me | 3,4-Me₂-Ph | 4-Me |
| 8-130 | H | H | Me | 4-Br-Ph | 4-Me |
| 8-131 | H | H | Me | 3,4-Me₂-Ph | H |
| 8-132 | H | H | Me | 3-Me-Ph | H |
| 8-133 | H | H | Me | 4-F-Ph | H |
| 8-134 | H | H | Me | 4-(Me-CO)-Ph | H |
| 8-135 | H | H | Me | 4-tBu-Ph | H |
| 8-136 | H | H | Me | 4-Cl-3-Me-Ph | H |
| 8-137 | H | H | n-Pr | 4-CI-Ph | 4-Me |
| 8-138 | H | H | Me | 3-Pyridyl | H |
| 8-139 | H | H | Me | 4-Pyridyl | H |
| 8-140 | H | H | C(O)OMe | Ph | H |
| 8-141 | H | H | Me | 6-Me-pyridin-3-yl | H |
| 8-142 | H | H | Me | 4-Cl-Ph | 4-SO₂Me |
| 8-143 | H | H | Me | 3-Pyridyl | 4-Me |
| 8-144 | H | H | Me | 2,3-Cl₂-Ph | 4-Me |
| 8-145 | H | H | Me | 2-Pyridyl | 4-Me |
| 8-146 | H | H | H | 4-Cl-Ph | 4-Me |
| 8-147 | H | H | Me | 6-Cl-pyridin-3-yl | H |
| 8-148 | H | H | Me | 4-Cl-Ph | 2-Me |
| 8-149 | H | H | Me | Ph | 2-Me |
| 8-150 | H | H | Me | 4-Me-pyridin-2-yl | H |
| 8-151 | H | H | Me | 4-Me-pyridin-2-yl | 4-Me |
| 8-152 | H | H | Me | 4-Me-pyridin-2-yl | 4-Cl |
| 8-153 | H | H | Me | 4-Me-pyridin-2-yl | 4-F |
| 8-154 | H | H | Me | 4-F-pyridin-2-yl | H |
| 8-155 | H | H | Me | 4-Cl-pyridin-2-yl | H |
| 8-156 | H | H | Me | 4-Br-pyridin-2-yl | H |
| 8-157 | H | H | Me | 4-OMe-pyridin-2-yl | H |
| 8-158 | H | H | Me | 5-CF₃-pyridin-2-yl | H |
| 8-159 | H | H | Me | 6-OMe-pyridin-2-yl | H |
| 8-160 | H | H | cyPr | 4-Cl-Ph | H |
| 8-161 | H | H | CN | 4-Cl-Ph | H |
| 8-162 | H | H | CN | 4-Cl-Ph | 4-Me |
| 8-163 | H | H | CN | 4-Me-Ph | H |
| 8-164 | H | H | CN | 4-Me-Ph | 4-Me |
| 8-165 | H | H | CN | Ph | H |
| 8-166 | H | H | CN | Ph | 4-Me |
| 8-167 | H | H | CN | 2-pyridyl | H |
| 8-168 | H | H | CN | 3-pyridyl | H |
| 8-169 | H | H | CN | 5-Cl-pyridin-2-yl | H |
| 8-170 | H | H | CN | 5-Br-pyridin-2-yl | H |
| 8-171 | H | H | CN | 5-F-pyridin-2-yl | H |
| 8-172 | H | H | CN | 5-Me-pyridin-2-yl | H |
| 8-173 | H | H | CN | 6-Me-pyridin-3-yl | H |
| 8-174 | H | H | CN | 4-Me-pyridin-2-yl | H |
| 8-175 | H | H | CN | 4-F-pyridin-2-yl | H |
| 8-176 | H | H | CN | 4-Cl-pyridin-2-yl | H |
| 8-177 | H | H | CN | 4-Br-pyridin-2-yl | H |
| 8-178 | H | H | CN | 4-OMe-pyridin-2-yl | H |
| 8-179 | H | H | formyl | 4-Cl-Ph | H |
| 8-180 | H | H | formyl | 4-Cl-Ph | 4-Me |
| 8-181 | H | H | formyl | 4-Me-Ph | H |
| 8-182 | H | H | formyl | 4-Me-Ph | 4-Me |
| 8-183 | H | H | formyl | Ph | H |
| 8-184 | H | H | formyl | Ph | 4-Me |
| 8-185 | H | H | formyl | 2-pyridyl | H |
| 8-186 | H | H | formyl | 3-pyridyl | H |
| 8-187 | H | H | formyl | 5-Cl-pyridin-2-yl | H |
| 8-188 | H | H | formyl | 5-Br-pyridin-2-yl | H |
| 8-189 | H | H | formyl | 5-F-pyridin-2-yl | H |
| 8-190 | H | H | formyl | 5-Me-pyridin-2-yl | H |
| 8-191 | H | H | formyl | 6-Me-pyridin-3-yl | H |
| 8-192 | H | H | formyl | 4-Me-pyridin-2-yl | H |
| 8-193 | H | H | formyl | 4-F-pyridin-2-yl | H |
| 8-194 | H | H | formyl | 4-Cl-pyridin-2-yl | H |
| 8-195 | H | H | formyl | 4-Br-pyridin-2-yl | H |
| 8-196 | H | H | formyl | 4-OMe-pyridin-2-yl | H |
| 8-197 | H | H | CH₂OH | 5-Me-pyridin-2-yl | H |
| 8-198 | H | H | CH₂OH | 4-Cl-Ph | H |
| 8-199 | H | H | CH₂OH | 4-Me-pyridin-2-yl | H |
| 8-200 | H | H | CH₂OH | 4-Me-Ph | H |
| 8-201 | H | H | CH₂OH | Ph | H |
| 8-202 | H | H | CH₂OH | 2-Pyridyl | H |
| 8-203 | H | H | Me | 2-Thiazolyl | H |
| 8-204 | H | H | Me | 2-Thiazolyl | 4-Cl |
| 8-205 | H | H | Me | 2-Thiazolyl | 4-Me |
| 8-206 | H | H | Me | 4-Me-thiazol-2-yl | H |
| 8-207 | H | H | Me | 4-Me-thiazol-2-yl | 4-Cl |
| 8-208 | H | H | Me | 4-Me-thiazol-2-yl | 4-Me |
| 8-209 | H | H | Me | 5-Me-thiazol-2-yl | H |
| 8-210 | H | H | Me | 5-Br-thiazol-2-yl | H |
| 8-211 | H | H | Me | 5-Br-thiazol-2-yl | 4-Me |
| 8-212 | H | H | Me | 5-Cl-thiazol-2-yl | H |
| 8-213 | H | H | Me | 4,6-Me₂-pyridin-2-yl | H |
| 8-214 | H | H | Me | 4,6-Me₂-pyridin-2-yl | 4-Me |
| 8-215 | H | H | Me | 2-Pyridyl | 4-F |
| 8-216 | H | H | Me | 2-Pyrazinyl | H |
| 8-217 | H | H | Me | 5-Me-Pyrazin-2-yl | H |
| 8-218 | H | H | Me | 2-Pyrazinyl | 4-Me |
| 8-219 | H | H | Me | 2-(1,3-Benzothiazolyl) | H |
| 8-220 | H | H | Me | 2-(1,3-Benzothiazolyl) | 4-Me |
| 8-221 | H | H | Me | 7-Cl-(1,3-benzothiazol-2-yl) | H |
| 8-222 | H | H | Me | 3-(1,5-Me₂-pyrazolyl) | H |
| 8-223 | H | H | Me | 3-(1,5-Me₂-pyrazolyl) | 4-Me |
| 8-224 | H | H | Me | 4,5-Me₂-thiazol-2-yl | H |
| 8-225 | H | H | Me | 4,5-Cl₂-thiazol-2-yl | H |
| 8-226 | H | H | Me | 2-Pyrimidinyl | H |
| 8-227 | H | H | Me | 2-Pyrimidinyl | 4-Me |
| 8-228 | H | H | Me | 5-F-pyrimidin-2-yl | H |
| 8-229 | H | H | Me | 5-Cl-pyrimidin-2-yl | H |
| 8-230 | H | H | Me | 5-Br-pyrimidin-2-yl | H |
| 8-231 | H | H | Me | 5-Me-pyrimidin-2-yl | H |
| 8-232 | H | H | Me | 5-Me-pyrimidin-2-yl | 4-Me |
| 8-233 | H | H | Me | 4,6-Me₂-pyrimidin-2-yl | H |
| 8-234 | H | H | Me | 4,6-Me₂-pyrimidin-2-yl | 4-Me |
| 8-235 | H | H | Me | 3-Pyridazinyl | H |
| 8-236 | H | H | Me | 6-Me-Pyridazin-3-yl | H |
| 8-237 | H | H | Me | 3-(1,2,4)-Triazinyl | H |
| 8-238 | H | H | Me | 6-Me-(1,2,4)-Triazin-3-yl | H |
| 8-239 | H | H | Me | Chinolin-2-yl | H |
| 8-240 | H | H | Me | Isochinolin-3-yl | H |
| 8-241 | H | H | Me | 2-Pyridyl | 4-COOEt |

**Tabelle 9: Verbindungen der Formel (Ic"")**

| | | | | |
|---|---|---|---|---|
| 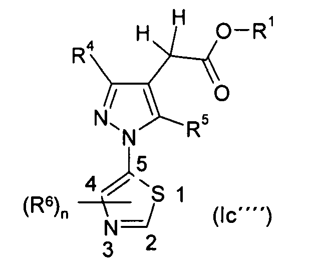 | | | | |

| Nr. | R¹ | R⁴ | R⁵ | (R⁶)ₙ |
|---|---|---|---|---|
| 9-1 | Et | Me | Ph | H |
| 9-2 | Et | Me | Ph | 4-Me |
| 9-3 | Et | Me | 3-Cl-Ph | H |
| 9-4 | Et | Me | 4-Cl-Ph | H |
| 9-5 | Et | Me | 4-Cl-Ph | 4-Me |
| 9-6 | Et | Me | 2-Thienyl | H |
| 9-7 | Et | Me | 3-Thienyl | H |
| 9-8 | Et | Me | 3-Me-2-thienyl | H |
| 9-9 | Et | Me | 4-Me-2-thienyl | H |
| 9-10 | Et | Me | 5-Br-2-thienyl | H |
| 9-11 | Et | Me | 5-Br-2-thienyl | 4-Me |
| 9-12 | Et | Me | 5-Cl-2-thienyl | H |
| 9-13 | Et | Me | 5-Cl-2-thienyl | 4-Me |
| 9-14 | Et | Me | 5-J-2-thienyl | H |
| 9-15 | Et | Me | 5-Me-2-thienyl | H |
| 9-16 | Et | Me | 3-Pyridyl | H |
| 9-17 | Et | Me | 6-MeO-pyridin-3-yl | H |
| 9-18 | Et | Me | 6-OH-pyridin-3-yl | H |
| 9-19 | Et | Me | 6-Me-pyridin-3-yl | H |
| 9-20 | Et | Me | 4-Me-Ph | H |
| 9-21 | Et | Me | 4-Me-Ph | 4-Me |
| 9-22 | Et | Me | 4-Br-Ph | H |
| 9-23 | Et | Me | 4-F-Ph | H |
| 9-24 | Et | Me | 4-F-Ph | 4-Me |
| 9-25 | Et | Me | 5-Cl-pyridin-2-yl | H |
| 9-26 | Et | Me | 5-Br-pyridin-2-yl | H |
| 9-27 | Et | Me | 5-F-pyridin-2-yl | H |
| 9-28 | Et | Me | 5-F-pyridin-2-yl | 4-Me |
| 9-29 | Et | Me | 5-Cl-pyridin-2-yl | 4-Me |
| 9-30 | Et | Me | 5-Br-pyridin-2-yl | 4-Me |
| 9-31 | Et | Me | 5-Me-pyridin-2-yl | H |
| 9-32 | Et | Me | 5-Me-pyridin-2-yl | 4-Me |
| 9-33 | Et | Me | 2-Pyridyl | 4-Me |
| 9-34 | Et | Me | 2-Pyridyl | H |
| 9-35 | Et | Me | 4-Pyridyl | H |
| 9-36 | Et | Me | 4-Me-pyridin-2-yl | H |
| 9-37 | Et | Me | 4-Me-pyridin-2-yl | 4-Me |
| 9-38 | Et | Me | 2-Thiazolyl | H |
| 9-39 | Et | Me | 4-Me-thiazol-2-yl | H |
| 9-40 | Et | Me | 5-Br-thiazol-2-yl | H |
| 9-41 | Et | Me | 5-Cl-thiazol-2-yl | H |
| 9-42 | Et | Me | 5-Me-thiazol-2-yl | H |
| 9-43 | Et | Me | 4,5-Me₂-thiazol-2-yl | H |
| 9-44 | Et | Me | 4,5-Cl₂-thiazol-2-yl | H |
| 9-45 | Et | Me | 4,6-Me₂-pyridin-2-yl | H |
| 9-46 | Et | Me | 2-Pyrazinyl | H |
| 9-47 | Et | Me | 2-Pyrimidinyl | H |
| 9-48 | Et | Me | 2-Pyrimidinyl | 4-Me |
| 9-49 | Et | Me | 5-Cl-pyrimidin-2-yl | H |
| 9-50 | Et | Me | 5-Br-pyrimidin-2-yl | H |
| 9-51 | Et | Me | 5-Me-pyrimidin-2-yl | H |
| 9-52 | Et | Me | 5-Me-pyrimidin-2-yl | 4-Me |
| 9-53 | Et | Me | 4,6-Me₂-pyrimidin-2-yl | H |
| 9-54 | Et | Me | 4,6-Me₂-pyrimidin-2-yl | 4-Me |
| 9-55 | Et | Me | 2-(1,3-Benzothiazolyl) | H |
| 9-56 | Et | Me | 7-Cl-(1,3-benzothiazol-2-yl) | H |
| 9-57 | Et | Me | 3-(1,5-Me₂-pyrazolyl) | H |
| 9-58 | Et | Me | 5-Me-Pyrazin-2-yl | H |
| 9-59 | Et | Me | 5-F-pyrimidin-2-yl | H |
| 9-60 | Et | Me | 4,6-Me₂-pyrimidin-2-yl | H |
| 9-61 | Et | Me | 3-Pyridazinyl | H |
| 9-62 | Et | Me | 6-Me-Pyridazin-3-yl | H |
| 9-63 | Et | Me | 3-(1,2,4)-Triazinyl | H |
| 9-64 | Et | Me | 6-Me-(1,2,4)-Triazin-3-yl | H |
| 9-65 | Et | Me | Chinolin-2-yl | H |
| 9-66 | Et | Me | Isochinolin-3-yl | H |
| 9-67 | Pr | Me | Ph | H |
| 9-68 | Pr | Me | 4-Cl-Ph | H |
| 9-69 | Pr | Me | 2-Thienyl | H |
| 9-70 | Pr | Me | 3-Pyridyl | H |
| 9-71 | Pr | Me | 6-Me-pyridin-3-yl | H |
| 9-72 | Pr | Me | 4-Me-Ph | H |
| 9-73 | Pr | Me | 4-Br-Ph | H |
| 9-74 | Pr | Me | 4-F-Ph | H |
| 9-75 | Pr | Me | 5-Cl-pyridin-2-yl | H |
| 9-76 | Pr | Me | 5-Br-pyridin-2-yl | H |
| 9-77 | Pr | Me | 5-F-pyridin-2-yl | H |
| 9-78 | Pr | Me | 5-Me-pyridin-2-yl | H |
| 9-79 | Pr | Me | 2-Pyridyl | H |
| 9-80 | Pr | Me | 4-Pyridyl | H |
| 9-81 | i-Pr | Me | Ph | H |
| 9-82 | i-Pr | Me | 4-Cl-Ph | H |
| 9-83 | i-Pr | Me | 2-Thienyl | H |
| 9-84 | i-Pr | Me | 3-Pyridyl | H |
| 9-85 | i-Pr | Me | 6-Me-pyridin-3-yl | H |
| 9-86 | i-Pr | Me | 4-Me-Ph | H |
| 9-87 | i-Pr | Me | 4-Br-Ph | H |
| 9-88 | i-Pr | Me | 4-F-Ph | H |
| 9-89 | i-Pr | Me | 5-Cl-pyridin-2-yl | H |
| 9-90 | i-Pr | Me | 5-Br-pyridin-2-yl | H |
| 9-91 | i-Pr | Me | 5-F-pyridin-2-yl | H |
| 9-92 | i-Pr | Me | 5-Me-pyridin-2-yl | H |
| 9-93 | i-Pr | Me | 2-Pyridyl | H |
| 9-94 | i-Pr | Me | 4-Pyridyl | H |
| 9-95 | CH₂Ph | Me | Ph | H |
| 9-96 | CH₂Ph | Me | 4-Cl-Ph | H |
| 9-97 | CH₂Ph | Me | 2-Thienyl | H |
| 9-98 | CH₂Ph | Me | 2-Pyridyl | H |
| 9-99 | prop-2-in-1-yl | Me | Ph | H |
| 9-100 | Prop-2-in-1-yl | Me | 4-Cl-Ph | H |
| 9-101 | Prop-2-in-1-yl | Me | 2-Thienyl | H |
| 9-102 | Prop-2-in-1-yl | Me | 3-Thienyl | H |
| 9-103 | Prop-2-in-1-yl | Me | 3-Me-2-thienyl | H |
| 9-104 | Prop-2-in-1-yl | Me | 4-Me-2-thienyl | H |
| 9-105 | Prop-2-in-1-yl | Me | 5-Cl-2-thienyl | H |
| 9-106 | Prop-2-in-1-yl | Me | 5-Me-2-thienyl | H |
| 9-107 | Prop-2-in-1-yl | Me | 3-Pyridyl | H |
| 9-108 | Prop-2-in-1-yl | Me | 6-MeO-pyridin-3-yl | H |
| 9-109 | Prop-2-in-1-yl | H | Ph | H |
| 9-110 | Prop-2-in-1-yl | Me | 6-Me-pyridin-3-yl | H |
| 9-111 | Prop-2-in-1-yl | Me | 4-Me-Ph | H |
| 9-112 | Prop-2-in-1-yl | Me | 4-Br-Ph | H |
| 9-113 | Prop-2-in-1-yl | Me | 4-F-Ph | H |
| 9-114 | Prop-2-in-1-yl | Me | 5-Cl-pyridin-2-yl | H |
| 9-115 | Prop-2-in-1-yl | Me | 5-Br-pyridin-2-yl | H |
| 9-116 | Prop-2-in-1-yl | Me | 5-F-pyridin-2-yl | H |
| 9-117 | Prop-2-in-1-yl | Me | 5-Me-pyridin-2-yl | H |
| 9-118 | Prop-2-in-1-yl | Me | 2-Pyridyl | H |
| 9-119 | Prop-2-in-1-yl | Me | 4-Pyridyl | H |
| 9-120 | Prop-2-in-1-yl | Me | 4-Cl-Ph | 4-Me |
| 9-121 | Prop-2-in-1-yl | Me | Ph | 4-Me |
| 9-122 | Cyclopropylmethyl | Me | Ph | H |
| 9-123 | Cyclopropylmethyl | Me | 4-Cl-Ph | H |
| 9-124 | Cyclopropylmethyl | Me | 2-Thienyl | H |
| 9-125 | Cyclopropylmethyl | Me | 3-Thienyl | H |
| 9-126 | Cyclopropylmethyl | Me | 3-Me-2-thienyl | H |
| 9-127 | Cyclopropylmethyl | Me | 3-Pyridyl | H |
| 9-128 | Cyclopropylmethyl | Me | 5-Cl-2-thienyl | H |
| 9-129 | Cyclopropylmethyl | Me | 5-Me-2-thienyl | H |
| 9-130 | Cyclopropylmethyl | Me | 4-Me-2-thienyl | H |
| 9-131 | Cyclopropylmethyl | Me | 6-MeO-pyridin-3-yl | H |
| 9-132 | Cyclopropylmethyl | Me | 6-OH-pyridin-3-yl | H |
| 9-133 | Cyclopropylmethyl | Me | 6-Me-pyridin-3-yl | H |
| 9-134 | Cyclopropylmethyl | Me | 4-Me-Ph | H |
| 9-135 | Cyclopropylmethyl | Me | 4-Br-Ph | H |
| 9-136 | Cyclopropylmethyl | Me | 4-F-Ph | H |
| 9-137 | Cyclopropylmethyl | Me | 5-Cl-pyridin-2-yl | H |
| 9-138 | Cyclopropylmethyl | Me | 5-Br-pyridin-2-yl | H |
| 9-139 | Cyclopropylmethyl | Me | 5-F-pyridin-2-yl | H |
| 9-140 | Cyclopropylmethyl | Me | 5-Me-pyridin-2-yl | H |
| 9-141 | Cyclopropylmethyl | Me | 2-Pyridyl | H |
| 9-142 | Cyclopropylmethyl | Me | 4-Pyridyl | H |
| 9-143 | Cyclopropylmethyl | Me | 4-Cl-Ph | 4-Me |
| 9-144 | Cyclopropylmethyl | Me | Ph | 4-Me |
| 9-145 | Cyclopropylmethyl | H | Ph | H |
| 9-146 | Cyclopropylmethyl | H | Chinolin-2-yl | H |
| 9-147 | Cyclopropylmethyl | H | Isochinolin-3-yl | H |
| 9-148 | 3,3-Dichlor-2-fluorprop-2-en-1-yl | Me | Ph | H |
| 9-149 | 3,3-Dichlor-2-fluorprop-2-en-1-yl | Me | 4-Cl-Ph | H |
| 9-150 | 3,3-Dichlor-2-fluorprop-2-en-1-yl | Me | 2-Thienyl | H |
| 9-151 | 3,3-Dichlor-2-fluorprop-2-en-1-yl | Me | 2-Pyridyl | H |
| 9-152 | (1-Methylcyclopropyl)-methyl | Me | Ph | H |
| 9-153 | (1-Methylcyclopropyl)-methyl | Me | 4-Cl-Ph | H |
| 9-154 | (1-Methylcyclopropyl)-methyl | Me | 2-Thienyl | H |
| 9-155 | (1-Methylcyclopropyl)-methyl | Me | 2-Pyridyl | H |
| 9-156 | 4-Chlorbut-2-in-1-yl | Me | Ph | H |
| 9-157 | 4-Chlorbut-2-in-1-yl | Me | 4-Cl-Ph | H |
| 9-158 | 4-Chlorbut-2-in-1-yl | Me | 2-Thienyl | H |
| 9-159 | 4-Chlorbut-2-in-1-yl | Me | 2-Pyridyl | H |
| 9-160 | (2,2-Dichlorcyclopropyl)-methyl | Me | Ph | H |
| 9-161 | (2,2-Dichlorcyclopropyl)-methyl | Me | 4-Cl-Ph | H |
| 9-162 | (2,2-Dichlorcyclopropyl)-methyl | Me | 2-Thienyl | H |
| 9-163 | (2,2-Dichlorcyclopropyl)-methyl | Me | 2-Pyridyl | H |
| 9-164 | But-2-in-1-yl | Me | Ph | H |
| 9-165 | But-2-in-1-yl | Me | 4-Cl-Ph | H |
| 9-166 | But-2-in-1-yl | Me | 2-Thienyl | H |
| 9-167 | But-2-in-1-yl | Me | 3-Thienyl | H |
| 9-168 | But-2-in-1-yl | Me | 3-Me-2-thienyl | H |
| 9-169 | But-2-in-1-yl | Me | 4-Me-2-thienyl | H |
| 9-170 | But-2-in-1-yl | Me | 5-Cl-2-thienyl | H |
| 9-171 | But-2-in-1-yl | Me | 5-Me-2-thienyl | H |
| 9-172 | But-2-in-1-yl | Me | 3-Pyridyl | H |
| 9-173 | But-2-in-1-yl | Me | 6-MeO-pyridin-3-yl | H |
| 9-174 | But-2-in-1-yl | H | Ph | H |
| 9-175 | But-2-in-1-yl | Me | 6-Me-pyridin-3-yl | H |
| 9-176 | But-2-in-1-yl | Me | 4-Me-Ph | H |
| 9-177 | But-2-in-1-yl | Me | 4-Br-Ph | H |
| 9-178 | But-2-in-1-yl | Me | 4-F-Ph | H |
| 9-179 | But-2-in-1-yl | Me | 5-Cl-pyridin-2-yl | H |
| 9-180 | But-2-in-1-yl | Me | 5-Br-pyridin-2-yl | H |
| 9-181 | But-2-in-1-yl | Me | 5-F-pyridin-2-yl | H |
| 9-182 | But-2-in-1-yl | Me | 5-Me-pyridin-2-yl | H |
| 9-183 | But-2-in-1-yl | Me | 2-Pyridyl | H |
| 9-184 | But-2-in-1-yl | Me | 4-Pyridyl | H |
| 9-185 | But-2-in-1-yl | Me | 4-Cl-Ph | 4-Me |
| 9-186 | But-2-in-1-yl | Me | Ph | 4-Me |
| 9-187 | 1-Methylprop-2-in-1-yl | Me | Ph | H |
| 9-188 | 1-Methylprop-2-in-1-yl | Me | 4-Cl-Ph | H |
| 9-189 | 1-Methylprop-2-in-1-yl | Me | 2-Thienyl | H |
| 9-190 | 1-Methylprop-2-in-1-yl | Me | 2-Pyridyl | H |
| 9-191 | 1-Cyclopropylethyl | Me | Ph | H |
| 9-192 | 1-Cyclopropylethyl | Me | 4-Cl-Ph | H |
| 9-193 | 1-Cyclopropylethyl | Me | 2-Thienyl | H |
| 9-194 | 1-Cyclopropylethyl | Me | 2-Pyridyl | H |
| 9-195 | Allyl | Me | Ph | H |
| 9-196 | Allyl | Me | 4-Cl-Ph | H |
| 9-197 | Allyl | Me | 2-Thienyl | H |
| 9-198 | Allyl | Me | 2-Pyridyl | H |
| 9-199 | 3-Methylbut-2-en-1-yl | Me | Ph | H |
| 9-200 | 3-Methylbut-2-en-1-yl | Me | 4-Cl-Ph | H |
| 9-201 | 3-Methylbut-2-en-1-yl | Me | 2-Thienyl | H |
| 9-202 | 3-Methylbut-2-en-1-yl | Me | 2-Pyridyl | H |
| 9-203 | 2-Methylprop-2-en-1-yl | Me | Ph | H |
| 9-204 | 2-Methylprop-2-en-1-yl | Me | 4-Cl-Ph | H |
| 9-205 | 2-Methylprop-2-en-1-yl | Me | 2-Thienyl | H |
| 9-206 | 2-Methylprop-2-en-1-yl | Me | 2-Pyridyl | H |
| 9-207 | (2E)-1-Methylbut-2-en-1-yl | Me | Ph | H |
| 9-208 | (2E)-1-Methylbut-2-en-1-yl | Me | 4-Cl-Ph | H |
| 9-209 | (2E)-1-Methylbut-2-en-1-yl | Me | 2-Thienyl | H |
| 9-210 | (2E)-1-Methylbut-2-en-1-yl | Me | 2-Pyridyl | H |
| 9-211 | 3-Phenylprop-2-in-1-yl | Me | Ph | H |
| 9-212 | 3-Phenylprop-2-in-1-yl | Me | 4-Cl-Ph | H |
| 9-213 | 3-Phenylprop-2-in-1-yl | Me | 2-Thienyl | H |
| 9-214 | 3-Phenylprop-2-in-1-yl | Me | 2-Pyridyl | H |
| 9-215 | Cyclobutylmethyl | Me | Ph | H |
| 9-216 | Cyclobutylmethyl | Me | 4-Cl-Ph | H |
| 9-217 | Cyclobutylmethyl | Me | 2-Thienyl | H |
| 9-218 | Cyclobutylmethyl | Me | 2-Pyridyl | H |
| 9-219 | Cyclopentylmethyl | Me | Ph | H |
| 9-220 | Cyclopentylmethyl | Me | 4-Cl-Ph | H |
| 9-221 | Cyclopentylmethyl | Me | 2-Thienyl | H |
| 9-222 | Cyclopentylmethyl | Me | 2-Pyridyl | H |
| 9-223 | Cyclohexylmethyl | Me | Ph | H |
| 9-224 | Cyclohexylmethyl | Me | 4-Cl-Ph | H |
| 9-225 | Cyclohexylmethyl | Me | 2-Thienyl | H |
| 9-226 | Cyclohexylmethyl | Me | 2-Pyridyl | H |
| 9-227 | But-3-en-1-yl | Me | Ph | H |
| 9-228 | But-3-en-1-yl | Me | 4-Cl-Ph | H |
| 9-229 | But-3-en-1-yl | Me | 2-Thienyl | H |
| 9-230 | But-3-en-1-yl | Me | 2-Pyridyl | H |
| 9-231 | 2-Chloroprop-2-en-1-yl | Me | Ph | H |
| 9-232 | 2-Chloroprop-2-en-1-yl | Me | 4-Cl-Ph | H |
| 9-233 | 2-Chloroprop-2-en-1-yl | Me | 2-Thienyl | H |
| 9-234 | 2-Chloroprop-2-en-1-yl | Me | 3-Thienyl | H |
| 9-235 | 2-Chloroprop-2-en-1-yl | Me | 3-Me-2-thienyl | H |
| 9-236 | 2-Chloroprop-2-en-1-yl | Me | 4-Me-2-thienyl | H |
| 9-237 | 2-Chloroprop-2-en-1-yl | Me | 5-Cl-2-thienyl | H |
| 9-238 | 2-Chloroprop-2-en-1-yl | Me | 5-Me-2-thienyl | H |
| 9-239 | 2-Chloroprop-2-en-1-yl | Me | 3-Pyridyl | H |
| 9-240 | 2-Chloroprop-2-en-1-yl | Me | 6-MeO-pyridin-3-yl | H |
| 9-241 | 2-Chloroprop-2-en-1-yl | Me | 6-OH-pyridin-3-yl | H |
| 9-242 | 2-Chloroprop-2-en-1-yl | Me | 6-Me-pyridin-3-yl | H |
| 9-243 | 2-Chloroprop-2-en-1-yl | Me | 4-Me-Ph | H |
| 9-244 | 2-Chloroprop-2-en-1-yl | Me | 4-Br-Ph | H |
| 9-245 | 2-Chloroprop-2-en-1-yl | Me | 4-F-Ph | H |
| 9-246 | 2-Chloroprop-2-en-1-yl | Me | 5-Cl-pyridin-2-yl | H |
| 9-247 | 2-Chloroprop-2-en-1-yl | Me | 5-Br-pyridin-2-yl | H |
| 9-248 | 2-Chloroprop-2-en-1-yl | Me | 5-F-pyridin-2-yl | H |
| 9-249 | 2-Chloroprop-2-en-1-yl | Me | 5-Me-pyridin-2-yl | H |
| 9-250 | 2-Chloroprop-2-en-1-yl | Me | 2-Pyridyl | H |
| 9-251 | 2-Chloroprop-2-en-1-yl | Me | 4-Pyridyl | H |
| 9-252 | 2-Chloroprop-2-en-1-yl | Me | 4-Cl-Ph | 4-Me |
| 9-253 | 2-Chloroprop-2-en-1-yl | Me | Ph | 4-Me |
| 9-254 | 2-Chloroprop-2-en-1-yl | H | Ph | H |
| 9-255 | 2-Chloroprop-2-en-1-yl | H | Chinolin-2-yl | H |
| 9-256 | 2-Chloroprop-2-en-1-yl | H | Isochinolin-3-yl | H |
| 9-257 | 2-Methoxyethyl | Me | Ph | H |
| 9-258 | 2-Methoxyethyl | Me | 4-Cl-Ph | H |
| 9-259 | 2-Methoxyethyl | Me | 2-Thienyl | H |
| 9-260 | 2-Methoxyethyl | Me | 2-Pyridyl | H |
| 9-261 | Tetrahydrofuran-2-yl-methyl | Me | Ph | H |
| 9-262 | Tetrahydrofuran-2-yl-methyl | Me | 4-Cl-Ph | H |
| 9-263 | Tetrahydrofuran-2-yl-methyl | Me | 2-Thienyl | H |
| 9-264 | Tetrahydrofuran-2-yl-methyl | Me | 2-Pyridyl | H |
| 9-265 | 2-(Dimethylamino)ethyl | Me | Ph | H |
| 9-266 | 2-(Dimethylamino)ethyl | Me | 4-Cl-Ph | H |
| 9-267 | 2-(Dimethylamino)ethyl | Me | 2-Thienyl | H |
| 9-268 | 2-(Dimethylamino)ethyl | Me | 2-Pyridyl | H |
| 9-269 | Oxetan-3-yl | Me | Ph | H |
| 9-270 | Oxetan-3-yl | Me | 4-Cl-Ph | H |
| 9-271 | Oxetan-3-yl | Me | 2-Thienyl | H |
| 9-272 | Oxetan-3-yl | Me | 2-Pyridyl | H |
| 9-273 | (3-Methyloxetan-3-yl)methyl | Me | Ph | H |
| 9-274 | (3-Methyloxetan-3-yl)methyl | Me | 4-Cl-Ph | H |
| 9-275 | (3-Methyloxetan-3-yl)methyl | Me | 2-Thienyl | H |
| 9-276 | (3-Methyloxetan-3-yl)methyl | Me | 2-Pyridyl | H |
| 9-277 | 2,2,2-Trifluorethyl | Me | Ph | H |
| 9-278 | 2,2,2-Trifluorethyl | Me | 4-Cl-Ph | H |
| 9-279 | 2,2,2-Trifluorethyl | Me | 2-Thienyl | H |
| 9-280 | 2,2,2-Trifluorethyl | Me | 3-Pyridyl | H |
| 9-281 | 2,2,2-Trifluorethyl | Me | 6-Me-pyridin-3-yl | H |
| 9-282 | 2,2,2-Trifluorethyl | Me | 4-Me-Ph | H |
| 9-283 | 2,2,2-Trifluorethyl | Me | 4-Br-Ph | H |
| 9-284 | 2,2,2-Trifluorethyl | Me | 4-F-Ph | H |
| 9-285 | 2,2,2-Trifluorethyl | Me | 5-Cl-pyridin-2-yl | H |
| 9-286 | 2,2,2-Trifluorethyl | Me | 5-Br-pyridin-2-yl | H |
| 9-287 | 2,2,2-Trifluorethyl | Me | 5-F-pyridin-2-yl | H |
| 9-288 | 2,2,2-Trifluorethyl | Me | 5-Me-pyridin-2-yl | H |
| 9-289 | 2,2,2-Trifluorethyl | Me | 2-Pyridyl | H |
| 9-290 | 2,2,2-Trifluorethyl | Me | 4-Pyridyl | H |
| 9-291 | CH₂(4-Cl-Ph) | Me | Ph | H |
| 9-292 | CH₂(4-Cl-Ph) | Me | 4-Cl-Ph | H |
| 9-293 | CH₂(4-Cl-Ph) | Me | 2-Thienyl | H |
| 9-294 | CH₂(4-Cl-Ph) | Me | 3-Pyridyl | H |
| 9-295 | CH₂(4-Cl-Ph) | Me | 6-Me-pyridin-3-yl | H |
| 9-296 | CH₂(4-Cl-Ph) | Me | 4-Me-Ph | H |
| 9-297 | CH₂(4-Cl-Ph) | Me | 4-Br-Ph | H |
| 9-298 | CH₂(4-Cl-Ph) | Me | 4-F-Ph | H |
| 9-299 | CH₂(4-Cl-Ph) | Me | 5-Cl-pyridin-2-yl | H |
| 9-300 | CH₂(4-Cl-Ph) | Me | 5-Br-pyridin-2-yl | H |
| 9-301 | CH₂(4-Cl-Ph) | Me | 5-F-pyridin-2-yl | H |
| 9-302 | CH₂(4-Cl-Ph) | Me | 5-Me-pyridin-2-yl | H |
| 9-303 | CH₂(4-Cl-Ph) | Me | 2-Pyridyl | H |
| 9-304 | CH₂(4-Cl-Ph) | Me | 4-Pyridyl | H |
| 9-305 | CH₂(4-F-Ph) | Me | Ph | H |
| 9-306 | CH₂(4-F-Ph) | Me | 4-Cl-Ph | H |
| 9-307 | CH₂(4-F-Ph) | Me | 2-Thienyl | H |
| 9-308 | CH₂(4-F-Ph) | Me | 3-Pyridyl | H |
| 9-309 | CH₂(4-F-Ph) | Me | 6-Me-pyridin-3-yl | H |
| 9-310 | CH₂(4-F-Ph) | Me | 4-Me-Ph | H |
| 9-311 | CH₂(4-F-Ph) | Me | 4-Br-Ph | H |
| 9-312 | CH₂(4-F-Ph) | Me | 4-F-Ph | H |
| 9-313 | CH₂(4-F-Ph) | Me | 5-Cl-pyridin-2-yl | H |
| 9-314 | CH₂(4-F-Ph) | Me | 5-Br-pyridin-2-yl | H |
| 9-315 | CH₂(4-F-Ph) | Me | 5-F-pyridin-2-yl | H |
| 9-316 | CH₂(4-F-Ph) | Me | 5-Me-pyridin-2-yl | H |
| 9-317 | CH₂(4-F-Ph) | Me | 2-Pyridyl | H |
| 9-318 | CH₂(4-F-Ph) | Me | 4-Pyridyl | H |
| 9-319 | CH₂(4-OMe-Ph) | Me | Ph | H |
| 9-320 | CH₂(4-OMe-Ph) | Me | 4-Cl-Ph | H |
| 9-321 | CH₂(4-OMe-Ph) | Me | 2-Thienyl | H |
| 9-322 | CH₂(4-OMe-Ph) | Me | 3-Pyridyl | H |
| 9-323 | CH₂(4-OMe-Ph) | Me | 6-Me-pyridin-3-yl | H |
| 9-324 | CH₂(4-OMe-Ph) | Me | 4-Me-Ph | H |
| 9-325 | CH₂(4-OMe-Ph) | Me | 4-Br-Ph | H |
| 9-326 | CH₂(4-OMe-Ph) | Me | 4-F-Ph | H |
| 9-327 | CH₂(4-OMe-Ph) | Me | 5-Cl-pyridin-2-yl | H |
| 9-328 | CH₂(4-OMe-Ph) | Me | 5-Br-pyridin-2-yl | H |
| 9-329 | CH₂(4-OMe-Ph) | Me | 5-F-pyridin-2-yl | H |
| 9-330 | CH₂(4-OMe-Ph) | Me | 5-Me-pyridin-2-yl | H |
| 9-331 | CH₂(4-OMe-Ph) | Me | 2-Pyridyl | H |
| 9-332 | CH₂(4-OMe-Ph) | Me | 4-Pyridyl | H |
| 9-333 | 2,2-Difluorethyl | Me | Ph | H |
| 9-334 | 2,2-Difluorethyl | Me | 4-Cl-Ph | H |
| 9-335 | 2,2-Difluorethyl | Me | 2-Thienyl | H |
| 9-336 | 2,2-Difluorethyl | Me | 2-Pyridyl | H |
| 9-337 | Ph | Me | Ph | H |
| 9-338 | Ph | Me | 4-Cl-Ph | H |
| 9-339 | Ph | Me | 2-Thienyl | H |
| 9-340 | Ph | Me | 2-Pyridyl | H |
| 9-341 | 2-Fluorethyl | Me | Ph | H |
| 9-342 | 2-Fluorethyl | Me | 4-Cl-Ph | H |
| 9-343 | 2-Fluorethyl | Me | 2-Thienyl | H |
| 9-344 | 2-Fluorethyl | Me | 2-Pyridyl | H |
| 9-345 | 2,2,3,3,3-Pentafluorpropyl | Me | Ph | H |
| 9-346 | 2,2,3,3,3-Pentafluorpropyl | Me | 4-Cl-Ph | H |
| 9-347 | 2,2,3,3,3-Pentafluorpropyl | Me | 2-Thienyl | H |
| 9-348 | 2,2,3,3,3-Pentafluorpropyl | Me | 2-Pyridyl | H |
| 9-349 | 1-Ethyl-5-methyl-1H-pyrazol-4-methyl | Me | Ph | H |
| 9-350 | 1-Ethyl-5-methyl-1H-pyrazol-4-methyl | Me | 4-Cl-Ph | H |
| 9-351 | 1-Ethyl-5-methyl-1H-pyrazol-4-methyl | Me | 2-Thienyl | H |
| 9-352 | 1-Ethyl-5-methyl-1H-pyrazol-4- methyl | Me | 2-Pyridyl | H |

**Tabelle 10: Verbindungen der Formel (III) (Zwischenprodukte)**

| | | | | | |
|---|---|---|---|---|---|
| 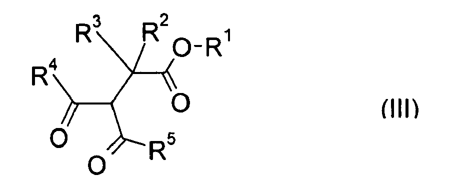 | | | | | |

| Nr. | R¹ | R² | R³ | R⁴ | R⁵ |
|---|---|---|---|---|---|
| 10-1 | Me | H | H | Ph | Ph |
| 10-2 | Me | H | H | Me | Ph |
| 10-3 | Me | H | H | Me | 2-Furyl |
| 10-4 | Me | Me | H | Me | Ph |
| 10-5 | Me | H | H | Me | 4-MeO-Ph |
| 10-6 | Me | H | H | Me | 4-Me-Ph |
| 10-7 | Me | H | H | Me | 3-CF₃-Ph |
| 10-8 | Me | H | H | Me | 3,4-Cl₂-Ph |
| 10-9 | Me | H | H | Me | 3-Cl-Ph |
| 10-10 | Me | H | H | Me | 2-Cl-Ph |
| 10-11 | Me | H | H | Me | 2,4-Cl₂-Ph |
| 10-12 | Me | H | H | Me | 4-CF₃-Ph |
| 10-13 | Me | H | H | Me | 4-Cl-Ph |
| 10-14 | Me | H | H | Me | 4-CF₃-Ph |
| 10-15 | Me | H | H | Me | 4-tBu-Ph |
| 10-16 | Me | H | H | Me | 3,5-Me₂-Ph |
| 10-17 | Me | H | H | Me | 4-Me-Ph |
| 10-18 | Me | H | H | Me | 4-F-Ph |
| 10-19 | Me | H | H | Me | 3-Me-Ph |
| 10-20 | Me | H | H | Me | 4-COOH-Ph |
| 10-21 | Me | H | H | Me | 3-Br-Ph |
| 10-22 | Me | H | H | Me | 4-Ph-Ph |
| 10-23 | Me | H | H | Me | 3-Cl-4-Me-Ph |
| 10-24 | Me | H | H | Me | 3-CF₃-4-Cl-Ph |
| 10-25 | Me | H | H | Me | 2-Thienyl |
| 10-26 | Me | H | H | Me | 3-Me-2-thienyl |
| 10-27 | Me | H | H | Me | 4-Me-2-thienyl |
| 10-28 | Me | H | H | Me | 5-Cl-2-thienyl |
| 10-29 | Me | H | H | Me | 5-J-2-thienyl |
| 10-30 | Me | H | H | Me | 3-Thienyl |
| 10-31 | Me | H | H | Me | 3-Pyridyl |
| 10-32 | Me | H | H | Me | 5-Me-2-thienyl |
| 10-33 | Me | H | H | Me | 6-MeO-pyridin-3-yl |
| 10-34 | Me | H | H | Me | 5-Br-2-thienyl |
| 10-35 | Me | H | H | Me | 4-Br-Ph |
| 10-36 | Me | H | H | Me | 1,3-Benzodioxol-5-yl |
| 10-37 | Me | H | H | Me | 4-J-Ph |
| 10-38 | Me | H | H | Me | 4-PhO-Ph |
| 10-39 | Me | H | H | Me | 6-OH-pyridin-3-yl |
| 10-40 | Me | H | H | H | Ph |
| 10-41 | Me | H | H | Et | Ph |
| 10-42 | Me | H | H | n-Pr | Ph |
| 10-43 | Me | H | H | CH₂Cl | Ph |
| 10-44 | Me | H | H | CHCl₂ | Ph |
| 10-45 | Me | H | H | CH₂F | Ph |
| 10-46 | Me | H | H | CHF₂ | Ph |
| 10-47 | Me | H | H | Cl | Ph |
| 10-48 | Me | H | H | n-Pr | 4-Cl-Ph |
| 10-49 | Me | H | H | CH₂Cl | 4-Cl-Ph |
| 10-50 | Me | H | H | CHCl₂ | 4-Cl-Ph |
| 10-51 | Me | H | H | CH₂F | 4-Cl-Ph |
| 10-52 | Me | H | H | CHF₂ | 4-Cl-Ph |
| 10-53 | Me | H | H | Cl | 4-Cl-Ph |
| 10-54 | Me | H | H | Et | 4-Me-Ph |
| 10-55 | Me | H | H | n-Pr | 4-Me-Ph |
| 10-56 | Me | H | H | CH₂Cl | 4-Me-Ph |
| 10-57 | Me | H | H | CHCl₂ | 4-Me-Ph |
| 10-58 | Me | H | H | CH₂F | 4-Me-Ph |
| 10-59 | Me | H | H | CHF₂ | 4-Me-Ph |
| 10-60 | Me | H | H | Cl | 4-Me-Ph |
| 10-61 | Me | H | H | Et | 2-Pyridyl |
| 10-62 | Me | H | H | n-Pr | 2-Pyridyl |
| 10-63 | Me | H | H | CH₂Cl | 2-Pyridyl |
| 10-64 | Me | H | H | CHCl₂ | 2-Pyridyl |
| 10-65 | Me | H | H | CH₂F | 2-Pyridyl |
| 10-66 | Me | H | H | CHF₂ | 2-Pyridyl |
| 10-67 | Me | H | H | Cl | 2-Pyridyl |
| 10-68 | Me | H | H | Me | 2-Pyridyl |
| 10-69 | Me | H | H | Me | 5-Cl-pyridin-2-yl |
| 10-70 | Me | H | H | Me | 5-Br-pyridin-2-yl |
| 10-71 | Me | H | H | Me | 5-F-pyridin-2-yl |
| 10-72 | Me | H | H | Me | 5-Me-pyridin-2-yl |
| 10-73 | Me | H | H | Me | 4-Me-pyridin-2-yl |
| 10-74 | Me | H | H | Me | 2,4-Cl₂-Ph |
| 10-75 | Me | H | H | Me | 4-CH₂COOH-Ph |
| 10-76 | Me | H | H | Me | 4-Me(CO)-Ph |
| 10-77 | Me | H | H | Me | 4-tBu-Ph |
| 10-78 | Me | H | H | Me | 4-Cl-3-Me-Ph |
| 10-79 | Me | H | H | n-Pr | 4-Cl-Ph |
| 10-80 | Me | H | H | Me | 3-Pyridyl |
| 10-81 | Me | H | H | Me | 4-Pyridyl |
| 10-82 | Me | H | H | C(O)OMe | Ph |
| 10-83 | Me | H | H | Me | 6-Me-pyridin-3-yl |
| 10-84 | Me | H | H | Me | 2,3-Cl₂-Ph |
| 10-85 | Me | H | H | H | 4-Cl-Ph |
| 10-86 | Me | H | H | Me | 6-Cl-pyridin-3-yl |
| 10-87 | Me | H | H | Me | 2-Thiazolyl |
| 10-88 | Me | H | H | Me | 4-Me-thiazol-2-yl |
| 10-89 | Me | H | H | Me | 5-Br-thiazol-2-yl |
| 10-90 | Me | H | H | Me | 5-Cl-thiazol-2-yl |
| 10-91 | Me | H | H | Me | 5-Me-thiazol-2-yl |
| 10-92 | Me | H | H | Me | 4,5-Me₂-thiazol-2-yl |
| 10-93 | Me | H | H | Me | 4,5-Cl₂-thiazol-2-yl |
| 10-94 | Me | H | H | Me | 4,6-Me₂-pyridin-2-yl |
| 10-95 | Me | H | H | Me | 2-Pyrazinyl |
| 10-96 | Me | H | H | Me | 2-Pyrimidinyl |
| 10-97 | Me | H | H | Me | 5-Cl-pyrimidin-2-yl |
| 10-98 | Me | H | H | Me | 5-Br-pyrimidin-2-yl |
| 10-99 | Me | H | H | Me | 5-Me-pyrimidin-2-yl |
| 10-100 | Me | H | H | Me | 4,6-Me₂-pyrimidin-2-yl |
| 10-101 | Me | H | H | Me | 2-(1,3-Benzothiazolyl) |
| 10-102 | Me | H | H | Me | 7-Cl-(1,3-benzothiazol-2-yl) |
| 10-103 | Me | H | H | Me | 3-(1,5-Me₂-pyrazolyl) |
| 10-104 | Me | H | H | Me | 5-Me-Pyrazin-2-yl |
| 10-105 | Me | H | H | Me | 5-F-pyrimidin-2-yl |
| 10-106 | Me | H | H | Me | 4,6-Me₂-pyrimidin-2-yl |
| 10-107 | Me | H | H | Me | 3-Pyridazinyl |
| 10-108 | Me | H | H | Me | 6-Me-Pyridazin-3-yl |
| 10-109 | Me | H | H | Me | 3-(1,2,4)-Triazinyl |
| 10-110 | Me | H | H | Me | 6-Me-(1,2,4)-Triazin-3-yl |
| 10-111 | Et | H | H | Ph | Ph |
| 10-112 | Et | H | H | Me | Ph |
| 10-113 | Et | H | H | Me | 2-Furyl |
| 10-114 | Et | Me | H | Me | Ph |
| 10-115 | Et | H | H | Me | 4-MeO-Ph |
| 10-116 | Et | H | H | Me | 4-Me-Ph |
| 10-117 | Et | H | H | Me | 3-CF₃-Ph |
| 10-118 | Et | H | H | Me | 3,4-Cl₂-Ph |
| 10-119 | Et | H | H | Me | 3-Cl-Ph |
| 10-120 | Et | H | H | Me | 2-Cl-Ph |
| 10-121 | Et | H | H | Me | 2,4-Cl₂-Ph |
| 10-122 | Et | H | H | Me | 4-CF₃-Ph |
| 10-123 | Et | H | H | Me | 4-Cl-Ph |
| 10-124 | Et | H | H | Me | 4-CF₃-Ph |
| 10-125 | Et | H | H | Me | 4-tBu-Ph |
| 10-126 | Et | H | H | Me | 3,5-Me₂-Ph |
| 10-127 | Et | H | H | Me | 4-Me-Ph |
| 10-128 | Et | H | H | Me | 4-F-Ph |
| 10-129 | Et | H | H | Me | 3-Me-Ph |
| 10-130 | Et | H | H | Me | 4-COOH-Ph |
| 10-131 | Et | H | H | Me | 3-Br-Ph |
| 10-132 | Et | H | H | Me | 4-Ph-Ph |
| 10-133 | Et | H | H | Me | 3-Cl-4-Me-Ph |
| 10-134 | Et | H | H | Me | 3-CF₃-4-Cl-Ph |
| 10-135 | Et | H | H | Me | 2-Thienyl |
| 10-136 | Et | H | H | Me | 3-Me-2-thienyl |
| 10-137 | Et | H | H | Me | 4-Me-2-thienyl |
| 10-138 | Et | H | H | Me | 5-Cl-2-thienyl |
| 10-139 | Et | H | H | Me | 5-J-2-thienyl |
| 10-140 | Et | H | H | Me | 3-Thienyl |
| 10-141 | Et | H | H | Me | 3-Pyridyl |
| 10-142 | Et | H | H | Me | 5-Me-2-thienyl |
| 10-143 | Et | H | H | Me | 6-MeO-pyridin-3-yl |
| 10-144 | Et | H | H | Me | 5-Br-2-thienyl |
| 10-145 | Et | H | H | Me | 4-Br-Ph |
| 10-146 | Et | H | H | Me | 1,3-Benzodioxol-5-yl |
| 10-147 | Et | H | H | Me | 4-J-Ph |
| 10-148 | Et | H | H | Me | 4-PhO-Ph |
| 10-149 | Et | H | H | Me | 6-OH-pyridin-3-yl |
| 10-150 | Et | H | H | H | Ph |
| 10-151 | Et | H | H | Et | Ph |
| 10-152 | Et | H | H | n-Pr | Ph |
| 10-153 | Et | H | H | CH₂Cl | Ph |
| 10-154 | Et | H | H | CHCl₂ | Ph |
| 10-155 | Et | H | H | CH₂F | Ph |
| 10-156 | Et | H | H | CHF₂ | Ph |
| 10-157 | Et | H | H | Cl | Ph |
| 10-158 | Et | H | H | n-Pr | 4-Cl-Ph |
| 10-159 | Et | H | H | CH₂Cl | 4-Cl-Ph |
| 10-160 | Et | H | H | CHCl₂ | 4-Cl-Ph |
| 10-161 | Et | H | H | CH₂F | 4-Cl-Ph |
| 10-162 | Et | H | H | CHF₂ | 4-Cl-Ph |
| 10-163 | Et | H | H | Cl | 4-Cl-Ph |
| 10-164 | Et | H | H | Et | 4-Me-Ph |
| 10-165 | Et | H | H | n-Pr | 4-Me-Ph |
| 10-166 | Et | H | H | CH₂Cl | 4-Me-Ph |
| 10-167 | Et | H | H | CHCl₂ | 4-Me-Ph |
| 10-168 | Et | H | H | CH₂F | 4-Me-Ph |
| 10-169 | Et | H | H | CHF₂ | 4-Me-Ph |
| 10-170 | Et | H | H | Cl | 4-Me-Ph |
| 10-171 | Et | H | H | Et | 2-Pyridyl |
| 10-172 | Et | H | H | n-Pr | 2-Pyridyl |
| 10-173 | Et | H | H | CH₂Cl | 2-Pyridyl |
| 10-174 | Et | H | H | CHCl₂ | 2-Pyridyl |
| 10-175 | Et | H | H | CH₂F | 2-Pyridyl |
| 10-176 | Et | H | H | CHF₂ | 2-Pyridyl |
| 10-177 | Et | H | H | Cl | 2-Pyridyl |
| 10-178 | Et | H | H | Me | 2-Pyridyl |
| 10-179 | Et | H | H | Me | 5-Cl-pyridin-2-yl |
| 10-180 | Et | H | H | Me | 5-Br-pyridin-2-yl |
| 10-181 | Et | H | H | Me | 5-F-pyridin-2-yl |
| 10-182 | Et | H | H | Me | 5-Me-pyridin-2-yl |
| 10-183 | Et | H | H | Me | 4-Me-pyridin-2-yl |
| 10-184 | Et | H | H | Me | 2,4-Cl₂-Ph |
| 10-185 | Et | H | H | Me | 4-CH₂COOH-Ph |
| 10-186 | Et | H | H | Me | 4-Me(CO)-Ph |
| 10-187 | Et | H | H | Me | 4-tBu-Ph |
| 10-188 | Et | H | H | Me | 4-Cl-3-Me-Ph |
| 10-189 | Et | H | H | n-Pr | 4-Cl-Ph |
| 10-190 | Et | H | H | Me | 3-Pyridyl |
| 10-191 | Et | H | H | Me | 4-Pyridyl |
| 10-192 | Et | H | H | C(O)OMe | Ph |
| 10-193 | Et | H | H | Me | 6-Me-pyridin-3-yl |
| 10-194 | Et | H | H | Me | 2,3-Cl₂-Ph |
| 10-195 | Et | H | H | H | 4-Cl-Ph |
| 10-196 | Et | H | H | Me | 6-Cl-pyridin-3-yl |
| 10-197 | Et | H | H | Me | 2-Thiazolyl |
| 10-198 | Et | H | H | Me | 4-Me-thiazol-2-yl |
| 10-199 | Et | H | H | Me | 5-Br-thiazol-2-yl |
| 10-200 | Et | H | H | Me | 5-Cl-thiazol-2-yl |
| 10-201 | Et | H | H | Me | 5-Me-thiazol-2-yl |
| 10-202 | Et | H | H | Me | 4,5-Me₂-thiazol-2-yl |
| 10-203 | Et | H | H | Me | 4,5-Cl₂-thiazol-2-yl |
| 10-204 | Et | H | H | Me | 4,6-Me₂-pyridin-2-yl |
| 10-205 | Et | H | H | Me | 2-Pyrazinyl |
| 10-206 | Et | H | H | Me | 2-Pyrimidinyl |
| 10-207 | Et | H | H | Me | 5-Cl-pyrimidin-2-yl |
| 10-208 | Et | H | H | Me | 5-Br-pyrimidin-2-yl |
| 10-209 | Et | H | H | Me | 5-Me-pyrimidin-2-yl |
| 10-210 | Et | H | H | Me | 4,6-Me₂-pyrimidin-2-yl |
| 10-211 | Et | H | H | Me | 2-(1,3-Benzothiazolyl) |
| 10-212 | Et | H | H | Me | 7-Cl-(1,3-benzothiazol-2-yl) |
| 10-213 | Et | H | H | Me | 3-(1,5-Me₂-pyrazolyl) |
| 10-214 | Et | H | H | Me | 5-Me-Pyrazin-2-yl |
| 10-215 | Et | H | H | Me | 5-F-pyrimidin-2-yl |
| 10-216 | Et | H | H | Me | 4,6-Me₂-pyrimidin-2-yl |
| 10-217 | Et | H | H | Me | 3-Pyridazinyl |
| 10-218 | Et | H | H | Me | 6-Me-Pyridazin-3-yl |
| 10-219 | Et | H | H | Me | 3-(1,2,4)-Triazinyl |
| 10-220 | Et | H | H | Me | 6-Me-(1,2,4)-Triazin-3-yl |
| 10-221 | Et | H | H | Me | Chinolin-2-yl |
| 10-222 | Et | H | H | Me | Isochinolin-3-yl |

Darüber hinaus wurden NMR-Daten für erfindungsgemäße Verbindungen der allgemeinen Formel (I), erzeugt. "NMR" der Beispielverbindungen wurde jeweils als ¹H-NMR-Spektum bei 400 MHz (CDCl₃) (¹H-Kernresonanz-Daten) gemessen. Nachfolgend sind charakteristische chemische Verschiebungen δ (ppm) für einige Beispielverbindungen aufgeführt:

NMR Verbindung 8-28 (CDCl₃, 400 MHz, δ in ppm):
2.31 (s, 3H); 3.35 (s, 2H); 3.70 (s, 3H); 7.22 (d, 2H); 7.29 (br s, 1H); 7.42 (d, 2H); 8.53 (br s, 1H).

NMR Verbindung 9-4 (CDCl₃, 400 MHz, δ in ppm):
1.24 (t, 3H); 2.33 (s, 3H); 3.32 (s, 2H); 4.15 (q, 2H); 7.25 (d, 2H); 7.29 (br s, 1H); 7.42 (d, 2H); 8.53 (br s, 1H).

NMR Verbindung 8-217 (CDCl₃, 400 MHz, δ in ppm):
2.33 (s, 3H); 2.61 (s, 3H); 3.54 (s, 2H); 3.69 (s, 3H); 7.49 (s, 2H); 8.49 (s, 1H); 8.53 (s, 1H); 8.62 (br s, 1H).

NMR Verbindung 5-238 (CDCl₃, 400 MHz, δ in ppm):
2.37 (s, 3H); 3.66 (s, 2H); 3.76 (s, 6H); 3.79 (s, 3H); 7.13 (m, 1H); 7.47 (m, 1H); 7.66 (m, 1H); 8.38 (br s, 1H).

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | |
|---|---|
| 75 Gewichtsteile | einer Verbindung der Formel (I), |
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | |
|---|---|
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen werden dann als wäßrige Suspension bzw. Emulsion mit einer Wasseraufwandmenge von umgerechnet 600 I/ha unter Zusatz von 0,2% Netzmittel auf die Oberfläche der Abdeckerde appliziert.
Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Erfindungsgemäße Verbindungen weisen eine gute herbizide Vorauflaufwirksamkeit gegen mehrere Schadpflanzen bei einer Aufwandmenge von 1 kg und weniger Aktivsubstanz pro Hektar auf. So haben Verbindungen aus Tabelle 1 bis 9, wie beispielsweise die Verbindung Nr. 8-217 sehr gute herbizide Wirkung gegen Schadpflanzen wie Alopecurus myosuroides, Avena fatua, Echinochloa crus galli und Setaria viridis im Vorauflaufverfahren bei einer Aufwandmenge von 1,0 kg oder 0,32 kg Aktivsubstanz pro Hektar. Andere Verbindungen aus Tabelle 1 bis 9 wie beispielsweise die Verbindung Nr. 9-4 weisen sehr gute herbizide Wirkung gegen Schadpflanzen wie Stellaria media, Veronica persica und Viola tricolor im Vorauflaufverfahren bei einer Aufwandmenge von 1,0 kg oder 0,32 kg Aktivsubstanz pro Hektar auf. In der Regel besitzen diese und andere Wirkstoffe aus den Tabellen 1 bis 9 aber noch ein wesentlich breiteres Wirkstoffspektrum.

Gleichzeitig lassen erfindungsgemäße Verbindungen zweikeimblättrige Kulturen wie Raps im Vorauflaufverfahren selbst bei hohen Wirkstoffdosierungen ungeschädigt und schonen darüber hinaus auch Gramineen-Kulturen wie Weizen, Mais und Reis. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen werden dann als wäßrige Suspension bzw. Emulsion mit einer Wasseraufwandmenge von umgerechnet 600 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen ein breites Spektrum von Ungräsern und Unkräutern bei einer Aufwandmenge von 1 kg und weniger Aktivsubstanz pro Hektar auf. So haben Verbindungen aus Tabelle 1 bis 9, wie beispielsweise die Verbindungen Nr. 7-29, 8-28, 8-217, 9-4 und 9-123 sehr gute herbizide Wirkung gegen Schadpflanzen wie Avena fatua, Echinochloa crus galli und Setaria viridis. Weiterhin haben Verbindungen aus Tabelle 1 bis 9 wie die Verbindungen Nr. 8-28 und 8-217 sehr gute herbizide Wirkung gegen Alopecurus myosuroides, jeweils im Nachauflaufverfahren bei einer Aufwandmenge von jeweils 1,0 kg oder 0,32 kg oder weniger Aktivsubstanz pro Hektar. In der Regel besitzen diese und andere Wirkstoffe aus den Tabellen 1 bis 9 aber noch ein wesentlich breiteres Wirkstoffspektrum.

## Patentansprüche

1. Verbindungen der Formel (I) oder deren Salze, 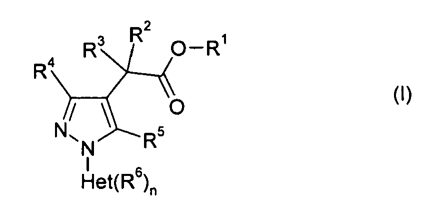 worin
Het einen fünfgliedrigen heteroaromatischen Rest mit zwei Heteroatomen als Ringatome, wobei eines der Heteroatome im Ring ein Stickstoffatom und das andere ein Schwefelatom ist und das Stickstoffatom im Ring in 1,3-Stellung zum Ring-C-atom steht, das am Pyrazolrest gebunden ist,
R¹ Wasserstoff oder einen hydrolysierbaren Rest,
R² Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
R³ Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
R² und R³ zusammen mit dem C-Atom, an das sie gebunden sind, einen carbocyclischen gesättigten oder teilweise ungesättigten Ring mit 3 bis 6 C- Atomen, der unsubstituiert oder durch einen oder mehrere Reste aus der Gruppe Halogen und (C₁-C₄)Alkyl substituiert ist, und
R⁴ Wasserstoff, Halogen, Cyano, (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, Cyano, Hydroxy, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist oder vorzugsweise unsubstituiert oder durch einen oder mehrere Reste aus der Gruppe bestehend aus Halogen, Cyano, (C₁-C₄)Alkoxy , (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
R⁵ einen Arylrest, der unsubstituiert oder vorzugsweise substituiert ist und inklusive Substituenten 6 bis 30 C-Atome, vorzugsweise 6 bis 24 C- Atome, insbesondere 6 bis 20 C-Atome aufweist, oder
einen heteroaromatischen Rest mit 1 bis 4 Heteroringatomen aus der Gruppe N, O und S, der unsubstituiert oder substituiert ist und inklusive Substituenten 1 bis 30 C-Atome aufweist, und
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio- (C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, (C₁-C₆)Haloalkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
n 0, 1, oder 2
bedeuten.

2. Verbindungen oder deren Salze gemäß Anspruch 1, **dadurch gekennzeichnet, dass**
R¹ Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, aufweist, oder
einen Rest der Formel SiR^{a}R^{b}R^{c}, -NR^{a}R^{b} oder -N=CR^{c}R^{d}, wobei in den letztgenannten 3 Formeln jeder der Reste R^{a}, R^{b}, R^{c} und R^{d} unabhängig voneinander Wasserstoff oder einen gegebenenfalls substituierten Kohlenwasserstoffrest bedeutet oder R^{a} und R^{b} zusammen mit dem N-Atom einen 3-bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten,
wobei jeder der Reste R^{a}, R^{b}, R^{c} und R^{d} inklusive Substituenten bis 30 C-Atome aufweist.

3. Verbindungen oder deren Salze gemäß Anspruch 1, **dadurch gekennzeichnet, dass**
R¹ Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome aufweist, oder einen Heterocyclylrest mit 3 bis 9 Ringatomen, der 1 bis 4 Heteroatome aus der Gruppe N, O und S enthält und der unsubstituiert oder oder substituiert ist und der inklusive Substituenten 1 bis 30 C-Atome aufweist, bedeutet.

4. Verbindungen oder deren Salze gemäß Anspruch 1, **dadurch gekennzeichnet, dass**
R¹ H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR^{*}R^{**}, -CO-NR^{*}R^{**} und -O-CO-NR^{*}R^{**},
wobei jeder der Reste R^{*} und R^{**} in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]- carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]- carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]- carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)a)koxy, -CO-O-NR'₂, -O-N=CR'₂, -N=CR'₂, -O-NR'₂, -CH(OR')₂ und -O-(CH₂)ₘ-CH(OR')₂, in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten,
substituiert ist,
bedeutet.

5. Verbindungen oder deren Salz gemäß einem der Ansprüche 1 bis 4, **dadurch gekennzeichnet, dass**
R² Wasserstoff, Halogen oder (C₁-C₄)Alkyl, das unsubstituiert oder durch einen oder mehrere Reste aus der Gruppe Halogen substituiert ist, bedeutet und
R³ Wasserstoff, Halogen oder (C₁-C₄)Alkyl, das unsubstituiert oder durch einen oder mehrere Reste aus der Gruppe Halogen substituiert ist, bedeutet oder
R² und R³ zusammen mit dem C-Atom, an das sie gebunden sind, (C₃-C₆)Cycloalkyl oder (C₅-C₆)Cycloalkenyl, wobei jeder der letztgenannten 2 Reste unsubstituiert oder durch einen oder mehrere Reste aus der Gruppe Halogen und (C₁-C₄)Alkyl substituiert ist, bedeuten.

6. Verbindungen oder deren Salze gemäß einem der Ansprüche 1 bis 5, **dadurch gekennzeichnet, dass**
R⁴ Wasserstoff, Halogen, Cyano, (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen und Hydroxy substituiert ist, vorzugsweise unsubstituiert oder durch einen oder mehrere Reste aus der Gruppe Halogen, wie Fluor und Chlor substituiert ist, oder
(C₃-C₆)Cycloalkyl, das unsubstituiert oder durch einen oder mehrere Reste aus der Gruppe bestehend aus Halogen und (C₁-C₄)Alkyl substituiert ist, oder
Phenyl, das unsubstituiert oder durch einen oder mehrere Reste aus der Gruppe bestehend aus Halogen, Nitro, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]- carbonyl substituiert ist, oder
(C₁-C₄)Alkanoyl, das unsubstituiert oder durch einen oder mehrere Reste aus der Gruppe bestehend aus Halogen, wie Fluor und Chlor, Cyano, (C₁-C₄)Alkoxy, (C₁-C₄)Haloalkoxy und (C₁-C₂)Alkoxy-(C₁-C₂)alkoxy substituiert ist, vorzugsweise Formyl, oder
[(C₁-C₄)Alkoxy]-carbonyl, das unsubstituiert oder durch einen oder mehrere Reste aus der Gruppe bestehend aus Halogen, wie Fluor und Chlor, substituiert ist, oder
[(C₃-C₆)Cycloalkoxy]-carbonyl, das unsubstituiert oder durch einen oder mehrere Reste aus der Gruppe bestehend aus Halogen und (C₁-C₄)Alkyl substituiert ist,
bedeutet.

7. Verbindungen oder deren Salze gemäß einem der Ansprüche 1 bis 6, **dadurch gekennzeichnet, dass**
R⁵ Phenyl, das unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy- (C₁-C₄)alkyl, (C₁-C₄)Alkylthio-(C₁-C₄)alkyl, Mono- und Di-[(C₁-C₄)alkyl]- aminoalkyl, Hydroxy-(C₁-C₄)alkyl, Carboxy-(C₁-C₄)alkyl, Cyano- (C₁-C₄)alkyl, (C₁-C₆)Alkoxy, das gegebenenfalls auch halogeniert sein kann [= (C₁-C₆)Haloalkoxy)], (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]- carbonyl, (C₁-C₆)Alkanoyl, (C₁-C₆)Haloalkanoyl, Mono- und Di- [(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
oder
R⁵ einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy- (C₁-C₄)alkyl, (C₁-C₄)Alkylthio-(C₁-C₄)alkyl, Mono- und Di-[(C₁-C₄)alkyl]- aminoalkyl, Hydroxy-(C₁-C₄)alkyl, Carboxy-(C₁-C₄)alkyl, Cyano- (C₁-C₄)alkyl, (C₁-C₆)Alkoxy, das gegebenenfalls auch halogeniert sein kann [= (C₁-C₆)Haloalkoxy], (C₁-C₄)Alkoxy-(C₁-C₄)alkoxy, (C₁-C₆)Alkylthio, [(C₁-C₆)Alkoxy]-carbonyl, [(C₁-C₆)Haloalkoxyl- carbonyl, (C₁-C₆)Alkanoyl, (C₁-C₆)Haloalkanoyl, Mono- und Di- [(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
bedeutet.

8. Verbindungen oder deren Salze gemäß einem der Ansprüche 1 bis 7, **dadurch gekennzeichnet, dass**
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Cyano, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl, (C₁-C₄)Alkoxycarbonyl oder (C₁-C₄)Haloalkylsulfonyl bedeutet, und
n 0, 1, oder 2
bedeuten.

9. Verfahren zur Herstellung einer Verbindung der Formel (I), wie sie gemäß einem der Ansprüche 1 bis 8 definiert ist, oder deren Salz, **dadurch gekennzeichnet, dass** man
(a) eine Verbindung der Formel (II),
H₂N-NH-Het(R⁶)ₙ (II)
worin Het und (R⁶)ₙ wie in Formel (I) definiert ist,
mit einer Verbindung der Formel (III), 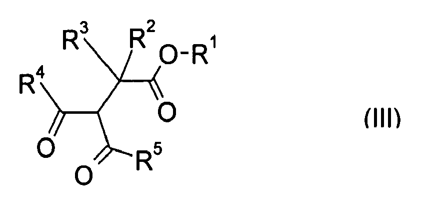 worin R¹, R², R³, R⁴ und R⁵ wie in Formel (I) definiert sind, zur Verbindung der Formel (I) oder deren Salz umsetzt,
(b) im Falle, dass R¹ in Formel (I) von Wasserstoff verschieden ist, eine Verbindung der Formel (I'), 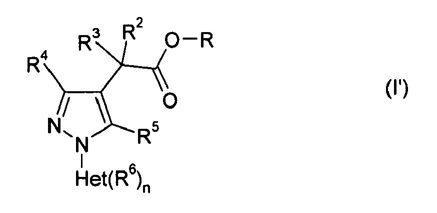 in welcher Het, R², R³, R⁴, R⁵ und R⁶ wie in Formel (I) definiert sind und
R einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet, bedeutet,
mit einer Verbindung der Formel (IV),
R¹ - OH (IV)
worin R¹ wie in Formel (I) definiert ist,
zur Verbindung der Formel (I) umsetzt
oder
(c) im Falle, dass R¹ in Formel (I) von Wasserstoff verschieden ist, eine Verbindung der Formel (I") 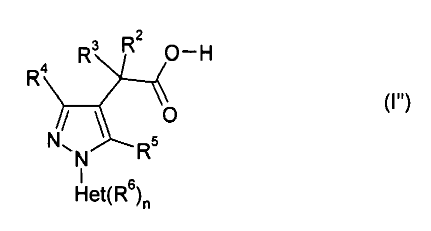 in welcher Het, R², R³, R⁴, R⁵ und R⁶ wie in Formel (I) definiert sind, gegebenenfalls nach Aktivierung der Säuregruppe, mit einer Verbindung der Formel (IV),
R¹ - OH (IV)
worin R¹ wie in Formel (I) definiert ist,
zur Verbindung der Formel (I) verestert
oder
(d) im Falle, dass die Verbindung der Formel (I), worin R = H, oder deren Salz hergestellt wird, eine Verbindung der Formel (I') [siehe Definition in Variante (b)] zur Verbindung der Formel (I) oder deren Salz hydrolysiert,
oder
g) eine Verbindung der allgemeinen Formel (XI) mit einem Bor-Derivat der Formel (XII) in Gegenwart eines Cu(I) oder Cu(II) Salzes und einer organischen Base gegebenenfalls in einem Lösemittel umsetzt, 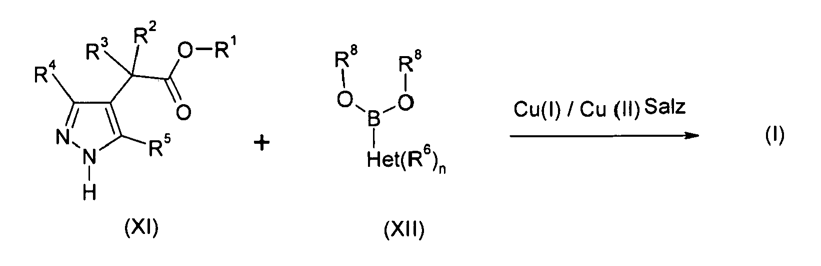 wobei in den Formeln (XI) und (XII) Het, R¹, R², R³, R⁴, R⁵, R⁶, die gemäß Formel (I) angegebenen Bedeutungen haben, und R⁸ gleich H oder (C₁-C₆)-Alkyl oder beide Alkylreste R⁸ zyklisch miteinander verknüpft sind,
oder
h) eine Verbindung der allgemeinen Formel (XI) mit einer Verbindung der Formel (XIII), worin R⁶ die gemäß Formel (I) zuvor angegebene Bedeutung hat, in Gegenwart eines geeigneten Katalysator/Liganden-Systems, mit einer geeigneten Base und in einem geeigneten Lösemittel zur Verbindung der Formel (I) oder deren Salz umsetzt, 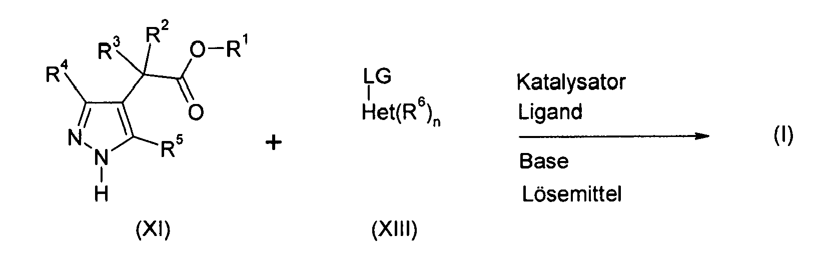 wobei in den Formeln (XI) und (XIII) die Reste Het, R¹, R², R³, R⁴, R⁵, R⁶, die gemäß Formel (I) angegebenen Bedeutungen haben und LG eine Abgangsgruppe bedeutet, oder
i) eine Verbindung der allgemeinen Formel (XV) mit einer Verbindung der allgemeinen Formel (III) in Gegenwart einer Säure gegebenenfalls in einem Lösemittel zur Verbindung der Formel (I) oder deren Salz umsetzt, 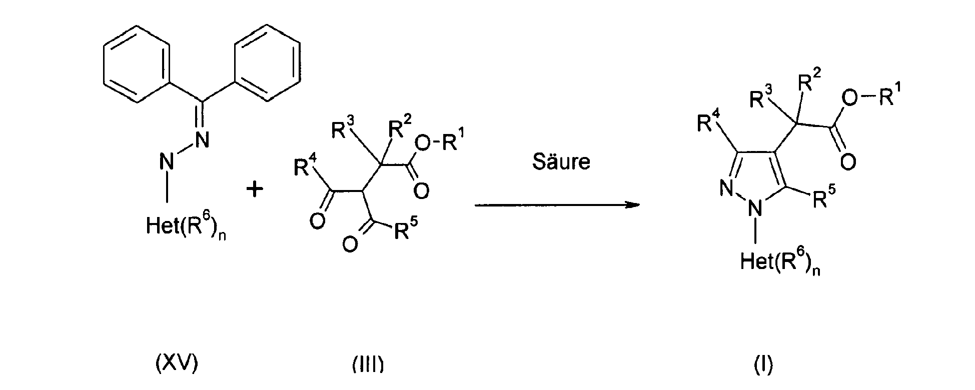 wobei in den Formeln (XV) und (III) die Reste Het, R¹, R², R³, R⁴, R⁵, R⁶ wie in Formel (I) definiert sind und LG eine Abgangsgruppe bedeutet,
oder
j) eine Verbindung der allgemeinen Formel (XVI), wobei R⁶ wie in Formel (I) definiert ist, mit Di-tert-butyl-azo-dicarboxylat (DBAD, XVII) in Gegenwart eines Kupfer-Salzes, gegebenenfalls in einem Lösemittel, zu einer Verbindung der Formel (XVIII) umsetzt, worin R⁶ wie in Formel (I) definiert ist, welche anschließend über eine Verbindung der Formel (II) oder deren Salze, worin R⁶ wie in Formel (I) definiert ist, und gemäß Verfahren a) zu Verbindungen der Formel (I) umgesetzt wird: 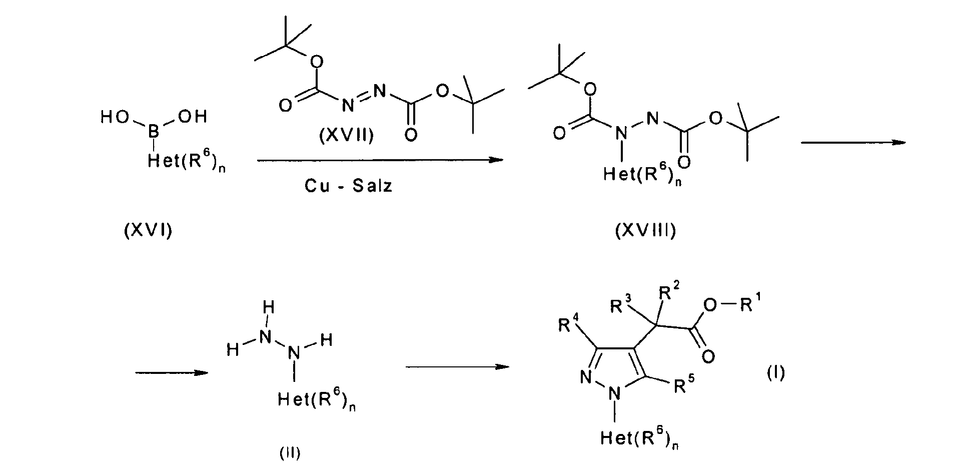 wobei in den Formeln (XVI), (XVII), (XVIII) und (II) die Reste Het, R¹, R², R³, R⁴, R⁵, R⁶ wie in Formel (I) definiert sind.

10. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 8 definiert sind, und im Pflanzenschutz übliche Formulierungshilfsmittel enthält.

11. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 8 definiert sind, auf die Pflanzen, Pflanzensamen, den Boden, in dem oder auf dem die Pflanzen wachsen, oder die Anbaufläche appliziert.

12. Verfahren nach Anspruch 11, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

13. Verfahren nach Anspruch 12, **dadurch gekennzeichnet, dass** die Kulturpflanzen transgene Kulturpflanzen sind.

14. Verwendung der Verbindungen der Formel (I) oder deren Salze gemäß einem Ansprüche 1 bis 8 als Herbizide oder Pflanzenwachstumsregulatoren.
